(19) Europäisches Patentamt
European Patent Office
Office européen des brevets

(11) Veröffentlichungsnummer: **0 076 452**
**A2**

(12) # EUROPÄISCHE PATENTANMELDUNG

(21) Anmeldenummer: 82108930.7

(22) Anmeldetag: 27.09.82

(51) Int. Cl.[3]: C 07 D 501/20
A 61 K 31/535
//C07D263/48

(30) Priorität: 30.09.81 CH 6303/81
17.05.82 CH 3049/82
11.08.82 CH 4807/82

(43) Veröffentlichungstag der Anmeldung:
13.04.83 Patentblatt 83/15

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Scartazzini, Riccardo, Dr.
Conrad Ferdinand Meyer-Strasse 38
CH-4059 Basel(CH)

(74) Vertreter: Zumstein, Fritz sen., Dr. et al,
Bräuhausstrasse 4
D-8000 München 2(DE)

(54) **Cephalosporinverbindungen, Verfahren zu ihrer Herstellung und sie enthaltende pharmazeutische Präparate.**

(57) 7β-Aminooxazolylacetylamino-3-cephem-4-carbonsäure-Verbindungen der Formel

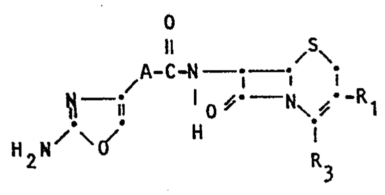,

worin hauptsächlich $R_1$ Wasserstoff, Methyl, Methoxy, Chlor oder eine Gruppe der Formel $-CH_2-R_2$, worin $R_2$ eine veresterte Hydroxy- oder Mercaptogruppe, z.B. Niederalkanoyloxy oder Heterocyclylthio, darstellt, $R_3$ Carboxyl und A durch Methoxyimino substituiertes Methylen bedeuten, Hydrate und Salze von diesen Verbindungen besitzen antibiotische Eigenschaften und sind gegen grampositive und gramnegative Mikroorganismen wirksam. Die neuen Verbindungen können z.B. in Form von antibiotisch wirksamen Präparaten zur Behandlung von Infektionen verwendet werden. Die neuen Verbindungen werden in an sich bekannter Weise hergestellt. Ebenfalls umfasst sind Zwischenprodukte.

EP 0 076 452 A2

Croydon Printing Company Ltd.

CIBA-GEIGY AG

Basel (Schweiz)

4-13575/1-3/=

BEZEICHNUNG GEÄNDERT
siehe Titelseite

Aminooxazolylverbindungen, Verfahren zu ihrer Herstellung, pharmazeutische Präparate, welche diese Verbindungen enthalten, und Verwendung von letzteren.

Die vorliegende Erfindung betrifft neue 7β-Aminooxazolylacetylamino-3-cephem-4-carbonsäure-verbindungen, Verfahren zu ihrer Herstellung, pharmazeutische Präparate, welche diese Verbindungen enthalten, und ihre Verwendung zur Herstellung von pharmazeutischen Präparaten oder als pharmakologisch wirksame Verbindungen, sowie neue Zwischenprodukte und Verfahren zu ihrer Herstellung.

Die vorliegende Erfindung betrifft 7β-Aminooxazolylacetylamino-3-cephem-4-carbonsäure-Verbindungen der Formel

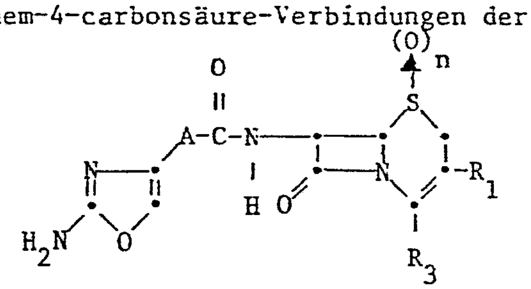

(I) ,

worin

n eine ganze Zahl von 0 bis 2, A Carbonyl, Methylen, oder eine Methylengruppe, welche durch Amino, geschütztes Amino, Hydroxy, geschütztes Hydroxy, Sulfo, geschütztes Sulfo oder durch eine Gruppe der Formel

$$=N-O-R_4$$

substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$ Wasserstoff, Niederalkyl, Niederalkoxy, Halogen oder eine Gruppe der Formel $-CH-R_2$, worin

$R_2$ Hydroxy, Mercapto, verestertes Hydroxy oder Mercapto, veräthertes Hydroxy oder Mercapto oder eine Ammoniogruppe darstellt, und $R_3$ Carboxyl oder geschütztes Carboxyl bedeuten, Hydrate und Salze von Verbindungen der Formel I, Verfahren zur Herstellung von Verbindungen der Formel I, pharmazeutische Präparate, welche Verbindungen der Formel I enthalten, und die Verwendung von Verbindungen der Formel I zur Herstellung von pharmazeutischen Präparaten oder als pharmakologisch wirksame Verbindungen.

In der Beschreibung der vorliegenden Erfindung bedeutet der im Zusammenhang mit Gruppen, z.B. Niederalkyl, Niederalkylen, Niederalkoxy, Niederalkanoyl etc., verwendete Ausdruck "Nieder", dass die entsprechenden Gruppen, falls nicht ausdrücklich anders definiert, bis zu 7 und bevorzugt bis zu 4 Kohlenstoffatomen enthalten.

In der Formel I bedeutet der Index n in erster Linie Null. Falls n den Wert 1 hat, kann die 1-Oxidogruppe in α- oder β-Stellung stehen, oder es liegt ein Gemisch von Verbindungen der Formel I mit der 1-Oxidogruppe in beiden Stellungen vor.

Der durch die Aminogruppe in 2-Stellung substituierte Oxazolylrest kann auch in tautomerer Form als ein durch eine Iminogruppe substituierter Dihydrooxazolylrest oder als Gemisch von beiden Tautomeren vorliegen. Die Lage des Gleichgewichts zwischen den beiden Tautomeren hängt von externen Parametern wie Temperatur, Lösungsmittel oder pH-Wert ab. In der vorliegenden Beschreibung wird der Aminooxazolylrest nur als Aminooxazolylrest bezeichnet. Es ist aber auch das Dihydroiminooxazolyl-Tautomere umfasst.

Die vor- und nachstehend verwendeten allgemeinen Definitionen haben im Rahmen der vorliegenden Beschreibung vorzugsweise die folgenden Bedeutungen:

Niederalkyl $R_1$ enthält 1-4 Kohlenstoffatome und ist beispielsweise Aethyl, Propyl, Butyl oder insbesondere Methyl.

Niederalkoxy $R_1$ enthält 1-4 Kohlenstoffatome und ist beispielsweise Aethoxy, Propoxy, Butoxy oder insbesondere Methoxy.

Halogen $R_1$ ist Fluor, Brom, Jod oder bevorzugt Chlor.

Verestertes Hydroxy oder Mercapto $R_2$ ist eine Hydroxy- oder Mercaptogruppe, welche durch eine aliphatische Carbonsäure, eine durch Acyl, z.B. Niederalkanoyl, z.B. Acetyl, substituierte, aliphatische Carbonsäure oder die Carbaminsäure verestert ist, beispielsweise Niederalkanoyloxy, z.B. Acetyloxy, Niederalkanoylniederalkanoyloxy, z.B. Acetylacetyloxy, oder Carbamoyloxy bzw. Niederalkanoylthio, z.B. Acetylthio oder Formylthio, oder Carbamoylthio.

Verestertes Hydroxy oder Mercapto $R_2$ ist ferner eine Hydroxy- oder Mercaptogruppe, welche durch eine N-substituierte Carbaminsäure verestert ist.

N-Substituenten sind beispielsweise Niederalkyl, z.B. Methyl oder Aethyl, oder durch Halogen, z.B. Chlor, oder Niederalkanoyloxy, z.B. Acetoxy, substituiertes Niederalkyl, z.B. 2-Chloräthyl oder 2-Acetoxyäthyl.

Durch eine N-substituierte Carbaminsäure verestertes Hydroxy oder Mercapto $R_2$ ist beispielsweise N-Methylcarbamoyloxy, N-Aethylcarbamoyloxy, N-(2-Chloräthyl)-carbamoyloxy, N-(2-Acetoxyäthyl)-carbamoyloxy oder N-Methylcarbamoylthio.

Veräthertes Hydroxy oder Mercapto $R_2$ ist eine Hydroxy- oder Mercaptogruppe, welche durch einen aliphatischen Kohlenwasserstoffrest veräthert ist, beispielsweise Niederalkoxy mit 1-4 C-Atomen, z.B. Methoxy oder Aethoxy, oder Niederalkylthio mit 1-4 C-Atomen, z.B. Methylthio.

Veräthertes Mercapto $R_2$ ist ebenfalls durch einen Heterocyclus veräthert, welcher über ein Ringkohlenstoffatom mit dem Schwefelatom der Mercaptogrüppe verbunden ist, z.B. durch einen monocyclischen Heterocyclus, welcher 1 bis 4 Stickstoffheteroatome und gegebenenfalls ein zusätzliches Sauerstoff- oder Schwefelatom besitzt oder durch einen bicyclischen Heterocyclus mit 1 bis 5 Stickstoffheteroatomen. Eine solche verätherte Mercaptogruppe wird im folgenden "Heterocyclylthiogruppe $R_2$" genannt.

Ein heterocyclischer Rest in einer Heterocyclylthiogruppe $R_2$ ist insbesondere ein aromatischer, monocyclischer, fünf- oder sechsgliedriger, Diaza-, Triaza-, Tetraaza-, Thiaza-, Thiadiaza-, Thia-, Oxaza- oder Oxadiazacyclylrest oder ist ein aromatischer oder partiell gesättigter, bicyclischer, fünf- oder sechs Ringatome pro Ring enthaltender Aza-, Diaza-, Triaza-, Tetraza- oder Pentazabicyclylrest.

Substituenten des genannten heterocyclischen Restes in einer Heterocyclylthiogruppe $R_2$ sind beispielsweise Niederalkyl, z.B. Aethyl, n-Propyl, Isopropyl, n-Butyl, Isobutyl oder tert.-Butyl, insbesondere Methyl, Niederalkenyl, z.B. Allyl, oder Niederalkyl, z.B. Methyl oder Aethyl, welches durch folgende Gruppen substituiert ist:

Hydroxy, verestertes Hydroxy, z.B. Niederalkanoyloxy, z.B. Acetyloxy, oder Halogen, z.B. Fluor oder Chlor, gegebenenfalls in Salzform, z.B. in Alkalimetall-, z.B. Natriumsalzform, vorliegendes Niederalkylphosphonyl, z.B. Methyl- oder Aethylphosphonyl, Diniederalkylphosphonyl, z.B. Dimethyl- oder Diäthylphosphonyl, gegebenenfalls in Salzform, z.B. in Alkalimetall- oder Ammoniumsalzform, z.B. in Natriumsalzform, vorliegendes Carboxyl oder Sulfo, verestertes Carboxyl, z.B. Niederalkoxycarbonyl, z.B. Aethoxycarbonyl, Sulfamoyl, Amino, Niederalkylamino, z.B. Methyl- oder Aethylamino, Diniederalkylamino, z.B. Dimethylamino oder Diäthylamino, Acylamino, z.B. Niederalkanoylamino, z.B. Acetylamino, oder durch Carboxyl oder Halogen, z.B. Chlor,

substituiertes Niederalkanoylamino, z.B. Carboxyacetylamino oder Chloracetylamino.

Ein solcher substituierter Niederalkylrest ist beispielsweise: Hydroxyniederalkyl, z.B. Hydroxymethyl oder 2-Hydroxyäthyl, Acetyloxyniederalkyl, z.B. Acetyloxymethyl oder 2-Acetyloxyäthyl, Halogenniederalkyl, z.B. Chlormethyl, 2-Chloräthyl, 2,2,2-Trichloräthyl oder Trifluormethyl, Niederalkylphosphononiederalkyl, z.B. Aethylphosphonomethyl, Diniederalkylphosphononiederalkyl, z.B. Diäthylphosphonomethyl, Carboxyniederalkyl, z.B. Carboxymethyl oder 2-Carboxyäthyl, Sulfoniederalkyl, z.B. Sulfomethyl oder 2-Sulfoäthyl, Niederalkoxycarbonylniederalkyl, z.B. Aethoxycarbonylmethyl oder 2-Aethoxycarbonyläthyl, Sulfamoylniederalkyl, z.B. Sulfamoylmethyl oder 2-Sulfamoyläthyl, Aminoniederalkyl, z.B. Aminomethyl oder 2-Aminoäthyl, Niederalkylaminoniederalkyl, z.B. Methylaminomethyl oder 2-Methylaminoäthyl, Diniederalkylaminoniederalkyl, z.B. Dimethylaminomethyl oder 2-Dimethylaminoäthyl, Niederalkanoylaminoniederalkyl, z.B. 2-Acetylaminoäthyl, Carboxyniederalkanoylaminoniederalkyl, z.B. 3-Carboxypropionylaminoäthyl oder 2-Carboxyacetylaminoäthyl, oder Halogenniederalkanoylaminoniederalkyl, z.B. 3-Chlorpropionylaminoäthyl oder 2-Chloracetylaminoäthyl.

Funktionelle Gruppen oder abgewandelte, z.B. geschützte, funktionelle Gruppen, z.B. Halogen, z.B. Fluor, Chlor oder Brom, unsubstituiertes oder substituiertes Amino, z.B. unsubstituiertes oder durch Niederalkyl, z.B. Methyl oder Aethyl, mono- oder disubstituiertes Amino, z.B. Amino, Methylamino oder Dimethylamino, Acylamino, z.B. Niederalkanoylamino, z.B. Acetylamino, oder Niederalkylsulfonylamino, z.B. Mesylamino, oder durch Halogen, z.B. Chlor, oder Carboxy substituiertes Niederalkanoylamino, z.B. 3-Chlorpropionylamino oder 3-Carboxypropionylamino, Nitro, Hydroxy, Niederalkoxy, z.B. Methoxy oder Aethoxy, Carboxy, verestertes Carboxy, z.B. Niederalkoxycarbonyl, z.B. Methoxycarbonyl oder Aethoxycarbonyl, gegebenenfalls substituiertes, z.B. mono- oder diniederalkyliertes Carbamoyl, z.B. Methylcarbamoyl oder Dimethylcarbamoyl, oder Cyan, sowie Oxo oder

Oxido sind ebenfalls Substituenten des heterocyclischen Restes in einer Heterocyclylthiogruppe $R_2$.

Eine Heterocyclylthiogruppe $R_2$, worin der heterocyclische Rest einen aromatischen, monocyclischen, fünfgliedrigen Rest darstellt, ist u.a. Imidazolylthio, z.B. 2-Imidazolylthio, Triazolylthio oder durch Niederalkyl, z.B. Methyl, und/oder Phenyl substituiertes Triazolylthio, z.B. 1H-1,2,3-Triazol-5-yl-thio, 1-Methyl-1H-1,2,3,triazol-4-ylthio, 1H-1,2,4,Triazol-3-ylthio, 5-Methyl-1H-1,2,4,-triazol-3-ylthio oder 4,5-Dimethyl-4H-1,2,4-triazol-3-ylthio, Tetrazolylthio z.B. 1H-Tetrazol-5-ylthio, oder durch Niederalkyl, z.B. Methyl oder Aethyl, oder substituiertes Niederalkyl, z.B. Aethyl- oder Diäthylphosphonomethyl, Carboxyäthyl, Sulfomethyl, 2-Sulfoäthyl, 2-Dimethylaminoäthyl oder Cyanomethyl substituiertes Tetrazolylthio z.B. 1-Methyl-1H-tetrazol-5-ylthio, 1-Aethyl- oder 1-Diäthylphosphonylmethyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio oder 1-Cyanomethyl-1H-tetrazol-5-ylthio, Thiazolylthio oder durch Niederalkyl, z.B. Methyl, substituiertes Thiazolylthio, z.B. 2-Thiazolylthio oder 4,5-Dimethyl-2-thiazolylthio, Isothiazolylthio, z.B. 3-Isothiazolylthio, 4-Isothiazolylthio oder 5-Isothiazolylthio, Thiadiazolylthio oder durch Niederalkyl, z.B. Methyl, substituiertes Thiadiazolylthio z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazol-5-ylthio, Oxazolylthio oder durch Niederalkyl, z.B. Methyl, substituiertes Oxazolylthio, z.B. 2- oder 5-Oxazolylthio, oder 4-Methyl-5-oxazolylthio, Isooxazolylthio oder durch Niederalkyl, z.B. Methyl, substituiertes Isooxazolylthio, z.B. 3-Methyl-5-isoxazolylthio, oder Oxadiazolylthio oder durch Niederalkyl, z.B. Methyl, substituiertes Oxadiazolylthio, z.B. 1,2,4-Oxadiazol-5-ylthio oder 2-Methyl-1,3,4-oxadiazol-5-ylthio.

Eine Heterocyclylthiogruppe $R_2$, worin der heterocyclische Rest einen aromatischen, monocyclischen, sechsgliedrigen Rest darstellt, enthält 1-3 Stickstoffatome und ist beispielsweise 5,6-Dioxotetrahydro-as-triazinylthio oder durch Niederalkyl, z.B. Methyl, Carboxyniederalkyl, z.B. Carboxymethyl, oder durch Sulfoniederalkyl, z.B. Sulfomethyl, substituiertes 5,6-Dioxotetrahydro-as-trianzinylthio, z.B. 1- oder 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, 1- oder 2-Carboxymethyl-5,6-dioxo-1,2,5,6-tetrahydro-as-triazin-3-ylthio, 4-Carboxymethyl-5,6-dioxo-1,4,5,6-tetrahydro-as-triazin-3-ylthio, 1- oder 2-Sulfomethyl-5,6-dioxo-1,2,5,6-tetrahydro-as-triazin-3-ylthio oder 4-Sulfomethyl-5,6-dioxo-1,4,5,6-tetrahydro-as-triazin-3-ylthio.

Eine Heterocyclylthiogruppe $R_2$, worin der heterocyclische Rest einen aromatischen oder partiell gesättigten, bicyclischen, fünf- oder sechs Ringatome pro Ring enthaltenden Rest darstellt, ist beispielsweise Indolylthio, durch Niederalkyl, z.B. Methyl, substituiertes Indolylthio, z.B. Indol-2-ylthio oder N-Methylindol-2-ylthio, Isoindolylthio, z.B. Isoindol-2-ylthio, Chinolylthio, z.B. 2-, 4- oder 8-Chinolylthio, Benzimidazolylthio, durch Niederalkyl, z.B. Methyl oder Carboxyniederalkyl, z.B. Carboxymethyl, substituiertes Benzimidazolylthio, z.B. 1-Methyl-, 1-Carboxymethyl- oder 1-(2-Carboxyäthyl)-benzimidazol-2-ylthio, Benzotriazolylthio,durch Niederalkyl, z.B. Methyl, oder Carboxyniederalkyl, z.B. Carboxymethyl, substituiertes Benzotriazolylthio, z.B. 1-Methyl- oder 1-Carboxymethyl-1H-benzo[d]-triazol-5-ylthio, oder insbesondere Tetrazolopyridazinylthio, durch Niederalkyl, z.B. Methyl oder Aethyl, Carboxy, Carboxyniederalkyl, z.B. Carboxymethyl, Carbamoyl, Niederalkylcarbamoyl,z.B. Methylcarbamoyl, Di-niederalkyl-carbamoyl, z.B. Dimethylcarbamoyl, Amino, Niederalkylamino, z.B. Methylamino, Di-niederalkyl-amino, z.B. Dimethylamino oder Diäthylamino substituiertes Tetrazolopyridazinylthio, z.B. 8-Methyl-, 8-Aethyl-, 8-Carboxy-, 8-Carboxymethyl-, 8-(2-Carboxyäthyl)-, 8-Carbamoyl-, 8-(N-Methylcarbamoyl)-, 8-(N,N-Dimethylcarbamoyl)-, 8-Amino-, 8-Dimethylamino- oder 8-Diäthylamino-tetrazolo[1,5-b]pyridazin-6-ylthio.

Eine Ammoniogruppe $R_2$ ist von einer tertiären organischen Base, beispielsweise von einem aliphatischen Amin oder vorzugsweise von einer heterocyclischen Stickstoffbase, abgeleitet und ist über das Stickstoffatom an die in 3-Stellung des Cephemgerüsts befindliche Methylengruppe gebunden. Die positive Ladung am quaternären Stickstoffatom wird beispielsweise durch die in 4-Stellung des Cephemgerüsts befindliche, negativ geladene Carboxylatgruppe kompensiert.

Eine Ammoniogruppe $R_2$, welche von einem aliphatischen Amin abgeleitet ist, ist beispielsweise Triniederalkylammonio, z.B. Trimethyl- oder Triäthylammonio.

Eine Ammoniogruppe $R_2$, welche von einer unsubstituierten oder substituierten heterocyclischen Stickstoffbase abgeleitet ist, ist beispielsweise unsubstituiertes oder durch Niederalkyl, z.B. Methyl oder Aethyl, Niederalkenyl, z.B. Vinyl oder Allyl, Carboxyniederalkyl, z.B. Carboxymethyl, Niederalkoxycarbonylniederalkyl, z.B. Methoxycarbonylmethyl, Sulfoniederalkyl, z.B. Sulfomethyl, Aminoniederalkyl, z.B. 2-Aminoäthyl, oder Diniederalkylaminoniederalkyl, z.B. 2-Dimethylaminoäthyl, in 2-Stellung substituiertes 1-Pyrazolio, z.B. 2-Methyl- oder 2-Aethyl-1-pyrazolio, 2-Allyl- oder 2-Vinyl-1-pyrazolio, 2-Carboxymethyl-1-pyrazolio, 2-Methoxycarbonylmethyl-1-pyrazolio, 2-Sulfomethyl-1-pyrazolio, 2-(2-Aminoäthyl)-1-pyrazolio oder 2-(2-Dimethylaminoäthyl)-1-pyrazolio.

Eine Ammoniogruppe $R_2$, welche von einer heterocyclischen Stickstoffbase abgeleitet ist, ist ebenfalls beispielsweise 1-Triazolio oder durch Niederalkyl, z.B. Methyl oder Aethyl, Carboxyniederalkyl, z.B. Carboxymethyl oder Diniederalkylaminoniederalkyl, z.B. 2-Dimethylaminoäthyl, in 3-Stellung substituiertes 1-Triazolio, z.B. 3-Methyl-1-triazolio, 3-Carboxymethyl-1-triazolio oder 3-(2-Dimethylaminoäthyl)-1-triazolio.

Eine Ammoniogruppe $R_2$, welche von einer unsubstituierten oder substituierten heterocyclischen Stickstoffbase abgeleitet ist, ist

ebenfalls beispielsweise Pyridinio oder durch Niederalkyl, z.B. Methyl, Carbamoyl, Niederalkylcarbamoyl, z.B. Methylcarbamoyl, Hydroxyniederalkenyl, z.B. Hydroxymethyl, Niederalkoxyniederalkyl, z.B. Methoxymethyl, Cyanniederalkyl, z.B. Cyanmethyl, Carboxyniederalkyl, z.B. Carboxymethyl, Sulfoniederalkyl, z.B. 2-Sulfoäthyl, Carboxyniederalkylen, z.B. 2-Carboxyvinyl, Carboxyniederalkylthio, z.B. Carboxymethylthio, Thiocarbamoyl, Halogen, z.B. Brom oder Chlor, Carboxy, Sulfo oder Cyan mono- oder disubstituiertes Pyridinio, z.B. Niederalkylpyridinio, z.B. 2-, 3- oder 4-Methylpyridinio oder 2-, 3- oder 4-Aethylpyridinio, Carbamoylpyridinio, z.B. 3- oder 4-Carbamoylpyridinio, Niederalkylcarbamoylpyridinio, z.B. 3- oder 4-Methylcarbamoylpyridinio, Diniederalkylcarbamoylpyridinio, z.B. 3- oder 4-Dimethylcarbamoylpyridinio, Hydroxyniederalkylpyridinio, z.B. 3- oder 4-Hydroxymethylpyridinio, Niederalkoxyniederalkylpyridinio, z.B. 4-Methoxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkyl-carbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanohalogenmethylpyridinio, z.B. 3-Cyano-4-trifluormethylpyridinio oder Amino-carboxypyridinio, z.B. 2-Amino-3-carboxypyridinio.

Eine quaternäre Ammoniogruppe $R_2$ ist bevorzugt 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B.

3-Methyl-1-triazolio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio.

$R_3$ mit der Bedeutung "geschütztes Carboxyl" ist durch eine der im folgenden beschriebenen Carboxylschutzgruppen verestertes Carboxyl, insbesondere unter physiologischen Bedingungen spaltbares Carboxyl.

In einer Methylengruppe A, welche durch in geschützter Form vorliegendes Amino, Hydroxy oder Sulfo substituiert ist, ist die betreffende Aminomethylen-, Hydroxymethylen oder Sulfomethylengruppe durch die im folgenden beschriebenen Amino-, Hydroxy- oder Sulfoschutzgruppen geschützt.

Die Gruppe der Formel $-O-R_4$ liegt in syn-(oder Z-)Stellung oder anti-(oder E-)Stellung vor, wobei die syn-(oder Z-)Stellung bevorzugt ist. In syn-Stellung ist $-O-R_4$ zum Cephalosporingerüst und in anti-Stellung entgegengesetzt gerichtet.

Niederalkyl $R_4$ hat vorzugsweise 1-4 C-Atome und ist beispielsweise Aethyl, Propyl, Butyl oder insbesondere Methyl.

Cycloalkyl $R_4$ hat vorzugsweise 3-8, in erster Linie 3-6, Ringglieder und ist z.B. Cyclobutyl, Cyclopentyl, Cyclohexyl oder insbesondere Cyclopropyl.

Substituenten einer Niederalkylgruppe oder Cycloalkylgruppe $R_4$ sind u.a. freies oder veräthertes Hydroxy, z.B. Niederalkoxy, primäres, sekundäres oder tertiäres Amino, z.B. Amino oder Diniederalkylamino, freies oder funktionell abgewandeltes, z.B. verestertes, amidiertes oder geschütztes, Carboxyl oder Sulfo, sowie gegebebenenfalls

durch Niederalkyl N-substituiertes Ureidocarbonyl. Bevorzugt ist eine substituierte Niederalkyl- und Cycloalkylgruppe $R_4$ durch eine Carboxyl- oder Sulfogruppe substituiert, wobei diese bevorzugt am Kohlenstoffatom, das mit dem Sauerstoffatom der Oxyiminogruppe verbunden ist, stehen.

Substituiertes Niederalkyl oder Cycloalkyl $R_4$ ist beispielsweise 2-Aminoäthyl, 2-Dimethylaminoäthyl, Carboxymethyl, 1- oder 2-Carboxyäthyl, 1-, 2- oder 3-Carboxyprop-1-yl, 1- oder 2-Carboxyprop-2-yl, 2-Carboxycycloprop-2-yl oder 1- oder 2-Carboxycyclobut-1-yl, sowie eine entsprechende durch Sulfo substituierte Niederalkyl- und Cycloalkylgruppe

Die Carboxy- und Sulfogruppen im Rest $R_4$ können beispielsweise durch Niederalkyl, z.B. Methyl oder Aethyl, oder durch eine physiologisch abspaltbare Gruppe, z.B. durch Pivaloyloxymethyl, verestert oder durch $NH_3$, ein primäres oder sekundäres Amin, z.B. ein Mono- oder Diniederalkylamin, z.B. Methyl- oder Aethylamin oder Dimethyl- oder Diäthylamin, amidiert sein oder durch die weiter unten genannten üblichen Schutzgruppen geschützt sein.

Substituiertes Carbamoyl $R_4$ ist beispielsweise eine Gruppe der Formel -C(=O)-NHR, worin R Niederalkyl, z.B. Methyl, Aethyl oder 1- oder 2-Propyl, Carboxyniederalkyl, z.B. Carboxymethyl, 1- oder 2-Carboxyäthyl oder 1-, 2- oder 3-Carboxypropyl, worin die Carboxygruppe in freier Form vorliegt oder durch eine der üblichen Carboxyschutzgruppen geschützt oder beispielsweise durch Niederalkyl, z.B. Methyl, Aethyl, n- oder Isopropyl, oder n- oder tert.-Butyl, verestert ist, Sulfoniederalkyl, z.B. Sulfomethyl, 1- oder 2-Sulfoäthyl oder 1-, 2- oder 3-Sulfopropyl, worin die Sulfogruppe in freier Form vorliegt oder durch eine der üblichen Sulfoschutzgruppen geschützt oder beispielsweise durch Niederalkyl, z.B. Methyl oder Aethyl, verestert ist, Hydroxyniederalkyl, z.B. Hydroxymethyl, 2-Hydroxyäthyl oder 2- oder 3-Hydroxypropyl, worin die Hydroxygruppe in freier Form vorliegt

oder durch eine der üblichen Hydroxyschutzgruppen geschützt oder beispielsweise acyliert, z.B. acetyliert, ist, Aminoniederalkyl, z.B. 2-Aminoäthyl, 2- oder 3-Aminopropyl oder 2-, 3- oder 4-Aminobutyl, worin die Aminogruppe in freier Form vorliegt oder durch eine der üblichen Aminoschutzgruppen geschützt oder beispielsweise acyliert, z.B. acetyliert, ist, Arylniederalkyl, beispielsweise Phenylniederalkyl, z.B. Benzyl oder 1- oder 2-Phenyläthyl, Halogenniederalkyl, beispielsweise Fluor-, Chlor- oder Bromniederalkyl, z.B. 2-Chlorpropyl- oder 4-Chlorbutyl, Aryl, z.B. Phenyl, oder durch Niederalkyl, z.B. Methyl, Niederalkoxy, z.B. Methoxy, Halogen, z.B. Chlor oder Nitro, ein- bis dreifach substituiertes Phenyl bedeutet.

Die in den Verbindungen der Formel I vorhandenen funktionellen Gruppen, insbesondere die Carboxyl- und Amino-, ferner die Hydroxy- und Sulfogruppen, sind gegebenenfalls durch übliche Schutzgruppen (=conventional protecting groups) geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art sowie ihre Abspaltung sind bespielsweise in "Protective Groups in Organic Chemistry", Plenum Press, London, New York, 1973, ferner in "The Peptides", Vol. I, Schröder and Lubke, Academic Press, London, New York, 1965, sowie in "Methoden der organischen Chemie", Houben-Weyl, 4. Auflage, Bd. 15/1, Georg Thieme Verlag, Stuttgart, 1974, beschrieben.

Eine Carboxylgruppe, z.B. die Carboxylgruppe $R_3$, ferner eine in $R_4$ vorhandene Carboxylgruppe ist üblicherweise in veresterter Form geschützt, wobei die Estergruppe unter schonenden Bedingungen leicht spaltbar ist. Eine in veresterter Form geschützte Carboxylgruppe

ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxylgruppe, welche durch eine Niederalkylgruppe verestert ist, welche in 1-Stellung der Niederalkylgruppe verzweigt ist, ist beispielsweise tert.-Niederalkoxycarbonyl, z.B. tert.-Butyloxycarbonyl, Arylmethoxycarbonyl mit einem oder zwei Arylresten, worin Aryl unsubstituiertes oder z.B. durch Niederalkyl, z.B. tert.-Niederalkyl, z.B. tert.-Butyl, Niederalkoxy, z.B. Methoxy, Hydroxy, Halogen, z.B. Chlor, und/oder Nitro, mono- oder polysubstituiertes Phenyl bedeutet, beispielsweise unsubstituiertes oder z.B. wie oben erwähnt substituiertes Benzyloxycarbonyl, z.B. 4-Nitrobenzyloxycarbonyl oder 4-Methoxybenzyloxycarbonyl, oder unsubstituiertes oder z.B. wie oben erwähnt substituiertes Diphenylmethoxycarbonyl, z.B. Diphenylmethoxycarbonyl oder Di-(4-methoxyphenyl)-methoxycarbonyl.

Eine geschützte Carboxylgruppe, welche durch eine Niederalkylgruppe verestert ist, welche in 1- oder 2-Stellung der Niederalkylgruppen durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxymethoxycarbonyl, 1-Niederalkylthioniederalkoxycarbonyl, z.B. 1-Methlythiomethoxycarbonyl oder 1-Aethylthioäthoxycarbonyl, Aroylmethoxycarbonyl, worin die Aroylgruppe unsubstituiert oder z.B. durch Halogen, wie Brom, substituiertes Benzoyl darstellt, z.B. Phenacyloxycarbonyl, sowie 2-Halogenniederalkoxycarbonyl, z.B. 2,2,2-Trichloräthoxycarbonyl, 2-Bromäthoxycarbonyl oder 2-Jodäthoxycarbonyl.

Eine Carboxylgruppe kann auch als organische Silyloxycarbonylgruppe geschützt sein. Eine Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch nur durch zwei Niederalkylgruppen, z.B. Methylgruppen, und die Carboxylgruppe oder Aminogruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine bevorzugte geschützte Carboxylgruppe ist beispielsweise tert.-Niederalkoxycarbonyl, z.B. tert.-Butyloxycarbonyl, unsubstituiertes oder wie oben erwähnt, substituiertes Benzyloxycarbonyl, z.B. 4-Nitro-benzyloxycarbonyl, oder Diphenylmethoxycarbonyl.

Eine unter physiologischen Bedingungen spaltbare, veresterte Carboxylgruppe $R_3$ ist in erster Linie eine Acyloxyniederalkoxycarbonylgruppe, worin Acyl, z.B. die Acylgruppe einer organischen Carbonsäure, in erster Linie einer gegebenenfalls substituierten Niederalkancarbonsäure bedeutet, oder worin Acyloxymethyl den Rest eines Lactons bildet.

Eine solche Gruppe ist beispielsweise Niederalkanoyloxyniederalkoxycarbonyl, z.B. Niederalkanoyloxymethoxycarbonyl oder Niederalkanoyloxyäthoxycarbonyl, z.B. Acetyloxymethoxycarbonyl, Pivaloyloxymethoxycarbonyl oder 2-(Propionyloxy)-äthoxycarbonyl, Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine Aminogruppe, z.B. die Aminogruppe in 2-Stellung des Oxazolylrestes, kann z.B. in Form einer leicht spaltbaren Acylamino-, Arylmethylamino-, verätherten Mercaptoamino-, 2-Acylniederalk-1-enylamino- oder Silylaminogruppe geschützt sein.

In einer leicht spaltbaren Acylaminogruppe ist Acyl beispielsweise die Acylgruppe einer organischen Carbonsäure mit bis zu 18 Kohlenstoffatomen, insbesondere einer unsubstituierten oder einer, z.B. durch Halogen oder Aryl, substituierten Alkancarbonsäure oder der unsubstituierten oder, z.B. durch Halogen, Niederalkoxy oder Nitro, substituierten Benzoesäure oder eines Kohlensäurehalbesters. Eine solche Acylgruppe ist beispielsweise Niederalkanoyl, z.B. Formyl, Acetyl, oder Propionyl, Halogenniederalkanoyl, z.B. 2-Halogenacetyl, insbesondere 2-Chlor-, 2-Brom-, 2-Jod-, 2,2,2-Trifluor- oder 2,2,2-Trichloracetyl, unsubstituiertes oder z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl.

In 1-Stellung des Niederalkylrests verzweigtes Niederalkoxycarbonyl ist beispielsweise tert.-Niederalkoxycarbonyl, z.B. tert.-Butyloxycarbonyl, Arylmethoxycarbonyl mit einem oder zwei Arylresten, worin Aryl vorzugsweise unsubstituiertes oder z.B. durch Niederalkyl, insbesondere tert.-Niederalkyl, z.B. tert.-Butyl, Niederalkoxy, z.B. Methoxy, Hydroxy, Halogen, z.B. Chlor, und/oder Nitro, mono- oder polysubstituiertes Phenyl bedeutet, beispielsweise unsubstituiertes oder substituiertes Diphenylmethoxycarbonyl, z.B. Benzhydryloxycarbonyl oder Di-(4-methoxyphenyl)-methoxycarbonyl.

In 1- oder 2-Stellung geeignet substituiertes Niederalkoxycarbonyl ist beispielsweise Triniederalkylsilyloxycarbonyl, z.B. Trimethylsilyloxycarbonyl, Aroylmethoxycarbonyl, worin Aroyl unsubstituiertes oder z.B. durch Halogen, z.B. Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen unsubstituierten oder z.B. durch Niederalkyl, Niederalkoxy, Aryl, Halogen oder Nitro, substituierten aliphatischen, araliphatischen, cycloaliphatischen oder aromatischen Kohlenwasserstoffrest mit bis zu 15 C-Atomen bedeuten, z.B. unsubstituiertes oder wie oben erwähnt substituiertes Niederalkyl, Phenylniederalkyl, Cycloalkyl oder Phenyl, beispielsweise 2-Triniederalkylsilyläthoxycarbonyl, z.B. 2-Trimethylsilyläthoxycarbonyl oder 2-(n-Butyldimethylsilyl)-äthoxycarbonyl, oder 2-Triarylsilyläthoxycarbonyl, z.B. 2-Triphenylsilyläthoxycarbonyl.

Weitere als Aminoschutzgruppen in Frage kommende Acylgruppen sind auch Acylgruppen von organischen Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, Diphenylphosphoryl, unsubstituiertes oder z.B. durch Nitro substituiertes Diphenylniederalkylphosphoryl, z.B. Dibenzylphosphoryl oder Di-4-nitrobenzylphosphoryl, Phenyloxyphenylphosphonyl, Diniederalkylphosphinyl, z.B. Diäthylphosphinyl, ferner Diphenylphosphinyl.

In einer Arylmethylaminogruppe, die Mono-, Di- oder insbesondere Triarylmethylamino darstellt, sind die Arylreste insbesonsere Phenylreste. Solche Gruppen sind beispielsweise Benzyl-, Diphenylmethyl- und insbesondere Tritylamino.

In einer verätherten Mercaptoaminogruppe ist die verätherte Mercaptogruppe in erster Linie Arylthio oder Arylniederalkylthio, worin Aryl insbesondere unsubstituiertes oder z.B. durch Niederalkyl, z.B. Methyl oder tert.-Butyl, Niederalkoxy, z.B. Methoxy, Halogen, z.B. Chlor, und/oder Nitro substituiertes Phenyl ist. Eine solche Aminoschutzgruppe ist z.B. 4-Nitrophenylthio.

In einer als Aminoschutzgruppe verwendbaren 2-Acylniederalk-1-en-ylgruppe ist Acyl z.B. die Acylgruppe einer Niederalkancarbonsäure, der unsubstituierten oder z.B. durch Niederalkyl, z.B. Methyl oder tert.-Butyl, Niederalkoxy, z.B. Methoxy, Halogen, z.B. Chlor und/oder Nitro substituierten Benzoesäure, oder insbesondere die Acylgruppe eines

Kohlensäurehalbesters, z.B. eines Kohlensäureniederalkylhalbesters. Solche Aminoschutzgruppen sind in erster Linie 1-Niederalkanoyl-prop-1-en-2-yl, z.B. 1-Acetylprop-1-en-2-yl, oder 1-Niederalkoxy-carbonylprop-1-en-2-yl, z.B. 1-Aethoxycarbonylprop-1-en-2-yl.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine Aminogruppe kann auch in protonierter Form geschützt werden. Als Anionen kommen in erster Linie die Anionen von starken anorganischen Säuren, wie von Halogenwasserstoffsäuren, z.B. das Chlor- oder Bromanion, oder von organischen Sulfonsäuren, wie p-Toluolsulfonsäure, in Frage.

Eine bevorzugte Aminoschutzgruppe ist beispielsweise der Acylrest eines Kohlensäurehalbesters, insbesondere tert.-Butyloxycarbonyl, unsubstituiertes oder z.B. wie angegeben substituiertes Benzyloxy-carbonyl, z.B. 4-Nitrobenzyloxycarbonyl oder Diphenylmethoxycarbonyl, 2-Halogenniederalkoxycarbonyl, z.B. 2,2,2-Trichloräthoxycarbonyl, ferner Trityl oder Formyl.

Eine Hydroxygruppe, z.B. die Hydroxygruppe in der Hydroxyiminogruppe $=N-O-R_4$ ($R_4$ = Wasserstoff), kann beispielsweise durch eine Acylgruppe, z.B. durch Halogen substituiertes Niederalkanoyl, z.B. 2,2-Dichloracetyl, oder insbesondere durch einen im Zusammenhang mit geschützten Aminogruppen genannten Acylrest eines Kohlensäurehalbesters geschützt sein. Eine bevorzugte Hydroxyschutzgruppe ist beispielsweise 2,2,2-Trichloräthoxycarbonyl, ein organischer Silylrest mit den weiter vorn genannten Substituenten, ferner eine leicht abspaltbare veräthernde Gruppe, wie tert.-Niederalkyl, z.B. tert.-Butyl, 2-oxa- der ein

2-thiaaliphatischer oder -cycloaliphatischer Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, 1-Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder ein entsprechendes Thiaanaloges, sowie unsubstituiertes oder substituiertes 1-Phenylniederalkyl, z.B. unsubstituiertes oder substituiertes Benzyl oder Diphenylmethyl, wobei die Phenylreste beispielsweise durch Halogen, wie Chlor, Niederalkoxy, z.B. Methoxy, und/oder Nitro substituiert sind.

Eine Sulfogruppe, z.B. eine in $R_2$ vorhandene Sulfogruppe, ist vorzugsweise durch einen aliphatischen, cycloaliphatischen, cycloaliphatisch-aliphatischen, aromatischen oder araliphatischen Alkohol, z.B. durch Niederalkanol, z.B. tert.-Butanol, oder durch eine Silylgruppe, z.B. durch Triniederalkylsilyl, verestert. Eine Sulfogruppe ist z.B. analog einer Carboxygruppe geschützt.

Salze von erfindungsgemässen Verbindungen sind in erster Linie pharmazeutisch verwendbare, nicht-toxische Salze von Verbindungen der Formel I.

Solche Salze werden beispielsweise von den in Verbindungen der Formel I vorhandenen sauren Gruppen, z.B. von Carboxyl- oder Sulfogruppen, gebildet und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische oder araliphatische, primäre, sekundäre oder tertiäre Mono-, Di- oder Polyamine, sowie heterocyclische Basen für die Salzbildung in Frage kommen. Solche Basen sind beispielsweise Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylamino-

BAD ORIGINAL

aminoäthylester, Niederalkylenamine, z.B. 1-Aethylpiperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

Die in den Verbindungen der Formel I vorhandenen basischen Gruppen, z.B. Aminogruppen, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden.

Liegen in Verbindungen der Formel I mehrere saure Gruppen, z.B. zwei Carboxylgruppen, oder mehrere basische Gruppen, z.B. zwei Aminogruppen, vor, können Mono- oder Polysalze gebildet werden. Wenn die Verbindungen der Formel I mindestens eine saure Gruppe, z.B die Carboxylgruppe $R_3$, und mindestens eine basische Gruppe in freier Form, z.B. die freie Aminogruppe in 2-Stellung des Oxazolylrestes besitzt, können Verbindungen der Formel I in Form eines inneren Salzes vorliegen. In Verbindungen der Formel I können eine saure und eine basische Gruppe in Form eines inneren Salzes und zusätzliche saure und/oder basische Gruppen beispielsweise als Säureadditions- und/oder Baseadditionssalz vorliegen.

Zur Isolierung oder Reinigung können auch pharmazeutisch ungeeignete Salze Verwendung finden. Zur therapeutischen Anwendung gelangen nur pharmazeutisch verwendbare, nicht toxische Salze, die deshalb vevorzugt sind.

Die Verbindungen der Formel I, worin die funktionellen Gruppen in freier Form und die Carboxylgruppen gegebenenfalls in physiologisch spaltbarer veresterter Form vorliegen, und ihre pharmazeutisch ververwendbaren, nicht-toxischen Salze sind wertvolle, antibiotisch wirksame Stoffe, die insbesondere als antibakterielle Antibiotika verwendet werden können. Beispielsweise sind sie in vitro gegen grampositive und gramnegative Mikroorganismen, inklusive β-Lactamase produzierende Stämme, beispielsweise gegen grampositive Kokken, z.B. gegen Staphylococcus aureus oder Streptokokken, z.B. Streptococcus pyogenes, Streptococcus pneumoniae oder Streptococcus faecalis, gegen gramnegative Kokken, z.B. Neisseria gonorrhoeae, in Minimalkonzentrationen von ca. 0,001 µg/ml bis ca. 32 µg/ml, gegen gramnegative Bakterien, beispielsweise Pseudomonas aeruginosa, Haemophilus influenzae oder gegen Enterobakterien, z.B. Escherichia coli, Proteus spp., Klebsiella pneumoniae, Serratia marcescens oder Enterobacter cloacae, in Minimalkonzentrationen von ca. 0,005 bis ca. 132 µg/ml wirksam. In vivo, bei subkutaner Anwendung an der Maus, sind sie gegen systemische Infektionen, welche durch grampositive Keime wie Staphylococcus aureus verursacht werden, in einem Dosisbereich von ca. 1,3 bis ca. 55 mg/kg und gegen systemische Infektionen, welche durch gramnegative Keime wie Enterobakterien, z.B. Escherichia coli, verursacht werden, in einem Dosisbereich von ca. 0,63 - 30 mg/kg wirksam.

Im folgenden Versuchsbericht wird anhand von ausgewählten Verbindungen die Wirksamkeit von Verbindungen der Formel I gezeigt:

Versuchsbericht

I. Getestete Verbindungen

1. 3-(1-Methyl-1H-tetrazol-5-ylthiomethyl)-7β-[2-(2-amino-4-oxazolyl)-2-(Z-methoxyimino-acetylamino]-3-cephem-4-carbonsäure-natriumsalz (Beispiel 5a)

2. 3-(1-Carboxymethyl-1H-tetrazol-5-ylthiomethyl)-7β-[2-(2-amino-4-oxazolyl)-2-Z-methoxyiminoacetylamino]-3-cephem-4-carbonsäure-cli= natriumsalz (Beispiel 6a)

3. 3-[1-(2-Dimethylaminoäthyl)-1H-tetrazol-5-ylthiomethyl]-7β-[2-(2-amino-4-oxazolyl)-2-Z-methoxyiminoacetylamino]-3-cephem-4-carbonsäure-dihydrochlorid (Beispiel 7)

4. 3-[1-(2-Dimethylaminoäthyl)-1H-tetrazol-5-ylthiomethyl]-7β-[2-(2-amino-4-oxazolyl-2-E-methoxyiminoacetylamino]-3-cephem-4-carbonsäure-dihydrochlorid (Beispiel 7)

5. 3-(4-Carbamoylpyridiniummethyl)-7β-[2-amino-4-oxazolyl)-2-Z-methoxyiminoacetylamino]-3-cephem-4-carboxylat (Beispiel 8)

6. 3-Acetoxymethyl-7β-[2-(2-amino-4-oxazolyl)-2-Z-hydroxyiminoacetylamino]-3-cephem-4-carbonsäure-natriumsalz (Beispiel 9a)

7. 3-Acetoxymethyl-7β-[2-(2-amino-4-oxazolyl)-2-Z-(2-carboxyprop-2-yloxyimino)-acetylamino]-3-cephem-4-carbonsäure-natriumsalz (Beispiel 10a)

8. 3-Acetoxymethyl-7β-[2-(2-amino-4-oxazolyl)-2-Z-(2-tert.-butyloxycarbonylprop-2-yloxyimino)-acetylamino]-3-cephem-4-carbonsäure (Beispiel 10b)

9. 3-Pyridiniomethyl-7β-[2-(2-amino-4-oxazolyl)-2-Z-(2-carboxyprop-2-yloxyimino)-acetylamino]-3-cephem-4-carboxylat-natriumsalz (Beispiel 11)

10. 3-(4-Carbamoylpyridiniomethyl)-7β-[2-(2-amino-4-oxazolyl)-2-Z-(2-carboxyprop-2-yloxyimino)-acetylamino]-2-cephem-4-carboxylat-natriumsalz (Beispiel 12)

11. 7β-[2-(2-Amino-4-oxazolyl)-2-Z-(2-carboxyprop-2-yloxyimino)-acetylamino]-3-cephem-4-carbonsäure-cli-natriumsalz (Beispiel 13a)

12. 7β-[2-(2-Amino-4-oxazolyl)-2-Z-(2-tert.-butyloxycarbonylprop-2-yloxyimino)-acetylamino]-3-cephem-4-carbonsäure-natriumsalz (Beispiel 13b)

13. 3-Acetoxymethyl-7β-[2-(2-amino-4-oxazolyl)-2-Z-methoxyiminoacetylamino]-3-cephem-4-carbonsäure-natriumsalz (Beispiel 1a)

14. 7β-[2-(2-Amino-4-oxazolyl)-2-Z-methoxyiminoacetylamino]-3-cephem-4-carbonsäure-natriumsalz (Beispiel 2a)

15. 7β-[2-(2-Amino-4-oxazolyl)-2-Z-methoxyiminoacetylamino]-3-cephem-4-carbonsäurepivaloyloxymethylester (Beispiel 3a)

16. 3-(3-Chlorpyridiniomethyl-7β-[2-(2-amino-4-oxazolyl)-2-Z-methoxyiminoacetylamino]-3-cephem-4-carboxylat (Beispiel 15)

17. 3-(4-Carboxylatpyridiniomethyl)-7β-[2-(2-amino-4-oxozolyl)-2-Z-methoxyiminoacetylamino]-3-cephem-4-carboxylat-natriumsalz (Beispiel 16)

18. 3-Carbamoyloxymethyl-7β-[2-(2-amino-4-oxazolyl)-2-Z-methoxyiminoacetylamino]-3-cephem-4-carbonsäure-natriumsalz (Beispiel 17a)

## II. Experimentelles:

A. Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibiting concentrations) werden in Mikrogramm pro Mililiter (μg/ml) für die geprüften Verbindungen in der Tabelle 1 angegeben.

B. Die chemotherapeutische Wirksamkeit in vivo gegen systemische Infektionen in weiblicher SPF, $MF_2$ Mäusen wurde nach der Methode von Zak, O. et al., 1979, Drugs Exptl. Clin. Res. 5, 45-59, ermittelt. Die gefundenen $ED_{50}$-Werte in Milligramm Substanz pro Kilogramm Maus (mg/kg) für eine Anzahl von Mikroorganismen werden für die subcutan (s.c.) applizierten Testverbindungen in der Tabelle 2 angegeben.

## III. Versuchsergebnisse:

Tabelle 1: Antibiotische Aktivität (in vitro)

| Testverbindung | MIC [μg/ml | | |
|---|---|---|---|
| | Streptococcus pyogenes Aronson | Escherichia coli 205 | Proteus rettgeri 856 |
| 1 | 0,005 | 0,1 | 0,005 |
| 2 | 0,1 | 0,5 | 0,005 |
| 3 | 0,02 | 0,1 | 0,02 |
| 4 | 0,05 | 8 | 2 |
| 5 | 0,02 | 0,2 | 0,02 |
| 6 | 0,005 | 0,1 | 0,02 |
| 7 | 0,05 | 0,2 | 0,01 |
| 8 | 0,02 | 4 | 1 |
| 9 | 0,5 | 1 | 0,2 |
| 10 | 0,2 | 0,5 | 0,5 |

Tabelle 1: Antibiotische Aktivität (in vitro) (Fortsetzung)

| Testverbindung | MIC [µg/ml] | | |
|---|---|---|---|
| | Streptococcus pyogenes Aronson | Escherichia coli 205 | Proteus rettgeri 856 |
| 11 | 0,2 | 0,2 | 0,01 |
| 12 | 0,02 | 4 | 0,2 |
| 13 | 0,01 | 0,1 | 0,005 |
| 14 | 0,01 | 0,2 | 0,005 |
| 15 | 0,2 | 8 | 0,1 |
| 16 | 0,02 | 0,5 | n.g. |
| 17 | 0,05 | 0,5 | 0,02 |
| 18 | 0,02 | 0,1 | 0,01 |

n.g.: nicht getestet

Tabelle 2: Chemotherapeutische Wirksamkeit (in vivo)

| Testverbindung | $ED_{50}$ [mg/kg, s.c.] | |
|---|---|---|
| | Staphylococcus aureus 10 B | Escherichia coli 205 |
| 1 | 12 | 1,5 |
| 2 | 55 | 0,63 |
| 3 | 28 | 1,7 |
| 5 | 5,5 | 2,4 |
| 6 | 1,35 | 1,0 |
| 7 | >30 | >30 |
| 8 | >30 | >30 |
| 9 | >30 | 5,6 |
| 10 | >30 | 1,6 |
| 11 | >30 | 4,4 |
| 12 | >30 | >30 |

Tabelle 2: Chemotherapeutische Wirksamkeit (in vivo) (Fortsetzung)

| Testverbindung | $ED_{50}$ [mg/kg, s.c.] | |
|---|---|---|
| | Staphyloccous aureus 10 B | Escherichia coli 205 |
| 13 | 5 | 1 |
| 14 | 30 | 9 |
| 15 | 30 | 14 |
| 16 | 5,5 | 2 |
| 17 | >30 | n.g. |
| 18 | 8 | >30 |

n.g.: nicht getestet

Verbindungen der Formel I, worin die funktionellen Gruppen geschützt sind, werden als Zwischenprodukte zur Herstellung von Verbindungen der Formel I verwendet, worin die funktionellen Gruppen in freier Form vorliegen oder worin die 4-Carboxylgruppe in Form einer unter physiologischen Bedingungen spaltbaren Carboxylgruppe vorliegt.

Die vorliegende Erfindung betrifft insbesondere Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, N-Niederalkylcarbamoyloxy, einen aromatischen, monocyclischen, fünf- oder sechsgliedrigen, Diaza-, Triaza-, Tetraaza-, Thiaza-, Thiadiaza-, Thia-, Oxaza- oder Oxadiazacyclylrest, z.B. Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazinylthio, z.B. 5,6-Dioxo-1,2,5,6-tetrahydro-as-triazin-3-ylthio oder 5,6-Dioxo-1,4,5,6-tetrahydro-as-triazin-3-ylthio, welcher durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl, Carboxyniederalkyl, z.B. Carboxymethyl, Amino, Carboxyniederalkylamino, z.B. 2-Carboxyäthylamino, oder durch Carbamoyl substituiert sein kann, oder Ammonio, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxyl oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl, oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A Amino, Aminomethylen, Hydroxymethylen, Sulfomethylen oder eine durch eine Gruppe der Formel $=N-O-R_4$ substituierte Methylengruppe, worin $R_4$ Wasserstoff, Niederalkyl, z.B. Methyl, Hydroxyniederalkyl, z.B. 2-Hydroxyäthyl, Aminoniederalkyl, z.B. 2-Aminoäthyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, darstellt, bedeuten, wobei die Gruppe der Formel $-O-R_4$ die syn-(oder Z-)Stellung aufweist, und Salze, insbesondere pharmazeutisch verwendbare Salze, von Verbindungen der Formel I, welche salzbildende Gruppen besitzen.

Die vorliegende Erfindung betrifft hauptsächlich Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Triazolylthio, z.B. 1H-1,2,3-Triazol-5-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B.

Sulfomethyl, Carboxyniederalkyl, z.B. Carboxymethyl, oder durch Carbamoyl substituiertes Tetrazolylthio, z.B. 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio, durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydrotriazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxyl, oder unter physiologischen Bedinungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl, oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Wasserstoff, Niederalkyl, z.B. Methyl, Carboxyniederalkyl, z.B. 2-Carboxy-2-propyl, Carbamoyl oder Niederalkylcarbamoyl, z.B. Methylcarbamoyl, darstellt, bedeuten, wobei die Gruppe der Formel $-O-R_4$ die syn-(oder Z-)Stellung aufweist und Salze, insbesondere pharmazeutisch verwendbare Salze, von Verbindungen der Formel I, welche salzbildende Gruppen besitzen.

Die vorliegende Erfindung betrifft in erster Linie Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Tetrazolylthio, z.B. 1H-Tetrazol-5-ylthio, durch Niederalkyl, z.B.

Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Bromypyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel $-O-R_4$ die syn-(oder Z-)Stellung aufweist, und Salze, insbesondere pharmazeutisch verwendbare Salze, von Verbindungen der Formel I, welche salzbildende Gruppen aufweisen.

Vor allem betrifft die vorliegende Erfindung die in den Beispielen beschriebenen Verbindungen der Formel I und deren Salze, insbesondere deren pharmazeutisch verwendbare Salze.

Verbindungen der Formel I und Salze von solchen Verbindungen, die eine salzbildende Gruppe aufweisen, werden beispielsweise hergestellt, indem man

a) in einer Verbindung der Formel

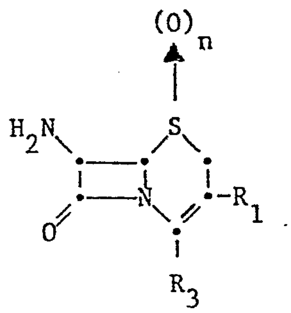

(II) ,

worin n, $R_1$ und $R_3$ die unter Formel I genannten Bedeutungen haben und die 7β-Aminogruppe in freier Form vorliegt oder durch eine die Acylierungsreaktion erlaubende Gruppe geschützt ist und in $R_1$ vorhandene funktionelle Gruppen geschützt sind, oder in einem Salz davon die 7β-Aminogruppe durch Umsetzung mit einem den Acylrest einer Carbonsäure der Formel

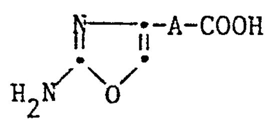

(III) ,

einführenden Acylierungsmittel, worin A die unter Formel I genannten Bedeutungen hat und die Aminogruppe in 2-Stellung des Oxazolylrests, sowie in A vorhandene funktionelle Gruppen gegebenenfalls geschützt sind, oder mit einem Salz davon acyliert oder

b) zur Herstellung einer Verbindung der Formel I, worin n Null bedeutet, eine 2-Cephem-Verbindung der Formel

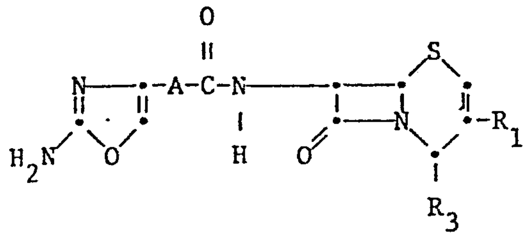

(IV) ,

worin $R_1$, $R_3$ und A die unter Formel I genannten Bedeutungen haben und die Aminogruppe in 2-Stellung des Oxazolylrests, sowie in $R_1$ und A vorhandene funktionelle Gruppen gegebenenfalls geschützt sind, oder ein Salz davon zur entsprechenden 3-Cephemverbindung isomerisiert oder

c) eine Verbindung der Formel

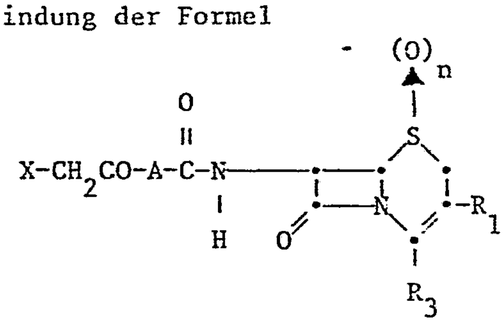

(V) ,

worin n, $R_1$, $R_3$ und A die unter Formel I genannten Bedeutungen haben und X Halogen bedeutet und in $R_1$ und A vorhandene funktionelle Gruppen gegebenenfalls geschützt sind, oder ein Salz davon mit Harnstoff kondensiert, oder

d) zur Herstellung einer Verbindung der Formel I, worin A durch Hydroxyimino substituiertes Methylen bedeutet, eine Verbindung der Formel

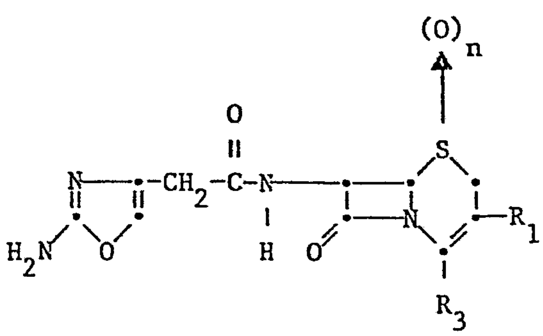

(VI) ,

worin n, $R_1$ und $R_3$ die unter Formel I genannten Bedeutungen haben und die Aminogruppe in 2-Stellung des Oxazolylrests und in $R_1$ vorhandene funktionelle Gruppen gegebenenfalls geschützt sind, oder ein Salz davon mit einem Nitrosierungsmittel behandelt und, wenn erwünscht, eine erhältliche Verbindung der Formel I in eine andere Verbindung der Formel I umwandelt und/oder eine erhältliche Verbindung, worin n Null bedeutet, in eine Verbindung überführt, worin n eins oder zwei bedeutet, und/oder eine erhältliche Verbindung, worin n eins oder zwei bedeutet, in eine Verbindung überführt, worin n Null bedeutet, und/oder in einer erhältlichen Verbindung in geschützter Form vorliegende funktionelle Gruppen in freie funktionelle Gruppen überführt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomeren auftrennt.

Verfahren a) (Acylierung):

In einem Ausgangsmaterial der Formel II ist eine in $R_1$ vorhandene funktionelle Gruppe, z.B. eine Carboxyl-, Amino- oder Hydroxylgruppe, durch eine der weiter vorn genannten Schutzgruppen geschützt.

Die 7β-Aminogruppe in einem Ausgangsmaterial der Formel II ist gegebenenfalls durch eine die Acylierungsreaktion erlaubende Gruppe geschützt. Eine solche Gruppe ist beispielsweise eine organische Silylgruppe, ferner eine Ylidengruppe, die zusammen mit der Aminogruppe eine Schiff'sche Base bildet. Eine organische Silylgruppe ist z.B. eine solche Gruppe, die auch mit einer Carboxylgruppe $R_3$ eine geschützte Carboxylgruppe bilden kann. Dies ist in erster Linie eine Triniederalkylsilylgruppe, insbesondere Trimethylsilyl. Bei der Silylierung zum Schutz einer 4-Carboxylgruppe in einem Ausgangsmaterial der Formel II kann bei Verwendung eines Ueberschusses des Silylierungs-

mittels die Aminogruppe ebenfalls silyliert werden. Eine Ylidengruppe ist in erster Linie eine 1-Arylniederalkyliden-, insbesondere eine 1-Arylmethylengruppe, worin Aryl besonders für einen carbocyclischen, in erster Linie monocyclischen, Arylrest steht, z.B. für gegebenenfalls durch Niederalkyl, Hydroxy, Niederalkoxy und/oder Nitro substituiertes Phenyl.

Ein den Acylrest einer Carbonsäure der Formel III einführendes Acylierungsmittel ist die Carbonsäure der Formel III selbst oder ein reaktionsfähiges funktionelles Derivat oder ein Salz davon. In einem Ausgangsmaterial der Formel III ist eine in $R_4$ vorhandene funktionelle Gruppe, z.B. eine Carboxyl-, Amino- oder Hydroxylgruppe, welche an der Acylierungsreaktion nicht teilnehmen soll, durch eine der weiter vorn genannten Schutzgruppen geschützt.

In einem Ausgangsmaterial der Formel III kann eine vorhandene Aminogruppe auch in ionischer Form geschützt sein, z.B. in Form eines Säureadditionssalzes, welches beispielsweise mit einer starken anorganischen Säure, z.B. einer Halogenwasserstoffsäure, z.B. Salzsäure oder Schwefelsäure, oder mit einer organischen Säure, z.B. p-Toluolsulfonsäure, gebildet wird.

Falls eine freie Säure der Formel III zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Aceto-

BAD ORIGINAL

nitril oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis etwa +50°C, und/oder in einer Inertgas-, z.B. Stickstoffatmosphäre, durchgeführt.

Ein reaktionsfähiges, d.h. ein Carboxamid-bildendes, funktionelles Derivat einer Carbonsäure der Formel III ist in erster Linie ein Anhydrid der Carbonsäure der Formel III, vorzugsweise ein gemischtes Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung des gemischten Anhydrids eignen, sind Phosphor-haltige Säuren, z.B. Phosphorsäure, Diäthylphosphorsäure oder phosphorige Säure, Schwefel-haltige Säuren, z.B. Schwefel oder Cyanwasserstoffsäure. Ein reaktionsfähiges, funktionelles Derivat einer Carbonsäure der Formel III wird ausserdem durch Kondensation mit einer organischen Carbonsäure gebildet, z.B. mit einer unsubstituierten oder durch Halogen, z.B. Fluor oder Chlor, substituierten Niederalkancarbonsäure, z.B. Pivalinsäure oder Trifluoressigsäure, mit einem Niederalkylhalbester der Kohlensäure, z.B. dem Aethyl- oder Isobutylhalbester der Kohlensäure, oder mit einer organischen, z.B. aliphatischen oder aromatischen Sulfonsäure, z.B. Methansulfonsäure oder p-Toluolsulfonsäure.

Ein reaktionsfähiges funktionelles Derivat einer Carbonsäure der Formel III ist ebenfalls ein aktivierter Ester der Carbonsäure der Formel III, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel III und z.B. Dimethyl-(1-chloräthyliden)-

BAD ORIGINAL

iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. ein Pentachlor-, 4-Nitrophenyl- oder 2,3-Dinitrophenylester, ein N-heteroaromatischer Ester, z.B. N-Benztriazolester, oder ein N-Diacyliminoester, z.B. ein N-Succinylimino- oder N-Phthalyliminoester.

Die Acylierung mit einem reaktionsfähigen funktionellen Derivat der Carbonsäure der Formel III, z.B. mit einem entsprechenden Anhydrid, insbesondere einem Säurehalogenid, wird bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, z.B. Triniederalkylamin, z.B. Trimethylamin, Triäthylamin oder Aethyl-diisopropylamin, oder ein N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Ein geeignetes säurebindendes Mittel ist ferner eine anorganische Base, beispielsweise ein Alkalimetall- oder Erdalkalimetallhydroxid, -carbonat- oder -hydrogencarbonat, z.B. Natrium-, Kalium- oder Calciumhydroxid, -carbonat oder -hydrogencarbonat, oder ist ein Oxiran, beispielsweise ein 1,2-Niederalkylenoxid, wie Aethylenoxid oder Propylenoxid.

Die Acylierung mit einem reaktionsfähigen, funktionellen Derivat der Carbonsäure der Formel III wird vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, wenn notwendig oder erwünscht, bei erniedrigter

oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung einer Verbindung der Formel II kann auch bei Verwendung eines geeigneten reaktionsfähigen, funktionellen Derivats der Säure der Formel III in Gegenwart einer geeigneten Acylase erfolgen. Solche Acylasen sind bekannt und können durch eine Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung wird insbesondere ein Amid, Ester oder Thioester, wie ein Niederalkyl-, z.B. Methyl- oder Aethylester, der Carbonsäure der Formel III als reaktionsfähiges, funktionelles Derivat eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Ein bei der Acylierungsreaktion eingesetztes, reaktionsfähiges funktionelles Derivat einer Säure der Formel III kann, wenn erwünscht, in situ gebildet werden. So kann man z.B. ein gemischtes Anhydrid in situ herstellen, indem man eine Säure der Formel III, worin funktionelle Gruppen gegebenenfalls geschützt sind, oder ein geeignetes Salz davon, z.B. ein Ammoniumsalz, welches z.B. mit einer organischen Base, wie Pyridin oder 4-Methylmorpholin gebildet wird, oder ein Metallsalz, z.B. ein Alkalimetallsalz, z.B. Natriumsalz, mit einem geeigneten Derivat einer anderen Säure, beispielsweise einem Säurehalogenid, einer unsubstituierten oder durch Halogen, z.B. Chlor, substituierten Niederalkancarbonsäure, z.B. Trichloracetylchlorid, einem Halbester eines Kohlensäurehalbhalogenids, z.B. Chlorameisensäureäthylester, oder -isobutylester, oder mit einem Halogenid einer

Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung bei der Acylierungsreaktion verwenden.

Verfahren b) (Isomerisierung):

In einem 2-Cephem-Ausgangsmaterial der Formel IV weist die gegebenenfalls geschützte 4-Carboxylgruppe vorzugsweise die α-Konfiguration auf.

2-Cephem-Verbindungen der Formel IV können isomerisiert werden, indem man sie mit einem schwach basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Picoline, Collidine oder Lutidine, ferner Chinolin, tertiäre aromatische Basen, z.B. solche des Anilintyps, wie N,N-Diniederalkylaniline, z.B. N,N-Dimethylanilin oder N,N-Diäthylanilin, oder tertiäre aliphatische, azacycloaliphatische oder araliphatische Basen, wie Triniederalkylamine, z.B. Trimethylamin oder N,N-Diisopropyläthylamin, N-Niederalkylazacycloalkane, z.B. N-Methylpiperidin, oder N,N-Diniederalkylphenylniederalkylamine, z.B. N,N-Dimethylbenzylamin, sowie Gemische von solchen basischen Mitteln, wie das Gemisch einer Base vom Pyridintyp, und eines Triniederalkylamins, z.B. Pyridin und Triäthylamin. Ferner können auch anorganische oder organische Salze von Basen, insbesondere von mittelstarken bis starken Basen mit schwachen Säuren, wie Alkalimetall- oder Ammoniumsalze von Niederalkancarbonsäuren, z.B. Natriumacetat, Triäthylammoniumacetat oder N-Methylpiperidinacetat, sowie andere analoge Basen oder Gemische von solchen basischen Mitteln verwendet werden.

Bei der Isomerisierung von 2-Cephem-Verbindungen der Formel IV mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete unter den Reaktionsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von 2-Cephem-Verbindungen der Formel IV zur entsprechenden 3-Cephem-Verbindung wird vorzugsweise durchgeführt, indem man diese in 1-Stellung oxydiert, wenn erwünscht, gegebenenfalls Isomerengemische der gebildeten 1-Oxide trennt, und die so erhältlichen 1-Oxide der entsprechenden 3-Cephem-Verbindungen reduziert.

Als geeignete Oxydationsmittel für die Oxydation in 1-Stellung von 2-Cephem-Verbindungen der Formel IV kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind beispielsweise Perjod- und Perschwefelsäure. Organische Persäuren sind beispielsweise Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure einzusetzen,

wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxydation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1 - 2 % und weniger, aber auch grössere Mengen der Säure einsetzen kann. Dabei hängt die Wirksamkeit des Gemisches in erster Linie von der Stärke der Säure ab. Geeignete Gemische sind z.B. Wasserstoffperoxid mit Essigsäure, Perchlorsäure oder Trifluoressigsäure.

Die obige Oxydation kann in Gegenwart von geeigneten Katalysatoren durchgeführt werden. So kann z.B. die Oxydation mit Percarbonsäuren durch die Anwesenheit einer Säure mit einer Dissoziationskonstante von wenigstens $10^{-5}$ katalysiert werden, wobei ihre Wirksamkeit als Katalysator von ihrer Säurestärke abhängt. Als Katalysatoren geeignete Säuren sind z.B. Essigsäure, Perchlorsäure und Trifluoressigsäure. Ueblicherweise verwendet man mindestens äquimolare Mengen des Oxydationsmittels, vorzugsweise einen geringen Ueberschuss von etwa 10 % bis etw 20 %, wobei man auch grössere Ueberschüsse, d.h. bis zur 10-fachen Menge des Oxydationsmittels oder darüber verwenden kann. Die Oxydation wird unter milden Bedingungen, z.B. bei Temperaturen von etwa -50°C bis etwa +100°C, vorzugseise von etwa -10° C bis etwa +40° C, durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten,

und die man gegebenenfalls zusammen mit einem geeigneten Trägermaterial, wie Kohle oder Bariumsulfat, einsetzt; Zinn-, Eisen-, Kupfer- oder Mangankationen mit reduzierender Wirkung, welche in Form von anorganischen oder organischen Verbindungen, oder Komplexen, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigäsure oder Nitrolotrieessigsäure, verwendet werden; Dithionit-, Jod- oder Eisen-II-cyanid-anionen, mit reduzierender Wirkung, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; trivalente anorganische oder organische Phosphorverbindungen mit reduzierender Wirkung, wie Phosphine, ferner Ester, Amide und Halogenide der phosphonigen oder phosphorigen Säure, sowie diesen Phosphor-Sauerstoffverbindungen entsprechende Phosphor-Schwefelverbindungen, worin in diesen Verbindungen organische Reste in erster Linie aliphatische, aromatische oder araliphatische Reste, z.B. gegebenenfalls substituiertes Niederalkyl, Phenyl oder Phenylniederalkyl darstellen, wie z.B. Triphenylphosphin, Tri-n-butylphosphin, Diphenylphosphonigsäuremethylester, Diphenylchlorphosphin, Phenyldichlorphosphin. Benzolphosphonigsäuredimethylester, Butanphosphonigsäuremethylester, Phosphorsäuretriphenylester, Phosphorigsäuretrimethylester, Phosphortrichlorid, Phosphortribromid, etc.; Halogensilanverbindungen mit reduzierender Wirkung, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, Di- oder Tribromsilan, Diphenylchorsilan, oder Dimethylchlorsilan, sowie auch Halgensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefel enthaltende Silane, wie 1,3-Dithia-2,4,-disilacyclobutane oder 1,3,5-Trithia-2,4,6-trisilacyclohexane, deren Siliciumatome durch Kohlenwasserstoffreste, wie insbesondere Niederalkyl substituiert sind, z.B. 2,2,4,4,Tetramethyl-1,3-dithia-2,4-disilacyclobutan oder 2,2,4,4,6,6-Hexamethyl-1,3,5-trithia-2,4,6-trisilacyclohexan, etc.; reduzierend wirkende quaternäre Chlormethyleniminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylenpyrrolidiniumchlorid; oder reduzierend wirkende komplexe Metallhydride, wie Natriumborhydrid, in Gegenwart von geeigneten Aktivierungsmittel, wie Cobalt-II-chlorid, sowie Borandichlorid.

Als aktivierende Mittel, die zusammen mit einem weiter vorn genannten Reduktionsmittel verwendet werden, welche keine oder schwache Lewissäure-Eigenschaften aufweisen, d.h. aktivierende Mittel, welche in erster Linie zusammen mit Dithionit-, Jod- oder Eisen-II-cyanid- und nicht-halogenhaltigen trivalenten Phosphor-Reduktionsmitteln oder bei der katalytischen Reduktion eingesetzt werden, eignen sich insbesondere organische Carbon- und Sulfonsäurehalogenide, ferner Schwefel-, Phosphor- oder Siliciumhalogenide mit gleicher oder grössere Hydrolysekonstante zweiter Ordnung als Benzoylchlorid, z.B. Phosgen, Oxalylchlorid, Essigsäurechlorid oder -bromid, Chloressigsäurechorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner Säureanhydride, wie Trifluoressigsäureanhydrid, oder cyclische Sultone, wie Aethansulton, Propansulton, 1,3-Butansulton oder 1,3-Hexansulton.

Die Reduktion wird vorzugsweise in Gegenwart von Lösungsmitteln oder Gemischen davon durchgeführt, deren Auswahl in erster Linie

durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Verfahren c) (Ringschluss):

In einem Ausgangsmaterial der Formel V bedeutet X Halogen, bevorzugt Chlor, ferner Brom, Jod oder Fluor. Eine Verbindung der Formel V mit einer salzbildenden Gruppe, z.B. $R_3$ mit der Bedeutung Carboxyl, kann als Salz, z.B. als Alkalimetall- oder als Ammoniumsalz, z.B. als Lithium-, Natrium-, Kalium-, Triniederalkyl-, z.B. Trimethyl- oder Triäthylammoniumsalz, eingesetzt werden.

Der Harnstoff wird in äquivalenter Menge oder im Ueberschuss zugesetzt.

Die Reaktion wird im allgemeinen in einem Lösungsmittel, wie Wasser oder einem organischen, inerten Lösungsmittel oder einer Mischung davon, durchgeführt. Als organische Lösungsmittel geeignet sind Alkohole, wie Methanol, Aethanol oder Isopropanol, Ketone, wie

BAD ORIGINAL

Aceton, Aether, wie Dioxan oder Tetrahydrofuran, Nitrile, wie Acetonitril, halogenierte Kohlenwasserstoffe, wie Methylenchlorid, Chloroform oder Tetrachlorkohlenstoff, Ester, wie Aethylacetat, oder Amide, wie Dimethylformamid oder Dimethylacetamid, und ähnliche. Die Reaktion kann, falls eine freie Verbindung der Formel V eingesetzt wird, in Gegenwart einer Base durchgeführt werden. Geeignete Basen sind Alkalimetallhydroxide, wie Natrium- oder Kaliumhydroxid, Alkalimetallcarbonate, wie Natrium- oder Kaliumcarbonat, oder organische tertiäre Stickstoffbasen, wie Triniederalkylamine, z.B. Trimethylamin, Triäthylamin, Aethyl-diisopropylamin, Pyridin und dergleichen. Die Reaktionstemperatur liegt oberhalb Raumtemperatur, vorzugsweise zwischen 60° und der Siedetemperatur des Reaktionsgemisches.

Die Reaktion kann auch stufenweise erfolgen, indem zunächst ein ringoffenes Zwischenprodukt, z.B. der Teilformel $NH_2-C(=NH)-O-CH_2-CO-A-$, entsteht, das dann in der zweiten Stufe durch Erwärmen dehydratisiert wird.

Verfahren d) (Bildung der unsubstituierten Hydroxyiminogruppe):
In einem Ausgangsmaterial der Formel VI sind die funktionellen Gruppen geschützt. Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, z.B. ein Niederalkylester, z.B. Butyl-, Pentyl- oder insbesondere Isoamylnitrit. Verwendet man als Nitrosierungsmittel ein Salz der salpetrigen Säure, so wird die Reaktion vorzugsweise in Gegenwart einer starken anorganischen oder organischen Säure, z.B. Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkanolates durchgeführt.

Die Nitrosierung wird in einem geeigneten Lösungsmittel, wie Wasser, einer Carbonsäure, z.B. Essigsäure, einem Niederalkanol, z.B. Methanol oder Aethanol, einem Aether, z.B. Dioxan oder Tetrahydrofuran, oder einem Kohlenwasserstoff, z.B. Hexan oder Benzol, oder in Mischungen davon, wenn notwendig unter Kühlen oder Erwärmen, insbesondere in einem Temperaturbereich von etwa -15°C bis etwa Raumtemperatur, und/oder in einer Inertgasatmosphäre, z.B. Stickstoffatmosphäre, durchgeführt.

Nachoperationen:

$R_1$-Umwandlungen:

In einer erhaltenen Verbindung der Formel I, worin funktionelle Gruppen gegebenenfalls geschützt sind, kann man in an sich bekannter Weise eine Gruppe $R_1$ durch einen anderen Rest $R_1$ ersetzen oder in einen anderen Rest $R_1$ umwandeln. So kann man beispielsweise in einer Verbindung der Formel I, worin $R_1$ eine Gruppe der Formel $-CH_2-R_2$ bedeutet und $R_2$ z.B. einen, durch nucleophile Substituenten ersetzbaren Rest darstellt, oder in einem Salz davon durch Behandeln mit einer Mercaptanverbindung, z.B. einer Heterocyclylmercaptan-Verbindung, oder mit einer Thiocarbonsäureverbindung einen solchen Rest $R_2$ durch eine verätherte Mercaptogruppe, z.B. Heterocyclylmercaptogruppe, bzw. eine veresterte Mercaptogruppe $R_2$ ersetzen.

Ein geeigneter, durch nucleophile Substituenten, z.B. eine verätherte Mercaptogruppe, ersetzbarer Rest ist beispielsweise eine durch eine niederaliphatische Carbonsäure veresterte Hydroxygruppe. Eine solche veresterte Hydroxygruppe ist insbesondere Acetyloxy und Acetoacetoxy.

Die Reaktion einer solchen Verbindung der Formel I mit einer geeigneten Mercaptanverbindung, z.B. Heterocyclylmercaptan-Verbindung, kann unter sauren, neutralen oder schwach basischen Bedingungen durchgeführt werden. Bei sauren Bedingungen arbeitet man in Gegenwart von konzentrierter Schwefelsäure, welche gegebenenfalls durch ein anorgani-

BAD ORIGINAL

sches Lösungsmittel, z.B. Polyphosphorsäure, verdünnt wird. Bei neutralen oder schwach basischen Bedingungen führt man die Reaktion in Gegenwart von Wasser und gegebenenfalls einem mit Wasser mischbaren organischen Lösungsmittel durch.

Die basischen Bedingungen können beispielsweise durch Zugabe einer anorganischen Base, wie eines Alkalimetall- oder Erdalkalimetallhydroxids, -carbonats oder -hydrogencarbonats, z.B. durch Zugabe von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, z.B. Dimethylformamid, oder Nitrile, z.B. Niederalkansäurenitrile, z.B. Acetonitril, verwendet werden.

In einer Verbindung der Formel I, worin $R_1$ eine Gruppe der Formel $-CH_2-R_2$ bedeutet, worin $R_2$ freies Hydroxy bedeutet, kann man die freie Hydroxygruppe durch den Acylrest einer gegebenenfalls N-substituierten Carbaminsäure verestern. Die Veresterung der freien Hydroxygruppe mit einer Isocyanat-Verbindung, z.B. Halogensulfonylisocyanat, z.B. Chlorsulfonylisocyanat, oder mit einem Carbaminsäurehalogenid, z.B. Carbaminsäurechlorid, führt zu N-unsubstituierten 3-Carbamoyloxymethyl-Cephalosporinen der Formel I. Die Veresterung der freien Hydroxygruppe mit einer N-substituierten Isocyanat-Verbindung oder mit einer N-mono- oder N,N-disubstituierten Carbaminsäure-Verbindung, z.B. einem entsprechend substituierten Carbaminsäurehalogenid, z.B. einem N-mono- oder N,N-disubstituierten Carbaminsäurechlorid, führt zu N-mono- oder N,N-disubstituierten 3-Carbamoyloxymethyl-Cephalosporinen der Formel I. Man arbeitet üblicherweise in Gegenwart eines Lösungs- oder Verdünnungsmittels und, wenn notwendig, unter Kühlen oder Erwärmen, in einem geschlossenen Gefäss und gegebenenfalls unter Inertgas-, z.B. Stickstoffatmosphäre. Die Verbindung der Formel I, worin $R_1$ eine Gruppe der Formel $-CH_2-R_2$ bedeutet, worin $R_2$ freies Hydroxy bedeutet, kann man aus einer Verbindung der Formel I durch Abspaltung des Acetyl-

rests aus einer Acetyloxygruppe $R_2$ herstellen, z.B. durch Hydrolyse in schwach-basischem Medium, z.B. in einer wässrigen Natriumhydroxydlösung bei pH 9-10, oder durch Behandeln mit einer geeigneten Esterase, wie einem entsprechenden Enzym aus Rhizobium tritolii, Rhizobium lupinii, Rhizobium japonicum oder Bacillus subtilis, oder einer geeigneten Citrus-Esterase, z.B. aus Orangenschalen.

Ferner kann man eine Verbindung der Formel I, worin $R_1$ eine Gruppe $-CH_2-R_2$ bedeutet, wobei $R_2$ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedingungen, bevorzugt bei einem pH-Wert von etwa 6,5, in Gegenwart von Wasser und gegebenenfalls in einem mit Wasser mischbaren organischen Lösungsmittel umsetzen und so zu Verbindungen der Formel I gelangen, worin $R_1$ den Rest der Formel $-CH_2-R_2$ und $R_2$ eine Ammoniogruppe darstellt. Die schwach sauren Bindungen können durch Zugabe einer geeigneten organischen oder anorganischen Säure, beispielsweise Essigsäure, Chlorwasserstoffsäure, Phosphorsäure oder Schwefelsäure, eingestellt werden. Als organische Lösungsmittel können beispielsweise die vorstehend genannten, mit Wasser mischbaren Lösungsmittel verwendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder von organischen Säuren, wie Niederalkancarbonsäuren, z.B. Essigsäure. Geeignete Salze sind beispielsweise Natriumjodid, Kaliumjodid und Kaliumthiocyanat. Auch Salze von geeigneten Anionenaustauschern, z.B. flüssige Ionenaustauscher in Salzform, wie z.B. Amberlite LA-1 (flüssige sekundäre Amine mit einem Molekulargewicht von 351-393; Oel-löslich und wasserunlöslich; mAeq./g = 2,5-2,7, z.B. in Acetatform), mit Säuren, z.B. Essigsäure, können für diesen Zweck verwendet werden.

Ammoniogruppen $R_2$ können vorteilhafterweise unter Verwendung eines Zwischenprodukts der Formel I, in welchem $R_2$ für eine substituierte, insbesondere für eine aromatische substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin $R_2$ im Rest $R_1$ eine veresterte Hydroxygruppe, insbesondere eine Niederalkanoyloxy- z.B. Acetyloxygruppe bedeutet, mit einem geeigneten Salz, z.B. dem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegebenenfalls substituierten Pyridin, umgesetzt, wobei man die entsprechende Verbindung mit einer Ammoniogruppe erhält. Die Reaktion wird üblicherweise in Gegenwart eines geeigneten Entschwefelungsmittels, insbesondere eines Quecksilbersalzes, z.B. Quecksilber-IIperchloriat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatmosphäre, durchgeführt.

Oxydation der Hydroxymethylengruppe A:

In einer erhaltenen Verbindung der Formel (I), kann eine Hydroxymethylengruppe A zu einer Oxomethylengruppe oxydiert werden. Die Oxydation kann, gegebenenfalls unter Schutz einer freien Amino- und Carboxylgruppe, auf an sich bekannte, d.h. wie für die Oxydation von Hydroxy- zu Oxogruppen bekannte Weise durchgeführt werden. Als Oxydationsmittel kommen oxydierende Oxide, z.B. des Mangans, Chroms, Stickstoffs oder Schwefels, wie Mangandioxid, Chromtrioxid, z.B. Jones Reagens oder Chromtrioxid in Gegenwart von Essigsäure, Schwefelsäure oder Pyridin, Distickstofftetroxid, Dimethylsulfoxid gegebenenfalls in Gegenwart von Dicyclohexylcarbodiimid oder Sauerstoff, und Peroxide, wie Wasserstoffperoxid, sauerstoffhaltige Säuren, wie Permangansäure, Chromsäure oder Hypochlorsäure oder Salze davon, wie Kaliumpermanganat, Natrium- oder Kaliumdichromat oder

Kaliumhypochlorit, in Frage. Die Hydroxymethylengruppe kann auch durch Oppenauer-Oxydation in die Oxomethylengruppe übergeführt werden, d.h. durch Behandeln mit dem Salz eines sterisch gehinderten Alkohols, wie Aluminium- oder Kalium-tert.-butoxid, -isopropoxid oder -phenoxid in Gegenwart eines Ketons, wie Aceton, Cyclohexanon oder Fluorenon. Eine weitere Möglichkeit die Hydroxymethylengruppe in die Oxomethylengruppe überzuführen besteht in der Dehydrierung, z.B. mit Raney-Nickel.

Die Oxydation wird je nach Oxydationsmittel in Wasser oder einem gegebenenfalls wasserhaltigen organischen Lösungsmittel bei Temperaturen von etwa 0° bis etwa 100° durchgeführt.

Umsetzung der Carbonylgruppe A mit einem Hydroxylaminderivat :

In einer erhaltenen Verbindung der Formel (I), worin A eine Carbonylgruppe bedeutet, kann diese, gegebenenfalls unter Schutz funktioneller Gruppen, durch Behandeln mit Hydroxylamin oder O-Methylhydroxylamin in eine Hydroxyiminomethylen- bzw. Methoxyiminomethylengruppe übergeführt werden.

Die Reaktion der Carbonylgruppe mit der Hydroxylaminverbindung wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, gegebenenfalls in einer Inertgas-, wie Stickstoffatmosphäre, reagieren lässt. Die Hydroxylaminverbindung kann, auch in situ aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyl-diisopropylamin, oder einer heterocyclischen tertiären Base, wie Pyridin, in Freiheit gesetzt werden.

Umwandlung zum 1-Oxid, 1-Dioxid und 1-Sulfid:

Eine Verbindung der Formel I, worin der Index n 0 bedeutet, kann mit den unter Verfahren b) beschriebenen Oxydationsmitteln in das entsprechende 1-Oxid, worin der Index n den Wert 1 hat, umgewandelt werden.

Eine Verbindung der Formel I, worin der Index n 0 oder 1 bedeutet, kann durch Umsetzung mit Sulfid- oder Sulfoxidgruppen in Sulfongruppen überführenden Mitteln in das entsprechende 1-Dioxid, woin n den Wert 2 hat, überführt werden.

Solche Mittel sind insbesondere Wasserstoffperoxid, organische Persäuren, insbesondere aliphatische Percarbonsäuren, z.B. Peressigsäure, Perbenzoesäure, Chlorperbenzoesäure, z.B. m-Chlorperbenzoesäure oder Monoperphthalsäure, oxydierende anorganische Säuren oder deren Salze, z.B. Salpetersäure, Chromsäure, Kaliumpermanganat, oder Alkalimetallhypochlorit, z.B. Natriumhypochlorit, sowie anodische Oxydation. Die Oxydation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, z.B. Diäthyläther, Dioxan oder Tetrahydrofuran, einem Amid, z.B. Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur, oder unter Kühlen oder leichtem Erwärmen, d.h. bei etwa -20 bis etwa +90°, bevorzugt bei etwa +18 bis etwa +30°, durchgeführt. Die Oxydation kann auch stufenweise durchgeführt werden, indem zunächst bei niederer Temperatur, d,h. bei etwa -20° bis etwa 0°, durch Zugabe einer äquivalenten Menge an Oxydationsmittel bis zur Sulfoxidstufe oxydiert wird, das gegebenenfalls isoliert wird, worauf in einem zweiten Schritt, bevorzugt bei höherer Temperatur, durch Zugabe einer weiteren äquivalenten Menge an Oxydationsmittel das Sulfoxid zum

Sulfon, d.h. dem 1,1-Dioxid der Formel (I), oxydiert wird.

Zur Aufarbeitung kann gegebenenfalls noch vorhandenes überschüssiges Oxydationsmittel durch Reduktion, insbesondere durch Behandeln mit einem Reduktionsmittel, wie einem Thiosulfat, z.B. Natriumthiosulfat, zerstört werden.

Ein 1-Oxid der Formel I, worin der Index n den Wert 1 hat, sowie ein 1-Dioxid, worin der Index n den Wert 2 hat, kann mit den unter Verfahren b) beschriebenen Reduktionsmitteln in das entsprechende 1-Sulfid, worin der Index n den Wert 0 hat, umgewandelt werden.

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse, gegebenenfalls stufenweise oder gleichzeigit, freigesetzt werden.

Eine geschützte Carboxylgruppe setzt man in an sich bekannter und je nach Art der Schutzgruppen in verschiedenartiger Weise, vorzugsweise mittels Solvolyse oder Reduktion, frei.
So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol, Anisol oder Aethylenthioglykol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz kann man wie oben beschrieben auch 2-Halogenniederalkoxycarbonyl, gegebenenfalls nach Umwandlung einer 2-Bromniederalkoxycarbonylgruppe in eine entsprechende 2-Jodniederalkoxycarbonylgruppe, oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silgruppe, wie Triniederalkylsilyl, z.B. Trimethylsilyl, verestertes Carboxyl kann üblicherweis solvolytisch, z.B. durch Behandeln mit Wasser, einem Alkohol oder einer Säure freigesetzt werden.

Eine geschützte Aminogruppe setzt man in an sich bekannter und je nach Art der Schutzgruppe in verschiedenartiger Weise, vorzugsweise mittels Solvolyse oder Reduktion, frei. 2-Halogennieder-

BAD ORIGINAL

alkoxycarbonylamino, gegebnenfalls nach Umwandlung einer 2-Bromniederalkoxycarbonylaminogruppe in eine 2-Jodniederalkoxycarbonylaminogruppe, Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino, z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acylniederalk-1-en-1-ylamino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe, z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Eine durch eine geeignete Acylgruppe, eine organische Silylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedinungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

Veresterung einer freien Carboxygruppe: Die Umwandlung einer freien Carboxygruppe, z.B. der freien Carboxygruppe $R_3$, in eine veresterte Carboxygruppe, insbesondere in eine Carboxygruppe, die unter physiologischen Bedingungen spaltbar ist, erfolgt nach an sich bekannten Veresterungsmethoden. Beispielsweise setzt man eine Verbindung der Formel I, worin die zu veresternde Carboxylgruppe in freier Form und andere funktionelle Gruppen, z.B. Amino- oder Hydroxylgruppen, in geschützter Form vorliegen, oder eine Verbindung der Formel I, worin die zu veresternde Carboxygruppe in Form eines reaktionsfähigen, funktionellen Derivates vorliegt, oder ein Salz einer Verbindung der Formel I mit dem entsprechenden Alkohol oder einem reaktionsfähigen, funktionellen Derivat dieses Alkohols um.

Bei der Veresterung einer Verbindung der Formel I, worin die zu veresternde Carboxylgruppe in freier Form vorliegt, mit dem gewünschten Alkohol werden die gleichen Kondensationsmittel, z.B. Carbodiimide, die gleichen Lösungsmittel verwendet und die gleichen Reaktionsbedingungen eingehalten wie bei der Acylierung gemäss Verfahren a).

Eine Verbindung der Formel I, worin die zu veresternde Carboxygruppe in Form eines reaktionsfähigen, funktionellen Derivates vorliegt, ist beispielsweise ein gemischtes Anhydrid oder ein aktivierter Ester, welcher in der unter Verfahren a) (Acylierung) geschilderten Weise durch Kondensation der Carbonsäure der Formel I mit einer anorganischen Säure, einer Carbonsäure, mit einem Halbester der Kohlensäure, einer Sulfonsäure oder durch Kondensation mit einem vinylogen Alkohol erhalten werden kann.

Ein reaktionsfähiges, funktionelles Derivat des zu veresternden Alkohols ist in erster Linie der Ester, welcher durch Kondensation mit einer starken anorganischen oder organischen Säure gebildet wird, beispielsweise das entsprechende Halogenid, z.B. Chlorid, Bromid oder Jodid, oder die entsprechende Niederalkyl-, oder Aryl-, z.B. die Methylsulfonyloxy- oder 4-Methylsulfonyloxyverbindung.

Bei der Veresterung einer Verbindung der Formel I, worin die zu veresternde Carboxylgruppe in Form eines reaktionsfähigen funktionellen Derivats vorliegt, mit dem entsprechenden Alkohol oder bei der Veresterung einer Verbindung der Formel I, worin die zu veresternde Carboxylgruppe in freier Form vorliegt, mit einem reaktionsfähigen, funktionellen Derivat des entsprechenden Alkohols werden die gleichen Lösungsmittel verwendet und die gleichen Reaktionsbedingungen eingehalten wie bei der Acylierung mit einem reaktionsfähigen, funktionellen Derivat einer Carbonsäure der Formel III gemäss Verfahren a).

Eine Verbindung der Formel I, worin die zu veresternde Carboxylgruppe in Form eines reaktionsfähigen, funktionellen Derivats vorliegt, kann man auch analog zu der im Verfahren a) (Acylierung) beschriebenen Methode <u>in situ</u> herstellen und ohne Isolierung mit dem entsprechenden Alkohol umsetzen.

<u>Salzbildung:</u> Salze von Verbindungen der Formel I können in an sich bekannter Weise hergestellt werden. So kann man Salze von Verbindungen der Formel I, z.B. durch Reaktion der sauren Gruppen mit Metallverbindungen, wie Alkalimetallsalzen von geeigneten Carbonsäuren, z.B. dem Natriumsalz der α-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Bei sämtlichen weiter vorn genannten Umsetzungen die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Pharmazeutische Präparate:

Die pharmakologisch verwendbaren Verbindungen der Formel I, deren Hydrate oder Salze können zur Herstellung von pharmazeutischen Präparaten verwendet werden.

Pharmazeutische Präparate enthalten eine wirksame Menge des reinen Wirkstoffs der Formel I selbst oder eine wirksame Menge des Wirkstoffs der Formel I im Gemisch mit anorganischen oder organischen, festen oder flüssigen, pharmazeutisch verwendbaren Trägerstoffen, die sich vorzugsweise zur parenteralen Verabreichung eignen.

Vorzugsweise verwendet man die Wirkstoffe der Formel I der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebrauch hergestellt werden können. Die pharmazeutischen Präparate sind vorzugsweise sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, enthalten von etwa 0,1% bis 100 %, insbesondere von etwa 1% bis etwa 50%, Lyophilisate bis zu 100% des Aktivstoffes.

Die pharmazeutischen Präparate werden in an sich bekannter Weise, z.B. mittels konventioneller Lösungs- oder Lyophilisierungsverfahren, hergestellt.

Verwendung:

Verbindungen der Formel I, deren Hydrate oder pharmazeutisch verwendbare Salze können als antibiotisch wirksame Mittel in Form von pharmazeutischen Präparaten in einem Verfahren zur therapeutischen Behandlung des menschlichen oder tierischen Körpers angewendet werden, z.B. zur Behandlung von Infektionen, welche durch grampositive oder gramnegative Bakterien und Kokken, z.B. durch Enterobakterien, z.B. Escherichia coli, Klebsiella pneumoniae oder Proteus spp., verursacht werden.

Je nach Art der Infektionen und Zustand des infizierten Organismus verwendet man tägliche Dosen von etwa 0,5 g bis etwa 5 g s.c., i.v. oder i.m. zur Behandlung von Warmblütern (Menschen und Tiere) von etwa 70 kg Gewicht.

Ausgangsstoffe:

Die im Verfahren zur Herstellung der Verbindungen der vorliegenden Erfindung verwendeten Ausgangsstoffe sind bekannt oder können, falls sie neu sind, in an sich bekannter Weise hergestellt werden.

Ausgangsmaterialien der Formel II, sowie entsprechende Verbindungen mit geschützten funktionellen Gruppen, sind bekannt oder können auf an sich bekannte Weise hergestellt werden.

Ausgangsmaterialien der Formel III, sowie deren Ester und reaktionsfähigen Derivate sind neu und sind ebenfalls Gegenstand der vorliegenden Erfindung.

Die zur Einführung des Acylrestes einer Carbonsäure der Formel III verwendete Carbonsäure der Formel III bzw. deren reaktionsfähigen funktionellen Derivate können in an sich bekannter Weise hergestellt werden. Sie werden beispielsweise erhalten, indem man eine Verbindung der Formel

$$X-CH_2-\overset{O}{\overset{\|}{C}}-A-COOH \qquad (VII)$$

worin X Halogen bedeutet und A die unter Formel I genannten Bedeutungen hat und die Carboxylgruppe in veresterter Form vorliegt bzw. durch eine übliche Carboxylschutzgruppe geschützt ist, mit Harnstoff umsetzt und, wenn erwünscht, in einer erhältlichen Verbindung, die Carboxylschutzgruppe abspaltet und/oder die in 2-Stellung des Oxazolylrestes befindliche Aminogruppe schützt und/oder eine in A befindliche funktionelle Gruppe schützt und/oder die Gruppe A in eine andere Gruppe A umwandelt.

In einer Verbindung der Formel VII ist X bevorzugt Chlor oder Brom, ferner Jod oder Fluor. Die Carboxylgruppe liegt in veresterter Form vor, z.B. als Methyl- oder Aethylester, bzw. ist durch eine der vorstehend genannten üblichen Schutzgruppen, z.B. durch den tert.-

Butylrest geschützt. Die Umsetzung einer Verbindung der Formel VII mit Harnstoff lässt sich im wesentlichen unter den gleichen im Verfahren c) genannten Bedingungen durchführen, wobei Dimethylformamid als Lösungsmittel bevorzugt ist. Vorzugsweise arbeitet man bei Temperaturen oberhalb von etwa 80° bis zum Siedepunkt des Reaktionsgemisches. Es lassen sich Verbindungen der Formel VII mit Harnstoff umsetzen, worin A beispielsweise Carbonyl, unsubstituiertes oder durch eine Gruppe der Formel $=N-O-R_4$, beispielsweise durch Methoxyimino, substituiertes Methylen darstellt.

Die in einer erhältlichen Verbindung der Formel III in 2-Stellung des Oxazolylrests befindliche Aminogruppe kann man anschliessend in eine z.B. durch tert.-Butyloxycarbonyl geschützte Aminogruppe überführen. Soll bei der Acylierungsreaktion gemäss Verfahren a) die freie Carbonsäure der Formel III eingesetzt werden, wandelt man vorher die geschützte Carboxylgruppe in eine freie Carboxylgruppe um. In einer Verbindung der Formel III lässt sich eine Carbonylgruppe A mit einer Hydroxylaminverbindung der Formel $H_2N-O-R_4$ in eine durch eine Gruppe der Formel $=N-O-R_4$ substituierte Methylengruppe A analog den oben unter Nachoperationen beschriebenen Verfahren nachträglich umwandeln. In einer Verbindung der Formel III lässt sich eine unsubstituierte Methylengruppe A mit einem geeigneten Nitrosierungsmittel, z.B. Natriumnitrit, in eine durch eine Hydroxyiminogruppe $=N-O-H$ substituierte Methylengruppe A analog den oben unter Nachoperationen beschriebenen Verfahren nachträglich umwandeln.

Verbindungen der Formel IV bis VII sind bekannt oder können, falls sie neu sind, nach an sich bekannten Verfahren hergestellt werden.

Neue Ausgangsverbindungen, sowie Zwischenprodukte und Verfahren zu ihrer Herstellung sind ebenfalls Gegenstand der vorliegenden Erfindung.

Die folgenden Beispiele dienen zur Illustration der Erfindung; Temperaturen werden in Celsiusgraden angegeben; BOC: tert.-Butyloxycarbonyl.

Beispiel 1:

a) 3-Acetoxymethyl-7β-[2-(2-amino-4-oxazolyl)-2-Z-methoxyiminoacetylamino]-3-cephem-4-carbonsäure-natriumsalz

Man rührt bei Raumtemperatur 0,26 g 3-Acetoxymethyl-7β-[2-(2-BOC-amino-4-oxazolyl)-2-Z-methoxyiminoacetylamino]-3-cephem-4-carbonsäurediphenylmethylester in 1,1 ml Methylenchlorid eine Stunde lang mit 1,1 ml Trifluoressigsäure und 0,2 ml Anisol, versetzt mit 50 ml eiskaltem Toluol und dampft anschliessend im Vakuum ein. Nach Digerieren des Rückstands mit Aether und Trocknen erhält man das entsprechende Trifluoressigsäuresalz als gelbliches Pulver. Man nimmt das Salz in 5 ml Methanol auf und versetzt anschliessend mit 0,2 ml einer 3-molaren methanolischen Natriumäthylhexanoatlösung. Zwecks Fällung des Natriumsalzes gibt man Diäthyläther hinzu, filtriert den ausgefallenen Niederschlag ab, wäscht mit Aether nach und trocknet. Man erhält die Titelverbindung in Form eines leicht beigen Pulvers. $R_f \sim 0.23$ (Silicagel; n-Butanol/Essigsäure/Wasser; 67:10:23); UV ($H_2O$): $\lambda_{max}$ = 213 nm (17200) und 261 nm (12100).

b) 3-Acetoxymethyl-7β-[2-(2-BOC-amino-4-oxazolyl)-2-Z-methoxyiminoacetylamino]-3-cephem-4-carbonsäurediphenylmethylester

Zu 0,1 ml Oxalylchlorid in 4 ml Methylenchlorid gibt man bei -10° 0,09 ml Dimethylformamid, lässt 30 Minuten rühren und versetzt anschliessend mit 2-(2-BOC-amino-4-oxazolyl)-2-Z-methoxyiminoessigsäure und 0,14 ml N-Methylmorpholin. Man lässt 30 Minuten bei ca. -5° bis -10° weiterrühren, versetzt das Reaktionsgemisch mit einer Lösung bestehend aus 526 mg 3-Acetoxymethyl-7β-amino-3-cephem-4-carbonsäure-diphenylmethylester und 0,14 ml N-Methylmorpholin in 5 ml Methylenchlorid und lässt zwei Stunden bei ca. 0° reagieren. Zur Aufarbeitung engt man das Reaktionsgemisch ein, nimmt den Rückstand in Essigsäureäthylester auf und schüttelt nacheinander mit ca. 0,5 N Salzsäure, gesättigter Natriumhydrogencarbonat- und gesättigter Kochsalzlösung aus. Nach Trocknen der organischen Phase über Natriumsulfat und Eindampfen erhält man die Titelverbindung als Rohprodukt, welche man durch präparative Dickschichtchromatographie (Silicagel, Essigsäureäthylester ) reinigt. $R_f$ ca. 0,53 (Silicagel; Essigsäureäthylester); IR (Methylenchlorid): 3400, 1785, 1758b, 1740sh, 1680 und 1630 $cm^{-1}$.

Man stellt die Ausgangsmaterialien folgendermassen her:

c) 2-(2-BOC-Amino-4-oxazolyl)-2-Z-methoxyiminoessigsäure

Man versetzt 1 g 2-(2-BOC-Amino-4-oxazolyl)-2-Z-methoxyiminoessigsäuremethylester in 20 ml Aethanol mit einer Lösung von 1,2 g Kaliumhydroxid in 10 ml Wasser und rührt 50 Minuten bei Raumtemperatur.
Man engt anschliessend das Reaktionsgemisch im Vakuum ein, verdünnt mit Essigester uns säuert mit 1 N Salzsäure auf pH 1-2 an.
Man trennt die organische Phase ab, schüttelt mit gesättigter Kochsalzlösung aus, trocknet über Natriumsulfat und dampft ein. Nach Digerieren des Rückstands mit einem Gemisch aus Essigsäureäthylester und Hexan erhält man die Titelverbindung als gelbliches Pulver.
$R_f$ ca. 0,38 (Silicagel; n-Butanol/Essigsäure/Wasser; 67:10:23);
IR (Nujol): 3400b, 1735b, 1710 und 1640 $cm^{-1}$.

d) 2-(2-BOC-Amino-4-oxazolyl)-2-Z-methoxyiminoessigsäuremethylester

Man suspendiert 2,0 g 2-(2-Amino-4-oxazolyl)-2-Z-methoxyimino-essigsäuremethylester in 3,05 g Di-tert.-butyldicarbonat, erwärmt auf 100° und versetzt mit 0,1 g 4-Dimethylaminopyridin. Man lässt 7 Minuten bei 100° reagieren, kühlt ab und verdünnt das Reaktionsgemisch mit Essigsäureäthylester, extrahiert zweimal mit gesättigter Kochsalz-Lösung, trocknet die organische Phase über Natriumsulfat und dampft im Vakuum ein. Man chromatographiert das Rohprodukt an 100 g Kieselgel, wobei man mit Toluol und 15 % Essigsäureäthylester die Titelverbindung eluiert, welche man anschliessend aus Aether auskristallisiert. Schmelzpunkt: 142 - 146°C; IR ($CH_2Cl_2$): 3415, 1740, 1715sh, 1638, 1515 $cm^{-1}$; UV (EtOH): $\lambda_{max}$ = 227 nm (16000) und 261 nm (7900).

e) 2-(2-Amino-4-oxazolyl)-2-Z-methoxyiminoessigsäuremethylester

Man lässt 47,6 g 4-Bromo-2-methoxyiminoacetessigsäuremethylester und 120 g Harnstoff in 480 ml Dimethylformamid 1 Stunde auf 100° erwärmen, versetzt mit zusätzlichen 30 g Harnstoff und lässt eine weitere Stunde bei 100° reagieren. Man giesst das dunkle Reaktionsgemisch auf Eiswasser, extrahiert mit Essigsäureäthylester erschöpfend, schüttelt die organische Phase dreimal mit Wasser und zweimal mit gesättigter Kochsalz-Lösung und trocknet über Natriumsulfat. Nach Eindampfen im Vakuum kristallisiert man die Titelverbindung aus einem Essigsäureäthylester-Diäthyläther Gemisch aus. Schmelzpunkt: 142 - 147°; IR (Nujol): u.a. 3438, 3181, 1701, 1680, 1588 $cm^{-1}$.

Beispiel 2:

a) 7β-[2-(2-Amino-4-oxazolyl)-2-Z-methoxyiminoacetylamino]-3-cephem-4-carbonsäure-natriumsalz

Man rührt bei Raumtemperatur 0,43 g 7β-[2-(2-BOC-Amino-4-oxazolyl)-2-Z-methoxyiminoacetylamino]-3-cephem-4-carbonsäurediphenylmethylester in 2,05 ml Methylenchlorid eine Stunde lang mit 2,05 ml Trifluoressigsäure und 0,37 ml Anisol, versetzt mit 50 ml eiskaltem Toluol und dampft anschliessend im Vakuum ein. Nach Digerieren des Rückstands mit Aether und Trocknen erhält man das entsprechende Trifluoressigsäuresalz als gelbliches Pulver. Man nimmt das Salz in 10 ml Methanol auf und versetzt anschliessend mit 0,4 ml einer 3-molaren methanolischen Natriumäthylhexanoatlösung. Zwecks Fällung des Natriumsalzes gibt man Diäthyläther hinzu, filtriert den ausgefallenen Niederschlag ab, wäscht mit Aether nach und trocknet. Man erhält die Titelverbindung in Form eines beigen Pulvers. $R_f$ ca. 0,24 (Silicagel: n-Butanol/Essigsäure/Wasser; 67:10:23); UV (in Wasser): $\lambda_{max}$ = 205 nm (17600) und 260 nm (8400).

b) 7β-[3-(2-BOC-Amino-4-oxazolyl)-2-Z-methoxyiminoacetylamino]-3-cephem-4-carbonsäurediphenylmethylester

Zu 0,2 ml Oxalylchlorid in 8 ml Chloroform gibt man bei -10° 0,18 ml Dimethylformamid, lässt 30 Minuten stehen und versetzt anschliessend mit 570 mg 2-(2-BOC-Amino-4-oxazolyl)-2-Z-methoxyiminoessigsäure und 0,28 ml N-Methylmorpholin. Man lässt 30 Minuten bei ca. -5° bis -10° weiterrühren. Man versetzt das Reaktionsgemisch mit einer Lösung bestehend aus 0,84 g 7β-Amino-3-cephem-4-carbonsäurediphenylmethylester und 0,28 ml N-Methylmorpholin in 10 ml Methylenchlorid und lässt anschliessend 45 Minuten bei 0 bis 5° weiterreagieren.

Zur Aufarbeitung engt man das Reaktionsgemisch ein, nimmt den Rückstand in Essigsäureäthylester auf und schüttelt nacheinander mit ca. 0,5 N Salzsäure, gesättigter Natriumhydrogencarbonat- und gesättigter Kochsalzlösung aus. Nach Trocknen der organischen Phase über Natriumsulfat und Eindampfen erhält man die Titelverbindung als Rohprodukt, welche man durch präparative Dickschichtchromatographie (Silicagel, Essigsäureäthylester) reinigt. $R_f$ ca. 0,3 (Silicagel; Essigsäureäthylester/Toluol; 1:1); IR (in Methylenchlorid): Banden bei 3402, 1788, 1755, 1730, 1680, 1628 und 1512 $cm^{-1}$; UV (in Aethanol): $\lambda_{max}$ = 260 nm (7000 sh); 215 nm (sh).

Beispiel 3:

a) 7β-[2-(2-Amino-4-oxazolyl)-2-Z-methoxyiminoacetylamino]-3-cephem-4-carbonsäurepivaloyloxymethylester

Eine Mischung von 0,11 ml Chlormethylpivalat und 0,45 g Natriumjodid in 1,5 ml Aceton wird 3 Stunden bei Raumtemperatur gerührt. Die Suspension wird anschliessend mit 0,10 g 7β-[-(2-Amino-4-oxazolyl)-2-Z-methoxyiminoacetylamino]-3-cephem-4-carbonsäure-natriumsalz in 3 ml Dimethylformamid versetzt und 1 Stunde bei Raumtemperatur weitergerührt. Das Reaktionsgemisch wird mit Aethylacetat verdünnt, mit gesättigter wässeriger Natriumchlorid-Lösung ausgeschüttelt, über Natriumsulfat getrocknet und im Vakuum eingedampft. Der Rückstand wird an Silicagel mit Toluol-Aethylacetat 1:1 und Aethylacetat chromatographiert und ergibt den 7β-[2-(2-Amino-4-oxazolyl)-2-Z-methoxy-iminoacetylamino]-3-cephem-4-carbonsäurepivaloyloxymethylester.

b) 7β-[2-(2-Amino-4-oxazolyl)-2-Z-methoxyiminoacetylamino]-3-cephem-4-carbonsäurepivaloyloxymethylester-hydrochlorid

Der Pivaloyloxymethylester wird durch Behandeln mit einer gesättigten HCl-Lösung in Methylenchlorid und Fällen mit Aether in das Hydrochloridsalz überführt.

$R_f \sim 0,25$ (Aethylacetat); IR ($CH_2Cl_2$): Banden bei 3490, 3380, 1780, 1751, 1677, 1620 $cm^{-1}$.

Beispiel 4:

a) 7β-[2-(2-Amino-4-oxazolyl)-2-Z-methoxyiminoacetylamino-3-cephem-4-carbonsäure-1-(äthyloxycarbonyloxy)-äthylester

Ein Gemisch von 0.75 g 7β-[2-(2-BOC-amino-4-oxazolyl)-2-Z-methoxyiminoacetylamino]-3-cephem-4-carbonsäure-1-(äthyloxycarbonyloxy)-äthylester, 3,9 ml Trifluoressigsäure und 3,9 ml Methylenchlorid wird 1 Stunde bei Raumtemperatur gerührt, mit kaltem Toluol versetzt und unter vermindertem Druck eingedampft. Man versetzt den Rückstand mit einem Toluol/Chloroform-Gemisch, dampft ein, versetzt mit einem Toluol/Aether-Gemisch und dampft nochmals ein. Man kristallisiert den Rückstand aus Aether. Das Trifluoracetat der Titelverbindung schmilzt bei 147°-150° unter Zersetzung; IR-Spektrum (Nujol): Banden bei 1790, 1745, 1730, 1670, 1640 und 1540 $cm^{-1}$. Das Trifluoracetat-Salz wird in Essigester aufgenommen und zweimal mit kalter, gesättigter Natriumbicarbonatlösung gewaschen. Nach Trocknen der organischen Phase über Natriumsulfat wird die Lösung unter reduziertem Druck eingeengt. Man chromatographiert den Rückstand (Kieselgel 60; Methylenchlorid/Aethanol) und erhält die Titelverbindung mit einem $R_f$-Wert von 0,62 (Kieselgel 60; Methylenchlorid/Aethanol 9:1).

b) 7β-[2-(2-BOC-amino-4-oxazolyl)-2-Z-methoxyiminoacetylamino]-3-cephem-4-carbonsäure-1-(äthyloxycarbonyloxy)-äthylester

Der als Ausgangsmaterial verwendete 7β-[2-(2-BOC-amino-4-oxazolyl)-2-Z-methoxyimino-acetylamino]-3-cephem-4-carbonsäure-(1-äthoxycarbonyloxyäthyl)-ester kann wie folgt hergestellt werden.

Eine Lösung von 0,45 g 1-Chloräthyl-äthylcarbonat in 10 ml Aceton wird mit 1.8 g Natriumjodid versetzt. Man rührt 2 1/2 Stunden bei Raumtemperatur und gibt dann eine Lösung von 1,16 g 7β-[2-(2-BOC-amino-4-oxazolyl)-2-Z-methoxyimino-acetylamino]-3-cephem-4-carbonsäure und 0,38 g 1,5-Diazabicyclo[5,4,0]undec-5-en in 20 ml Aceton zu. Das Reaktionsgemisch wird 4 Stunden bei Raumtemperatur gerührt, mit Essigester verdünnt und mit gesättigter Natriumchlorid-Lösung gewaschen. Die organische Phase wird über Natriumsulfat getrocknet und unter vermindertem Druck eingedampft. Der Rückstand wird säulenchromatographisch (Kieselgel 60; Methylenchlorid/Aethanol) gereinigt.

## Beispiel 5:

a) 3-(1-Methyl-1H-tetrazol-5-ylthiomethyl)-7β-[2-(2-amino-4-oxazolyl)-2-Z-methoxyiminoacetylamino]-3-cephem-4-carbonsäure-natriumsalz

Man rührt bei Raumtemperatur 5,5 g 3-(1-Methyl-1H-tetrazol-5-ylthiomethyl)-7β-[2-(2-BOC-amino-4-oxazolyl)-2-Z-methoxyiminoacetylamino]-3-cephem-4-carbonsäurediphenylmethylester in 22 ml Methylenchlorid 1 Stunde lang mit 22 ml Trifluoressigsäure und 3,9 ml Anisol, versetzt mit 200 ml eiskaltem Toluol und dampft im Vakuum ein. Nach Digerieren des Rückstands und Trocknen in Aether erhält man das entsprechende Trifluoressigsäuresalz als gelbliches Pulver. Man suspendiert das Salz in 50 ml Wasser und stellt den pH durch Zugabe von 1 N NaOH-Lösung auf 7,0 ein. Man chromatographiert die trübe Lösung an einer Säule mit 4 cm Durchmesser gefüllt mit 400 ml Amberlite XAD-2® Adsorberharz. Nach Eluieren mit 12 %-iger wässriger Isopropanollösung, Vereinigen der dünnschichtchromatographisch einheitlichen, produkthaltigen Fraktionen und Lyophilisieren erhält man die Titelverbindung. $R_f \sim 0.2$ (Silicagel; n-Butanol/Eisessig/Wasser; 67:10:23); IR (Nujol): u.a. 3330, 1770, 1667, 1606 $cm^{-1}$, UV ($H_2O$): $\lambda_{max}$ = 270 nm (15000).

b) 3-(1-Methyl-1H-tetrazol-5-ylthiomethyl)-7β-[2-(2-BOC-amino-4-oxazolyl)-2-Z-methoxyiminoacetylamino]-3-cephem-4-carbonsäurediphenylmethylester

Zu 1,26 ml Dimethylformamid in 14 ml Methylenchlorid gibt man bei ca. -10° 1,40 ml Oxalylchlorid in 42 ml Methylenchlorid, rührt 45 Minuten lang bei -5° bis -10° und versetzt anschliessend mit 3,99 g 2-(2-BOC-Amino-4-oxazolyl)-2-Z-methoxyiminoessigsäure und 1,96 ml N-Methylmorpholin. Man lässt 30 Minuten bei -5° bis -10° weiterrühren. Anschliessend versetzt man dieses Reaktionsgemisch mit einer Lösung hergestellt aus 7,3 g 3-(1-Methyl-1H-tetrazol-5-ylthiomethyl)-7β-amino-3-cephem-4-carbonsäurediphenylmethylester und 1,96 ml N-Methylmorpholin in 70 ml Methylenchlorid und lässt 45 Minuten bei 0° bis 5° reagieren. Zur Aufarbeitung engt man das Reaktionsgemisch ein, nimmt in Essigester auf und schüttelt nacheinander mit 0,5 N Salzsäure, gesättigter $NaHCO_3$-Lösung und gesättigter Kochsalzlösung aus. Nach Trocknen über Natriumsulfat und Eindampfen erhält man das Rohprodukt, welches man an 200 g Kieselgel chromatographiert. Nach Eluieren mit Toluol und Toluollösung mit steigenden Anteilen Essigester (10 % - 25 %) und Eindampfen erhält man die Titelverbindung als gelben Schaum. $R_f \sim 0.55$ (Essigester; Silicagel); IR ($CH_2Cl_2$): u.a. 3410, 1793, 1758, 1730, 1684, 1632 $cm^{-1}$; UV (EtOH): $\lambda_{max}$ = 261 nm (16200).

Beispiel 6:

a) 3-(1-Carboxymethyl-1H-tetrazol-5-ylthiomethyl)-7β-[2-(2-amino-4-oxazolyl)-2-Z-methoxyiminoacetylamino]-3-cephem-4-carbonsäure-dinatriumsalz

3,5 g 3-(1-Carboxymethyl-1H-tetrazol-5-ylthiomethyl)-7β-[2-(2-BOC-amino-4-oxazolyl)-2-Z-methoxyiminoacetylamino)-3-cephem-4-carbonsäure werden bei Raumtemperatur eine Stunde lang in 16,6 ml Methylenchlorid und 16,6 ml Trifluoressigsäure gerührt und mit 100 ml eiskaltem Toluol versetzt. Anschliessend dampft man ein, digeriert

den Rückstand mit Aether, filtriert ab und trocknet. Man suspendiert das Trifluoressigsäuresalz in Wasser, bringt die Lösung auf pH 7 und chromatographiert die Lösung an Amberlite XAD-2 Adsorberharz. Nach Eluieren mit Wasser, Vereinigen der dünnschichtchromatographisch einheitlichen Fraktionen und Lyophilisieren erhält man die reine Titelverbindung. $R_f \sim 0.55$ (Silyliertes Kieselgel $UPC_{12}$; Wasser/Acetonitril; 95:5); IR (Nujol): u.a. 3325b, 1768, 1665, 1628 $cm^{-1}$; UV ($H_2O$): $\lambda_{max}$ = 268 nm (14000).

b) 3-(1-Carboxymethyl-1H-tetrazol-5-ylthiomethyl)-7ß-[2-(2-BOC-amino-4-oxazolyl)-2-Z-methoxyiminoacetylamino)-3-cephem-4-carbonsäure-dinatriumsalz

Zu 0,92 ml Dimethylformamid in 13 ml Methylenchlorid gibt man bei -25° tropfenweise innerhalb von 10 Minuten eine Lösung von 1,15 ml Oxalylchlorid in 13 ml Methylenchlorid, lässt 30 Minuten bei -25° rühren und versetzt anschliessend mit einer Lösung von 2,85 g 2-(2-BOC-Amino-4-oxazolyl)-2-Z-methoxyiminoessigsäure und 1,34 ml N-Methylmorpholin in 28 ml Methylenchlorid. Man lässt 30 Minuten bei -25° weiterrühren (Lösung A).

Eine Suspension von 4,48 g 3-(1-Carboxymethyl-1H-tetrazol-5-ylthiomethyl)-7β-amino-3-cephem-4-carbonsäure in 40 ml Methylenchlorid wird 1,5 Stunden mit 11,70 ml Bis-(trimethylsilyl)-acetamid (BSA) bei Raumtemperatur gerührt, mit 1,60 ml N-Methylmorpholin versetzt und auf -25° . gekühlt (Lösung B).

Man tropft Lösung B zu Lösung A und lässt das Gemisch anschliessend drei Stunden lang bei Raumtemperatur reagieren. Zur Aufarbeitung giesst man das Reaktionsgemisch auf Eiswasser, stellt den pH-Wert mit 1 N Natronlaugelösung auf pH 7 und schüttelt die wässrige Phase dreimal mit Essigester aus. Nach Trennung der Phasen wird die wässrige Phase noch zweimal mit einem Essigester-Aceton (4:1)-Gemisch extrahiert. Man vereinigt die organischen Phasen, schüttelt mit gesättigter Kochsalzlösung aus, trocknet über Natriumsulfat und dampft ein. Man erhält die Titelverbindung als braunes Pulver. Zur Reinigung wird das Rohprodukt an 100 g Kieselgel, welches durch Zugabe von 5 % Wasser vorher desaktiviert wurde, chromatographiert. Nach Eluieren mit Essigester enthaltend 25 % $CH_2Cl_2$, reinem Essigester und Essigester enthaltend 10 % Aceton erhält man die Titelverbindung. $R_f$ ~0.2 (Silicagel; n-Butanol/Eisessig/Wasser; 67:10:23);

Beispiel 7: 3-[1-(2-Dimethylaminoäthyl)-1H-tetrazol-5-ylthiomethyl]-7β-[2-(2-amino-4-oxazolyl)-2-Z-methoxyiminoacetylamino]-3-cephem-4-carbonsäure-dihydrochlorid

Zu einer auf 70° erwärmten Lösung von 29,96 g Natriumjodid und 2,25 g 1-(2-Dimethylaminoäthyl)-1H-tetrazol-5-ylmercaptan in 15 ml Wasser und 0,93 ml Eisessig werden 4,62 g 3-Acetoxymethyl-7β-[2-(2-amino-4-oxazolyl)-2-Z-methoxyiminoacetylamino]-3-cephem-4-carbonsäure-natriumsalz (Beispiel 1a) gegeben. Man rührt das Gemisch 75 Minuten lang bei 70° unter Stickstoffatmosphäre. Anschliessend giesst man die braungefärbte Reaktionslösung auf 200 ml Eiswasser, stellt mit 1 N Natronlauge auf pH 5,5 ein und chromatographiert an einer Säure gefüllt mit Amberlite XAD-2® Adsorberharz. Nach Eluieren mit Wasser und einer wässrigen Lösung mit steigenden Isopropanolanteilen (5-15 %) und Vereinigen der dünnschichtchromatographisch einheitlichen Fraktionen erhält man die Titelverbindung und die entsprechende 2-E-Verbindung. Nach erneutem Chromatographieren an silyliertem Kieselgel (Opti $UPC_{12}$) und Eluieren mit einem Wasser/Acetonitril (9:1)-Gemisch erhält man die dünnschichtchromatographisch einheitliche Titelverbindung und die entsprechende 2-E-Verbindung, welche man jeweils in Methylenchlorid aufnimmt, mit HCl-haltigem Methylenchlorid behandelt und in Aether zutropft. Man filtriert den ausgefallenen Niederschlag ab und trocknet. 2-Z-Verbindung: $R_f$ ~0.45 (Silicagel OPTI UPC-12; $H_2O$/Acetonitril; 4:1); IR (Nujol): u.a. 3320, 1782, 1718, 1680, 1635 $cm^{-1}$; UV ($H_2O$): $\lambda_{max}$ = 270 nm (14500); NMR (d-DMSO): Oxazol-H bei $\delta$ = 7.75, N-$OCH_3$ bei $\delta$ = 3.95. 2-E-Verbindung: $R_f$ ~0.25 (Silicagel OPTI UPC-12;

$H_2O$/Acetonitril; 4:1); IR (Nujol): u.a. 330, 1784, 1718, 1682, 1632 $cm^{-1}$; UV ($H_2O$): 270sh (6500); NMR (d-DMSO): Oxazol-H bei $\delta$ = 8.35, $CH_3O$-N bei $\delta$ = 4.15.

Beispiel 8: 3-(4-Carbamoylpyridiniomethyl)-7β-[2-(2-amino-4-oxazolyl)-2-Z-methoxyiminoacetylamino]-3-cephem-4-carboxylat

Zu einer auf 70° erwärmten Lösung von 22,5 g Natriumjodid und 1,24 g Isonicotinamid in 11,25 ml Wasser und 0,43 ml Eisessig werden 3,46 g 3-Acetoxymethyl-7β-[2-(2-amino-4-oxazolyl)-2-Z-methoxyiminoacetyl-amino]-3-cephem-4-carbonsäure-natriumsalz (Beispiel 1a) gegeben. Man rührt das Reaktionsgemisch eine Stunde lang bei 70° unter Stickstoffatmosphäre. Nach Abkühlen stellt man den pH-Wert der braungefärbten Reaktionslösung mit 1 N Natronlauge auf 5,2 ein und chromatographiert an einer Säule gefüllt mit Amberlite XAD-2® Adsorberharz. Nach Eluieren mit einer 12 %igen wässrigen Isopropanollösung und Vereinigen der dünnschichtchromatographisch einheitlichen, produkthaltigen Fraktionen und Lyophilisieren erhält man die Titelverbindung als gelbliches Lyophilisat. $R_f$: 0.6 (silyliertes Kieselgel $UPC_{12}$; Wasser/Acetonitril; 4:1); IR (Nujol): u.a. 3340b, 1776, 1655, 1615 $cm^{-1}$; UV ($H_2O$): 212 nm (22300), 261 nm (15400).

Beispiel 9:

a) 3-Acetoxymethyl-7β-[2-(2-amino-4-oxazolyl)-2-Z-hydroxyimino-acetylamino]-3-cephem-4-carbonsäure-natriumsalz

Man rührt eine Stunde lang bei Raumtemperatur 4,2 g 3-Acetoxymethyl-7β-[2-(2-BOC-amino-4-oxazolyl)-2-Z-(2-tetrahydropyranyloxyimino)-acetylamino]-3-cephem-4-carbonsäurediphenylmethylester in 16 ml Methylenchlorid mit 16,5 ml Trifluoressigsäure und 2,8 ml Anisol, versetzt das braungefärbte Reaktionsgemisch mit 100 ml eiskaltem Toluol und dampft im Vakuum ein. Nach Trocknen und Digerieren des Rückstands in Aether erhält man das entsprechende Trifluoressigsäuresalz als gelbliches Pulver. Man suspendiert das Salz in 50 ml Wasser und

stellt den pH-Wert durch Zugabe von 2 N Natronlauge auf 7.4 ein. Man chromatographiert die trübe Lösung an einer Säule mit 3,5 cm Durchmesser gefüllt mit Amberlite XAD-2 ® Adsorberharz. Nach Eluieren mit einer 12 %igen wässrigen Isopropanollösung, Vereinigen der dünnschichtchromatographisch einheitlichen, produkthaltigen Fraktionen und Lyophiliseren erhält man die Titelverbindung. $R_f \sim 0.45$ (silyliertes Kieselgel OPTI-UPC$_{12}$; Wasser/Acetonitril; 4:1); IR (Nujol): u.a. 3325b, 1770, 1665, 1608 $cm^{-1}$; UV ($H_2O$): 209 (20000); 260 (12200).

b) <u>3-Acetoxymethyl-7β-[2-(2-BOC-amino-4-oxazolyl)-2-Z-(2-tetrahydropyranyloxyimino)-acetylamino]-3-cephem-4-carbonsäurediphenylmethylester</u>

Zu 0,9 ml Dimethylformamid in 40 ml Methylenchlorid gibt man bei -5° 1,0 ml Oxalylchlorid in 30 ml Methylenchlorid, rührt 20 Minuten lang bei -5° bis -10° und versetzt anschliessend mit 3,55 g 2-(2-BOC-Amino-4-oxazolyl)-2-Z-(2-tetrahydropyranyloxyimino)-essigsäure und 1,4 ml N-Methylmorpholin. Man lässt 30 Minuten bei -10° weiterrühren.

Man versetzt das Reaktionsgemisch anschliessend mit einer Lösung aus 4,39 g 3-Acetoxymethyl-7β-amino-3-cephem-4-carbonsäurediphenylmethylester und 1,4 ml N-Methylmorpholin in 44 ml Methylenchlorid und lässt 30 Minuten bei -5° und 75 Minuten bei 0° reagieren. Zur Aufarbeitung engt man das Reaktionsgemisch ein, nimmt in Essigester auf und schüttelt nacheinander mit 0,5 N Salzsäure, gesättigter $NaHCO_3$-Lösung und gesättigter Kochsalzlösung aus. Nach Trocknen über Natriumsulfat und Eindampfen erhält man das Rohprodukt, welches man an 250 g Kieselgel chromatographiert. Nach Eluieren mit Toluol und Toluollösung mit steigenden Anteilen Essigester (10 % - 50 %) und Vereinigen der dünnschichtchromatographisch einheitlichen, produkthaltigen Fraktionen und Eindampfen erhält man die Titelverbindung. $R_f \sim 0.5$ (Silicagel; Essigester); IR ($CH_2Cl_2$): u.a. 3410, 1796, 1748, 1683, 1631 $cm^{-1}$; UV (EtOH): $\lambda_{max}$ = 258 nm (15100).

Man stellt die Ausgangsmaterialien folgendermassen her:

c) 2-(2-BOC-Amino-4-oxazolyl)-2-Z-(2-tetrahydropyranyloxyimino)-essigsäure

Eine Lösung von 15,8 g 2-(2-BOC-Amino-4-oxazolyl)-2-Z-(2-tetrahydropyranyloxyimino)-essigsäuremethylester in 158 ml Aethanol wird nacheinander mit einer Lösung von 11,66 g Kaliumhydroxid in 100 ml Wasser und mit 158 ml Aethanol versetzt und 30 Minuten lang bei ca. 15°C gerührt. Zur Aufarbeitung engt man im Vakuum ein, extrahiert die wässrige Phase zweimal mit Essigester, stellt mit 2 N Salzsäure auf pH 2,4 und extrahiert die wässrige Phase viermal mit einem Essigester-Aceton (4:1)-Gemisch. Man schüttelt die vereinigten organischen Phasen mit Wasser und gesättigter wässriger Kochsalzlösung, trocknet über Natriumsulfat und dampft ein. Man digeriert den Rückstand mit Hexan, filtriert ab und trocknet. Man erhält die Titelverbindung in Form eines hellgelben, mikrokristallinen Pulvers mit einem Schmelzbereich von 122 - 125°.

d) 2-(2-BOC-Amino-4-oxazolyl)-2-Z-(2-tetrahydropyranyloxyimino)-essigsäuremethylester

Zu 18,4 g 2-(2-Amino-4-oxazolyl)-2-Z-(2-tetrahydropyranyloxyimino)-essigsäuremethylester und 44,7 g Di-tert.-butyldicarbonat werden portionenweise 1,0 g Dimethylaminopyridin gegeben. Man rührt das Gemisch bei Raumtemperatur 4 Stunden lang. Zur Aufarbeitung verdünnt man mit Essigester, schüttelt nacheinander mit Wasser, gesättigter, wässriger Natriumhydrogencarbonatlösung und Kochsalzlösung aus, trocknet über Natriumsulfat und dampft ein. Nach Eluieren mit Toluol und Toluollösung mit steigenden Anteilen Essigester (5 % - 10 %) und Vereinigen der dünnschichtchromatographisch einheitlichen, produkthaltigen Fraktionen und Eindampfen erhält man die Titelverbindung. $R_f \sim 0{,}58$ (Silicagel; Toluol/Essigester; 1:1).

e) 2-(2-Amino-4-oxazolyl)-2-Z-(2-tetrahydropyranyloxyimino)-essigsäuremethylester

181,8 g 4-Brom-2-Z-(2-tetrahydropyranyloxyimino)-essigsäuremethylester werden in 900 ml Dimethylformamid und 354,4 g Harnstoff 1 Stunde lang auf 100° erwärmt. Man giesst das Reaktionsgemisch anschliessend auf Eiswasser, salzt mit Kochsalz aus und extrahiert viermal mit Essigester. Man schüttelt die vereinigten organischen Extrakte mit gesättigter wässriger Kochsalzlösung aus, trocknet über Natriumsulfat und dampft ein. Man erhält die rohe Titelverbindung als dunkles Oel, welches man chromatographisch an Kieselgel reinigt. Nach Elution mit Toluol/Essigester (2:1 bis 1:1) erhält man die einheitliche Titelverbindung, welche man aus Aether kristallisiert. Schmelzbereich: 145° - 149° (unter Zersetzung).

f) 4-Brom-2-Z-(2-tetrahydropyranyloxyimino)-acetessigsäuremethylester

Der gemäss Beispiel 1 g) erhältliche, rohe 4-Brom-2-Z-hydroxyimino-acetessigsäuremethylester wird in 1,65 l Dioxan gelöst und mit 20,1 ml 3,4-Dihydro-2H-pyran und 0,69 g p-Toluolsulfonsäuremonohydrat 15 Stunden lang bei Raumtemperatur gerührt. Zur Aufarbeitung engt man das Reaktionsgemisch ein, nimmt den Rückstand in Essigester auf, schüttelt mit Wasser und gesättigter, wässriger Natriumhydrogencarbonatlösung und Kochsalzlösung aus, trocknet über Natriumsulfat und dampft ein. Man erhält ein dunkelrotes Oel mit der Titelverbindung. $R_f \sim 0,6$ und 0,54 (Silicagel, Toluol/Essigester 1:1).

g) 4-Brom-2-Z-hydroxyiminoacetessigsäuremethylester

145,12 g 2-Z-Hydroxyininoacetessigsäuremethylester in 550 ml Chloroform werden bei 40° innerhalb einer Stunde mit einer Lösung von 50,9 ml Brom in 200 ml Chloroform versetzt und anschliessend 1,5 Stunden lang bei Raumtemperatur weitergerührt. Zur Aufarbeitung schüttelt man das Reaktionsgemisch dreimal mit gesättigter, wässriger Kochsalzlösung

aus, trocknet die organische Phase über Natriumsulfat und dampft im Vakuum ein. Man erhält die rohe Titelverbindung, welche ohne Reinigung direkt weiterverarbeitet wird (Beispiel 9f). $R_f$~0,29 (Silicagel; Toluol/Essigester; 1:1).

Beispiel 10:

a) 3-Acetoxymethyl-7β-[2-(2-amino-4-oxazolyl)-2-Z-(2-carboxyprop-2-yloxyimino)-acetylamino]-3-cephem-4-carbonsäure-natriumsalz

Man rührt bei Raumtemperatur 9,3 g 3-Acetoxymethyl-7β-[2-(2-amino-4-oxazolyl)-2-Z-(2-BOC-prop-2-yloxyimino)-acetylamino]-3-cephem-4-carbonsäure in 10 ml Methylenchlorid 75 Minuten lang mit 34 ml Trifluoressigsäure und 5,8 ml Anisol, versetzt mit 200 ml eiskaltem Toluol und dampft im Vakuum ein. Nach Digerieren des Rückstands in Aether und Trocknen erhält man ein beiges Pulver, das man in 50 ml Wasser suspendiert. Nach Einstellen des pH-Werts auf 7,5 mit 1 N Natronlauge chromatographiert man die Lösung an einer Säule mit 3 cm Durchmesser gefüllt mit 200 ml Amberlite XAD-2® Adsorberharz. Nach Eluieren mit Wasser, Vereinigen der dünnschichtchromatographisch einheitlichen, produkthaltigen Fraktionen und Lyophilisieren erhält man die Titelverbindung. $R_f$~0.23; (Silicagel; n-Butanol/Eisessig/Wasser; 67:10:23); IR (Nujol): 3350b, 1770, 1668, 1600 $cm^{-1}$; UV ($H_2O$): $\lambda_{max}$ = 264 nm (12700); 210 nm(sh).

b) 3-Acetoxymethyl-7β-[2-(2-amino-4-oxazolyl)-2-Z-(2-tert.-butyloxycarbonylprop-2-yloxyimino)-acetylamino]-3-cephem-4-carbonsäure

Man rührt bei Raumtemperatur ein Gemisch aus 2,5 g 3-Acetoxymethyl-7β-[2-(2-BOC-amino-4-oxazolyl)-2-Z-(2-BOC-prop-2-yloxyimino)-acetylamino]-3-cephem-4-carbonsäurediphenylmethylester, 25 ml Methylenchlorid, 4,5 ml Trifluoressigsäure und 0,48 ml Anisol 75 Minuten lang, verdünnt mit eiskaltem Toluol und dampft im Vakuum ein. Digerieren des Rückstands mit Aether, Abfiltrieren und Trocknen ergibt das entsprechende Trifluoressigsäuresalz als beiges Pulver. Dieses suspendiert man in 10 ml eiskaltem Methanol und versetzt mit 2 ml einer 3 m methanolischen Natrium-α-äthylhexanoatlösung. Man dekantiert vom ausgefallenen öligen Niederschlag ab, digeriert den Rückstand in Aether und einer Aether-Hexan-(1:1)-Mischung, filtriert ab und trocknet. Man chromatographiert das erhaltene Rohprodukt mittels Dickschicht-chromatographie an silylierten Silicagelplatten ($UPC_{12}$, Laufmittel: Wasser/Acetonitril; 3:1). Nach Eluieren der produkthaltigen Zone mit 50 ml Acetonitril/Wasser (1:1)-Gemisch, Filtrieren durch einen Millipore-Filter und Eindampfen erhält man die reine Titelverbindung, welche aus einem Aceton/Aether-Gemisch umgefällt wird. $R_f \sim 0.4$ (Silicagel; n-Butanol/Eisessig/Wasser; 67:10:23); IR (Nujol): 3320b, 1770sh, 1730, 1674, 1598 $cm^{-1}$; UV ($H_2O$): 210 nm(sh); 263 nm (10900).

c) <u>3-Acetoxymethyl-7β-[2-(2-BOC-amino-4-oxazolyl)-2-Z-(2-BOC-prop-2-yloxyimino)-acetylamino]-3-cephem-4-carbonsäurediphenylmethylester</u>

Zu 0,9 ml Dimethylformamid in 40 ml Oxalylchlorid gibt man bei ca. -5° 1,0 ml Oxalylchlorid in 30 ml Methylenchlorid, rührt 20 Minuten bei -5° bis -10° und versetzt anschliessend mit 3,55 g 2-(2-BOC-Amino-4-oxazolyl)-2-Z-(2-BOC-prop-2-yloxyimino)-essigsäure und 1,4 ml N-Methylmorpholin. Man lässt 30 Minuten bei ca. -10° weiterrühren, versetzt anschliessend das Reaktionsgemisch mit einer Lösung aus 4,39 g 3-Acetoxymethyl-7β-amino-3-cephem-4-carbonsäurediphenyl-methylester und 1,4 ml N-Methylmorpholin in 44 ml Methylenchlorid und lässt 30 Minuten bei -5° und 75 Minuten bei 0° reagieren. Zur Aufarbeitung engt man das Reaktionsgemisch ein, nimmt in Essigester auf und schüttelt nacheinander mit 0,5 N Salzsäure, gesättigter $NaHCO_3$-Lösung und gesättigter Kochsalzlösung aus. Nach Trocknen über Natriumsulfat und Eindampfen erhält man das Rohprodukt als bräunlichen Schaum, welches man an 250 g Kieselgel chromatographiert. Nach Eluieren mit Toluol und Toluollösung mit steigenden Anteilen Essigester (10 % - 50 %), Vereinigen der dünnschichtchromatographisch einheitlichen, produkthaltigen Fraktionen und Eindampfen erhält man die Titelverbindung. $R_f \sim 0.52$ (Silicagel; Toluol/Essigester; 1:1); IR ($CH_2Cl_2$): u.a. 3420, 1800, 1745, 1690, 1633 $cm^{-1}$; UV (EtOH):

$\lambda_{max}$ = 258 nm (15400).

Man stellt die Ausgangsmaterialien folgendermassen her:

d) <u>2-(2-BOC-Amino-4-oxazolyl)-2-Z-(2-BOC-prop-2-yloxyimino)-essigsäure</u>

10,55 g 2-(2-Bis-(BOC)-amino-4-oxazolyl)-2-Z-(2-BOC-prop-2-yloxyimino)-essigsäuremethylester in 75 ml Aethanol werden mit einer Lösung von 7,08 g KOH in 50 ml Wasser und mit 75 ml Aethanol versetzt. Man rührt das Gemisch eine Stunde lang bei Raumtemperatur. Anschliessend engt man im Vakuum ein, stellt den pH-Wert mit 4 N Salzsäure auf ca. 1,6 und extrahiert mit Essigester mehrmals. Man schüttelt die vereinigten organischen Phasen mit Wasser und wässriger, gesättigter Kochsalzlösung aus, trocknet über Natriumsulfat und dampft ein. Nach Kristallisation des Rohprodukts erhält man die Titelverbindung in Form eines hellgelben Pulvers. Schmelzbereich: 140° bis 144° (unter Zersetzung).

e) <u>2-(2-Bis-(BOC)-amino-4-oxazolyl)-2-Z-(2-BOC-prop-2-yloxyimino)-essigsäuremethylester</u>

30,2 g 2-(2-Amino-4-oxazolyl)-2-Z-(2-BOC-prop-2-yloxyimino)-essigsäuremethylester suspendiert in 60,5 g Di-tert.-butyldicarbonat werden mit 0,9 g 4-Dimethylaminopyridin versetzt und eine Stunde lang bei Raumtemperatur gerührt. Anschliessend verdünnt man mit Essigester, schüttelt mit gesättigter Kochsalzlösung aus, trocknet über Natriumsulfat und dampft ein. Nach Chromatographieren des öligen Rückstands an 600 g Kieselgel, Eluieren mit Toluol und Toluollösung mit steigenden Anteilen Essigester (2,5 % - 5 %) und Eindampfen erhält man die dünnschichtchromatographisch einheitliche Titelverbindung.
UV ($C_2H_5OH$): $\lambda_{max}$ = 211 nm (10500) u. 254 nm (9200); IR ($CH_2Cl_2$): 1812, 1783, 1748, 1600, 1587 $cm^{-1}$.

f) 2-(2-Amino-4-oxazolyl)-2-Z-(2-BOC-prop-2-yloxyimino)-essigsäure-methylester

Zu einer Lösung von 11,25 g Bromisobuttersäure-tert.-butylester und 16,2 g Kaliumcarbonat in 45 ml Dimethylsulfoxid wird unter Eiskühlung eine Lösung von 8,31 g 2-(2-Amino-4-oxazolyl)-2-Z-hydroxyiminoessigsäuremethylester in 45 ml Dimethylsulfoxid gegeben. Anschliessend rührt man das Reaktionsgemisch 18 Stunden lang bei Raumtemperatur. Zur Aufarbeitung giesst man auf Eiswasser, extrahiert mit Essigester, schüttelt die organische Phase mit Wasser und gesättigter, wässriger Kochsalzlösung aus, trocknet über Natriumsulfat und dampft ein. Nach Kristallisation des Rückstands aus Aether erhält man die Titelverbindung in Form von gelblichen Kristallen. Schmelzbereich: 156° - 160°.

g) 2-(2-Amino-4-oxazolyl)-2-Z-hydroxyiminoessigsäuremethylester

26,9 g 2-(2-Amino-4-oxazolyl)-2-Z-(2-tetrahydropyranyloxyimino)-essigsäuremethylester in 300 ml Methylenchlorid werden mit 300 ml Trifluoressigsäure zwei Stunden lang bei Raumtemperatur gerührt, anschliessend mit kaltem Toluol versetzt und im Vakuum eingedampft. Man nimmt den Rückstand in Essigester auf, extrahiert nacheinander mit gesättigter, wässriger Natriumhydrogencarbonat- und Kochsalzlösung, trocknet über Natriumsulfat und dampft ein. Nach Kristallisation des öligen Rückstands aus Aether erhält man die Titelverbindung. Schmelzbereich: 151° - 155° (unter Zersetzung).

Beispiel 11: 3-Pyridiniomethyl-7β-[2-(2-amino-4-oxazolyl)-2-Z-(2-carboxyprop-2-yloxyimino)-acetylamino]-3-cephem-4-carboxylat-natriumsalz

Zu einer auf 70° erwärmten Lösung von 30 g Natriumjodid in 16 ml Wasser werden 1,2 ml Eisessig, 1,15 ml Pyridin und 5,55 g 3-Acetoxymethyl-7β-[2-(2-amino-4-oxazolyl)-2-Z-(2-carboxyprop-2-yloxyimino)-acetylamino]-3-cephem-4-carbonsäure-natriumsalz gegeben. Man rührt das

Gemisch 1,5 Stunden lang unter Stickstoffatmosphäre. Nach Abkühlen stellt man den pH-Wert durch Zugabe von 1 N Natronlauge auf 5,1 ein und chromatographiert das Gemisch an einer Säule mit 4,5 cm Durchmesser gefüllt mit ca. 750 ml Amberlite XAD-2® Adsorberharz. Nach Eluieren mit einer 10 %igen wässrigen Isopropanollösung, Vereinigen der dünnschichtchromatographisch einheitlichen, produkthaltigen Fraktionen, Digerieren und Eindampfen mit Aceton erhält man die Titelverbindung in Form eines gelben Pulvers. $R_f \sim 0.47$ (silyliertes Silicagel $UPC_{12}$; Wasser/Acetonitril; 6:1); IR (Nujol): u.a. 3345, 1770, 1665, 1600b $cm^{-1}$; UV ($H_2O$): $\lambda_{max}$ = 255 nm (12000).

Beispiel 12: 3-(4-Carbamoylpyridiniomethyl)-7β-[2-(2-amino-4-oxazolyl-2-Z-(2-carboxyprop-2-yloxyimino)-acetylamino]-3-cephem-4-carboxylat-natriumsalz

Zu einer auf 70° erwärmten Lösung von 21 g Natriumjodid in 11 ml Wasser werden nacheinander 1,16 g Isonicotinamid, 0,8 ml Eisessig und 3,88 g 3-Acetoxymethyl-7β-[2-(2-amino-4-oxazolyl)-2-Z-(2-carboxyprop-2-yloxyimino)-acetylamino]-3-cephem-4-carbonsäurenatriumsalz gegeben. Man rührt das Gemisch 1,5 Stunden lang unter Stickstoffatmosphäre. Nach Abkühlen stellt man den pH-Wert durch Zugabe von 1 N Natronlauge auf 5,3 ein und chromatographiert das Gemisch auf einer Säule mit 4,5 cm Durchmesser gefüllt mit 650 ml Amberlite XAD-2® Adsorberharz. Nach Eluieren mit Wasser und Vereinigen der dünnschichtchromatographisch einheitlichen, produkthaltigen Fraktionen, Eindampfen und mehrmaligem Digerieren mit Aceton zwecks Entfernung von noch vorhandenem Isonicotinamid erhält man die Titelverbindung. $R_f \sim 0.6$ (silyliertes Silicagel $UPC_{12}$; Wasser/Acetonitril; (6:1 ); IR (Nujol): u.a. 3320b; 1770; 1665; 1600b $cm^{-1}$; UV ($H_2O$): $\lambda_{max}$ : 220 nm (18600) und 261 nm (15400).

Beispiel 13:

a) 7β-[2-(2-Amino-4-oxazolyl)-2-Z-(2-carboxyprop-2-yloxyimino)-acetylamino]-3-cephem-4-carbonsäure-dinatriumsalz

Man rührt 3,04 g 7β-[2-(2-BOC-Amino-4-oxazolyl)-2-Z-(2-BOC-prop-2-yloxyimino)-acetylamino]-3-cephem-4-carbonsäurediphenylmethylester, 15 ml Trifluoressigsäure, 2,0 ml Anisol und 3,0 ml Methylenchlorid 1,5 Stunden bei Raumtemperatur, versetzt anschliessend das Gemisch mit 100 ml eiskaltem Toluol und dampft im Vakuum ein. Nach Digerieren und Trocknen erhält man das entsprechende Trifluoressigsäuresalz, welches man in 5 ml Methanol löst und mit 3 ml einer 3 molaren methanolischen Natrium-α-äthylhexanoat-Lösung versetzt. Zur Vervollständigung der Fällung gibt man Aether hinzu, filtriert und wäscht mit Aether nach. Man löst den erhaltenen Niederschlag in Wasser, stellt den pH-Wert mit 1 N Natronlauge auf 7,5 ein und chromatographiert die Lösung an einer Säule mit 3,5 cm Durchmesser gefüllt mit 250 ml Amberlite XAD-2® Adsorberharz. Nach Eluieren mit einer 10 %igen wässrigen Isopropanollösung, Vereinigen der dünnschichtchromatographisch einheitlichen, produkthaltigen Fraktionen und Lyophilisieren erhält man die Titelverbindung. $R_f \sim 0.25$ (Silicagel; n-Butanol/Eisessig/Wasser; 67:10:23); IR (Nujol): 3350b, 1770, 1668b, 1595 $cm^{-1}$; UV ($H_2O$): $\lambda_{max}$: 264 nm (9700).

b) 7β-[2-(2-Amino-4-oxazolyl)-2-Z-(2-BOC-prop-2-yloxyimino)-acetylamino]-3-cephem-4-carbonsäure-natriumsalz

5,0 g 7β-[2-(2-BOC-Amino-4-oxazolyl)-2-Z-(2-BOC-prop-2-yl-oxyimino)-acetylamino]-3-cephem-4-carbonsäurediphenylmethylester in 50 ml Methylenchlorid werden mit 1,05 ml Anisol und 6,25 ml Trifluoressigsäure drei Stunden lang bei Raumtemperatur unter Stickstoffatmosphäre gerührt, mit 200 ml eiskaltem Toluol versetzt und im Vakuum eingedampft. Nach Digerieren des Rückstands und Eindampfen erhält man das entsprechende Trifluoressigsäuresalz, das man in 50 ml Wasser suspendiert, mit 1 N NaOH auf einen pH-Wert von 7.3 bringt und an

einer Säule gefüllt mit 500 ml Amberlite XAD-2$^{R}$ Adsorberharz chromatographiert. Nach Eluieren mit Wasser und einer wässrigen Lösung mit steigenden Anteilen Isopropanol (5 - 50 %), Vereinigen der dünnschichtchromatographisch einheitlichen, produkthaltigen Fraktionen, Eindampfen und Trocknen erhält man die Titelverbindung. $R_f$∼0.44 (Silicagel; n-Butanol/Eisessig/Wasser; 67:10:23); IR (Nujol): u.a. 3340b, 1764, 1737, 1668, 1600 $cm^{-1}$; UV ($H_2O$): 205 nm (20800); 262 nm (10000).

c) 7β-[2-(2-BOC-Amino-4-oxazolyl)-2-Z-(2-BOC-prop-2-yloxyimino)-acetylamino]-3-cephem-4-carbonsäurediphenylmethylester

1,35 ml Dimethylformamid und 1,5 ml Oxalylchlorid lässt man in 100 ml Methylenchlorid 30 Minuten lang bei ca. -10° reagieren, versetzt mit 6,2 g 2-(2-BOC-Amino-4-oxazolyl)-2-Z-(2-BOC-prop-2-yloxyimino)-essigsäure und 2,1 ml N-Methylmorpholin und lässt 30 Minuten bei -5° bis -10° weiterreagieren. Man versetzt anschliessend das Reaktionsgemisch mit einer Lösung aus 5,5 g 7β-Amino-3-cephem-4-carbonsäurediphenylmethylester und 2,1 ml N-Methylmorpholin in 55 ml Methylenchlorid und lässt 30 Minuten bei -5° reagieren. Zur Aufarbeitung engt man das Reaktionsgemisch ein, nimmt in Essigester auf und schüttelt nacheinander mit 0,1 N Salzsäure, gesättigter $NaHCO_3$-Lösung und gesättigter Kochsalzlösung aus. Nach Trocknen über Natriumsulfat erhält man das Rohprodukt, welches man an 250 g Kieselgel chromatographiert. Nach Eluieren mit Toluol und Toluollösung mit steigenden Anteilen Essigester (10 % - 20 %), Vereinigen der dünnschichtchromatographisch einheitlichen, produkthaltigen Fraktionen und Eindampfen erhält man die Titelverbindung. $R_f$∼0.55 (Silicagel; Toluol/Essigester; 1:1); IR ($CH_2Cl_2$): 3418, 1800, 1758, 1733, 1685, 1632 $cm^{-1}$; UV (EtOH): $\lambda_{max}$ = 260 nm (sh) (6700).

Beispiel 14:

a) 7β-[2-(2-Amino-4-oxazolyl)-2-Z-(2-BOC-prop-2-yloxyimino)-acetylamino]-3-cephem-4-carbonsäurepivaloyloxymethylester

0,8 g 7β-[2-(2-Amino-4-oxazolyl)-2-Z-(2-BOC-prop-2-yloxyimino)-acetylamino]-3-cephem-4-carbonsäure-natriumsalz in 8 ml Dimethylformamid lässt man mit 0,4 ml Jodmethylpivalat 30 Minuten lang bei 0° reagieren, rührt unter Zugabe eines Phosphatpuffers von pH 8 und extrahiert mit Essigester. Man schüttelt die organische Phase mit gesättigter $NaHCO_3$-Lösung und gesättigter Kochsalzlösung aus, trocknet über Natriumsulfat und dampft ein. Man chromatographiert das Rohprodukt an 17 g Kieselgel. Nach Eluieren mit Toluol und Toluollösung enthaltend steigende Anteile Essigester (10 % bis 50 %), Vereinigung der dünnschichtchromatographisch einheitlichen, produkthaltigen Fraktionen und Eindampfen erhält man die Titelverbindung.

b) 7β-[2-(2-Amino-4-oxazolyl)-2-Z-(2-BOC-prop-2-yloxyimino)-acetylamino]-3-cephem-4-pivaloyloxymethylester-hydrochlorid

Zur Ueberführung in das Hydrochlorid löst man den gemäss Beispiel 14a) erhältlichen Pivaloyloxymethylester in 5 ml Aether, fügt 1,6 ml 0,5 N Salzsäure in Methylenchlorid hinzu und fällt mit Hexan aus. Nach Filtrieren, Umfällen aus Aether/Hexan (1:1) und Trocknen erhält man die Titelverbindung. $R_f \sim 0{,}39$ (Silicagel, Essigester).

Beispiel 15: 3-(3-Chlorpyridiniomethyl)-7β-[2-(2-amino-4-oxazolyl)-2-Z-methoxyiminoacetylamino]-3-cephem-4-carboxylat

Zu einer auf 70° vorgewärmten Lösung von 11,25 g Natriumjodid und 560 mg 3-Chlorpyridin in 5,6 ml Wasser gibt man 1,7 g 3-Acetoxymethyl-7β-[2-(2-amino-4-oxazolyl)-2-Z-methoxyiminoacetylamino]-3-cephem-4-carbonsäure-natriumsalz und 0,21 ml Essigsäure. Nach einstündigem Rühren bei +70° unter Stickstoffatmosphäre wird die Lösung mit 1 N Natronlauge auf pH 5,2 gestellt und über Amberlite ER-180®

chromatographiert. Man erhält die Titelverbindung vom $R_f$-Wert 0.69 ($UPC_{12}$-Platten; Wasser/Acetonitril; 4:1); UV (Wasser): $\lambda_{max}$ = 215 nm (Schulter) und 265 nm (14000).

Beispiel 16: 3-(4-Carboxylatpyridiniomethyl)-7β-[2-(2-amino-4-oxazolyl)-2-Z-methoxyiminoacetylamino]-3-cephem-4-carboxylat-natriumsalz

Zu einer auf 70° vorgewärmten Lösung von 11,25 g Natriumjodid und 610 mg Isonicotinsäure in 5,6 ml Wasser gibt man 1,7 g 3-Acetoxymethyl-7β-[2-(2-amino-4-oxazolyl)-2-Z-methoxyiminoacetylamino]-3-cephem-4-carbonsäure-natriumsalz und 0,21 ml Essigsäure. Nach einstündigem Rühren bei 70° unter Stickstoffatmosphäre wird die Lösung mit 1 N Natronlauge auf pH 7.2 gestellt und über Amberlite ER-180® chromatographiert. Man erhält die Titelverbindung vom $R_f$-Wert 0.75 ($UPC_{12}$-Platten, Wasser/Acetonitril; 4:1); UV (Wasser): $\lambda_{max}$ = 216 nm (21200) und 260 nm (13300).

Beispiel 17:

a) 3-Carbamoyloxymethyl-7β-[2-(2-amino-4-oxazolyl)-2-Z-methoxyiminoacetylamino]-3-cephem-4-carbonsäure-natriumsalz

Eine Lösung von 4,5 g 7β-[2-(2-Amino-4-oxazolyl)-2-Z-methoxyiminoacetylamino]-3-carbamoyloxymethyl-3-cephem-4-carbonsäurediphenylmethylester in 20 ml Methylenchlorid wird 1 Stunde mit 19,7 ml Trifluoressigsäure und 3,3 ml Anisol gerührt, mit 250 ml eiskaltem Toluol versetzt und eingedampft. Nach Digerieren des Rückstandes mit Aether, Abnutschen und Trocknen enthält man das Trifluoressigsäuresalz der Titelverbindung als beiges Pulver. Es wird in 20 ml Wasser suspendiert. Man stellt die Lösung bei 0° mit 1 N Natronlauge auf pH 7.2 und chromatographiert über Amberlite ER-180®. Man erhält die Titelverbindung vom $R_f$-Wert 0.77 (Kieselgel 60; n-Butanol/Essigsäure/Wasser; 67:10:23); UV (Wasser): $\lambda_{max}$ 212 nm (18000) und 260 nm (12400).

b) 3-Carbamoyloxymethyl-7β-[2-(2-BOC-amino-4-oxazolyl)-2-Z-methoxyiminoacetylamino]-3-cephem-4-carbonsäurediphenylmethylester

Unter Stickstoffatmosphäre lässt man ein Gemisch von 1 ml Oxalylchlorid, 0,9 ml Dimethylformamid und 40 ml Methylenchlorid 30 Minuten bei ca. -5° bis -10° rühren und versetzt anschliessend mit 2,85 g 2-(2-tert.-Butyloxycarbonylamino-4-oxazolyl)-2-Z-methoxyiminoessigsäure und 1,4 ml N-Methylmorpholin. Nach weiteren 30 Minuten Rühren bei dieser Temperatur wird eine Lösung aus 4,39 g 3-Carbamoyloxymethyl-7β-amino-3-cephem-4-carbonsäurediphenylmethylester und 1,4 ml N-Methylmorpholin in 45 ml Methylenchlorid zugegeben. Man lässt 30 Minuten bei ca. -5° und 1 Stunde bei 0° nachreagieren. Das Reaktionsgemisch wird eingeengt, den Rückstand in Essigsäureäthylester aufgenommen und nacheinander mit 1 N Salzsäure, gesättigten Natriumhydrogencarbonat- und gesättigter Kochsalzlösung ausgeschüttelt. Die organische Phase wird über Natriumsulfat getrocknet. Nach Filtrieren und Eindampfen wird die Titelverbindung roh erhalten. Diese wird säulenchromatographisch (Kieselgel 60; Toluol/Essigsäureäthylester) gereinigt und schmilzt bei 70°.

Beispiel 18: 3-Carbamoyloxymethyl-7β-[2-(2-amino-4-oxazolyl)-2-Z-methoxyimino-acetylamino]-3-cephem-4-carbonsäure-pivaloyloxymethylester

Eine Lösung von 2,3 g Jodmethylpivalat in 15 ml Dimethylformamid wird bei 0° mit 2,6 g 3-Carbamoyloxymethyl-7β-[2-(2-amino-4-oxazolyl)-2-Z-methoxyiminoacetylamino]-3-cephem-4-carbonsäure-natriumsalz versetzt und 3 Stunden bei Raumtemperatur gerührt. Das Reaktionsgemisch wird mit Essigsäureäthylester verdünnt, nacheinander mit 25 ml Wasser, 10 ml gesättigter wässriger Natriumbicarbonat- und 10 ml gesättigter, wässriger Kochsalzlösung ausgeschüttelt, über Natriumsulfat getrocknet und im Vakuum eingedampft. Der Rückstand wird säulenchromatographisch gereinigt (Kieselgel 60; Methylenchlorid/Aethanol). Die Titelverbindung hat einen $R_f$-Wert von 0,75 (Kieselgel 60; Methylenchlorid/Aethanol; 9:1); IR (Methylenchlorid): 1790, 1740, 1685, 1600 und 1525 $cm^{-1}$.

Beispiel 19: 3-Pyridiniomethyl-7β-[2-(2-amino-4-oxazolyl)-2-Z-methoxy-iminoacetylamino]-3-cephem-4-carboxylat

Zu einer auf 70° vorgewärmten Lösung von 11,25 g Natriumjodid und 0,4 g Pyridin in 5,6 ml Wasser gibt man 1,7 g 3-Acetoxymethyl-7β-[2-(2-amino-4-oxazolyl)-2-Z-methoxyiminoacetylamino]-3-cephem-4-carbonsäure-natriumsalz und 0,21 ml Essigsäure. Man rührt 1 Stunde bei 70°, stellt die Lösung mit 1N Natronlauge auf pH 5,2 und chromatographiert über Amberlite MZM®. Man erhält die Titelverbindung mit einem $R_f$-Wert von 0,25 ($UPC_{12}$-Platten, Wasser/Acetonitril 4:1); UV-Spektrum (Wasser): $\lambda$max = 257 (13000).

Beispiel 20:

a) 7β-[2-(2-Amino-4-oxazolyl)-2-Z-methoxyiminoacetylamino]-3-cephem-4-carbonsäure-tert.-butyloxycarbonylmethylester

Ein Gemisch von 0,75 g 7β-[2-(2-BOC-amino-4-oxazolyl)-2-Z-methoxyimino-acetylamino]-3-cephem-4-carbonsäure-tert.-butyloxycarbonylmethylester, 3,9 ml Trifluoressigsäure und 3,9 ml Methylenchlorid wird 1 Stunde bei Raumtemperatur gerührt, mit kaltem Toluol versetzt und unter vermindertem Druck eingedampft. Dieser Vorgang wird mit einem Gemisch Toluol/Chloroform und einem Gemisch Toluol/Aether wiederholt. Der Rückstand wird aus Aether kristallisiert. Das Trifluoracetat-Salz der Titelverbindung schmilzt bei 145-118° unter Zersetzung; IR-Spektrum (Nujol): Banden bei 1785, 1740, 1660, 1630, 1540 $cm^{-1}$.

Das Trifluoracetat-Salz wird in Essigester aufgenommen und zweimal mit kalter, gesättigter Natriumbicarbonat-Lösung gewaschen. Nach Trocknen der organischen Phase über Natriumsulfat wird die Lösung unter reduziertem Druck eingeengt. Man chromatographiert den Rückstand (Kieselgel 60; Methylenchlorid/Aethanol) und erhält die Titelverbindung mit einem $R_f$-Wert von 0,21 (Kieselgel 60; Methylenchlorid/Aethanol 9:1).

b) 7β-[2-(2-BOC-Amino-4-oxazolyl)-2-Z-methoxyiminoacetylamino]-3-cephem-4-carbonsäure-tert.-butyloxycarbonylmethylester

Ein Gemisch von 2,3 g 7β-[2-(2-BOC-Amino-4-oxazolyl)-2-Z-methoxyimino-acetylamino]-3-cephem-4-carbonsäure und 18 ml N,N-Dimethylacetamid wird bei 20° mit 1,0 g Kaliumphthalimid versetzt. Nach Kühlen auf 0° werden 1,33 g Jodessigsäure-tert.-butylester zugegeben. Nach 30-minütigem Rühren gibt man je 50 ml Essigester und 50 ml Waser zu, stellt mit 1N Natronlauge auf pH 7 und trennt die Esterphase ab. Man extrahiert erneut mit Ester, wäscht die vereinigten Esterphasen mit gesättigter Natriumchlorid-Lösung, trocknet und dampft unter vermindertem Druck ein. Der Rückstand wird säulenchromatographisch (Kieselgel 60; Methylenchlörid/Aethanol) gereinigt.

Beispiel 21: 3-Carbamoyloxymethyl-7β-[2-(2-amino-4-oxazolyl)-2-Z-methoxyiminoacetylamino]-3-cephem-4-carbonsäure-1-(äthoxycarbonyloxy)-äthylester

Eine Mischung von 1,76 g 1-(1-Chloräthyl)-äthylcarbonat, 1,99 g Natriumjodid und 15 ml absolutem Aceton wird 35 Minuten unter Rückfluss erhitzt. Das Aceton wird unter vermindertem Druck abdestilliert. Der Rückstand wird zwischen 25 ml Wasser und 25 ml Aether verteilt, die Aetherphase abgetrennt und mit Wasser und einer wässrigen Natriumchlorid-Lösung gewaschen. Die Aetherlösung wird getrocknet und unter vermindertem Druck eingedampft. Das verbleibende 1-(1-Jodäthyl)-äthylcarbonat wird in 5 ml Dimethylformamid gelöst und diese Lösung zu einer Lösung von 1,15 g 3-Carbamoylmethyl-7β-[2-(2-amino-4-oxazolyl)-2-Z-methoxyiminoacetylamino]-3-cephem-4-carbonsäure-natriumsalz in 8 ml Dimethylformamid gegeben. Die Mischung wird 1 Stunde bei Raumtemperatur gerührt und dann mit 50 ml Essigester versetzt. Die organische Phase wird nacheinander mit Wasser, einer gesättigten wässrigen Natriumbicarbonat-Lösung und einer gesättigten wässrigen Natriumchlorid-Lösung gewaschen. Die Essigester-Phase wird über Magensiumsulfat getrocknet und filtriert. Das Lösungsmittel wird unter vermindertem Druck abdestilliert. Der Rückstand wird säulenchromatographisch (Kieselgel 60; Methylenchlorid/Aethanol) gereinigt. Die Titelverbindung hat einen Rf-Wert von 0,47 (Kieselgel 60; Methylenchlorid/Aethanol 9:1); IR-Spektrum (Methylenchlorid): Banden bei 1790, 1765, 1740, 1675, 1655, 1586 $cm^{-1}$.

Beispiel 22: 3-Carbamoyloxymethyl-7β-[2-(2-amino-4-oxazolyl)-2-Z-methoxyiminoacetylamino]-3-cephem-4-carbonsäurepropionyloxymethyl-ester

Zu einer Lösung von 2,3 g 3-Carbamoyl-7β-[2-(2-amino-4-oxazolyl)-2-Z-methoxyiminoacetylamino]-3-cephem-4-carbonsäurenatriumsalz in 15 ml absolutem Dimethylformamid werden bei 0° 1,25 g Propionsäurejodmethylester gegeben. Nach zweistündigem Rühren bei 0° wird das Reaktionsgemisch mit 100 ml Essigester verdünnt und nacheinander mit Wasser und gesättigten Natriumcarbonat- und Natriumchlorid-Lösungen gewaschen. Nach Trocknen über Natriumsulfat wird die Lösung unter vermindertem Druck eingedampft und der verbleibende Rückstand chromatographiert (Kieselgel 60; Methylenchlorid/Aethanol). Man erhält so die Titelverbindung mit einem $R_f$-Wert von 0,47 (Kieselgel 60; Methylenchlorid/Aethanol 9:1); IR-Spektrum (KBr): Banden bei 1785, 1735, 1660, 1600 $cm^{-1}$.

Beispiel 23: Analog Beispiel 4a, 20a und 21 kann man folgende Ester:

3-Carbamoyloxymethyl-7β-[2-(2-amino-4-oxazolyl)-2-Z-methoxyiminoacetylamino]-3-cephem-4-carbonsäure-1-(propionyloxy)-äthylester - IR ($CH_2Cl_2$): 1790, 1760, 1740, 1675, 1655, 1520 $cm^{-1}$; $R_f$: ca. 0,44 (Kieselgel, $CH_2Cl_2$/EtOH 9:1) -

7β-[2-(2-Amino-4-oxazolyl)-2-Z-methoxyiminoacetylamino]-3-cephem-4-carbonsäure-1-(propionyloxy)-äthylester - IR ($CH_2Cl_2$): 1792, 1760, 1740, 1660, 1640, 1520 $cm^{-1}$; $R_f$: ca. 0,61 (Kieselgel, $CH_2Cl_2$/EtOH 9:1) -

3-(1-Methyl-1-H-tetrazol-5-ylthiomethyl)-7β-[2-(2-amino-4-oxazolyl)-2-Z-methoxyiminoacetylamino]-3-cephem-4-carbonsäure-1-(propionyloxy)-äthylester - IR ($CH_2Cl_2$): 1790, 1765, 1742, 1678, 1655, 1785 $cm^{-1}$; $R_f$: ca. 0,23 (Kieselgel, $CH_2Cl_2$/EtOH 9:1) -

3-(1-Methyl-1H-tetrazol-5-ylthiomethyl)-7β-[2-(2-amino-4-oxazolyl)-2-Z-methoxyiminoacetylamino]-3-cephem-4-carbonsäurepivaloyloxymethyl-ester - IR ($CH_2Cl_2$): 1785, 1740, 1670, 1620, 1520 $cm^{-1}$; $R_f$: ca. 0,38 (Kieselgel, $CH_2Cl_2$/EtOH 9:1) -

7β-[2-(2-Amino-4-oxazolyl)-2-hydroxyiminoacetylamino]-3-cephem-4-carbonsäurepivaloyloxymethylester - IR ($CH_2Cl_2$): 1790, 1745, 1675, 1625, 1548 $cm^{-1}$; $R_f$: ca. 0,42 (Kieselgel, $CH_2Cl_2$/EtOH 9:1) -

3-(1-Methyl-1H-tetrazol-5-ylthiomethyl)-7β-[2-(2-amino-4-oxazolyl)-2-Z-methoxyiminoacetylamino]-3-cephem-4-carbonsäure-1-(äthoxycarbonyl-oxy)-äthylester - IR ($CH_2Cl_2$): 1785, 1745, 1673, 1620, 1545 $cm^{-1}$; $R_f$: ca. 0, 28 (Kieselgel, $CH_2Cl_2$/EtOH 9:1) -

7β-[2-(2-Amino-4-oxazolyl)-2-hydroxyiminoacetylamino]-3-cephem-4-carbonsäure-1-(äthoxycarbonyloxy)-äthylester - IR ($CH_2Cl_2$): 1788, 1762, 1740, 1672, 1640, 1555 $cm^{-1}$; $R_f$: ca. 0,38 (Kieselgel, $CH_2Cl_2$/EtOH 9:1) -

herstellen.

Beispiel 24: In analoger Weise zu Beispiel 1-3 und 5-19 kann man bei Verwendung von geeigneten Ausgangsmaterialien folgende Verbindungen erhalten:

3-(1,3,4-Thiadiazol-5-ylthiomethyl)-7β-[2-(2-amino-4-oxazolyl)-2-Z-methoxyiminoacetylamino]-3-cephem-4-carbonsäure-natriumsalz - UV ($\lambda_{max}$): 215 (16800), 257 (13200) nm; $R_f$: ca. 0.78 ($UPC_{12}$-Platten, $H_2O/CH_3CN$) -

3-(2-Methyl-5,6-dioxo-1,2,5,6-tetrahydro-as-triazin-3-ylthiomethyl)-7β-[2-(2-amino-4-oxazolyl)-2-Z-methoxyiminoacetylamino]-3-cephem-4-carbonsäure-natriumsalz - UV ($\lambda_{max}$): 218 (17400), 265 (14200) nm; $R_f$: ca. 0,63 ($UPC_{12}$-Platten, $H_2O/CH_3CN$) -

3-(4-Hydroxymethylpyridiniomethyl)-7β-[2-(2-amino-4-oxazolyl)-2-Z-methoxyiminoacetylamino]-3-cephem-4-carboxylat - UV ($\lambda_{max}$): 216 (s), 262 (13800) nm; $R_f$: ca. 0,72 ($UPC_{12}$-Platten, $H_2O/CH_3CN$) -

3-Carbamoyloxymethyl-7β-[2-(2-amino-4-oxazolyl)-2-Z-hydroxyiminoacetyl-amino]-3-cephem-4-carbonsäure-natriumsalz - UV ($\lambda_{max}$): 211 (18100), 260 (13400) nm; $R_f$: ca. 0,68 ($UPC_{12}$-Platten, $H_2O/CH_3CN$) -

7β-[2-Amino-4-oxazolyl-2-hydroxyiminoacetylamino]-3-cephem-4-carbon-säure-natriumsalz - UV ($\lambda_{max}$): 207 (17400), 261 (12800) nm; $R_f$: ca. 0,72 ($UPC_{12}$-Platten, $H_2O/CH_3CN$) -

3-(1-Methyl-1H-tetrazol-5-ylthiomethyl)-7β-[2-(2-amino-4-oxazolyl)-2-hydroxyiminoacetylamino]-3-cephem-4-carbonsäure-natriumsalz - UV ($\lambda_{max}$): 216 (s), 264 (14400) nm; $R_f$: ca. 0,63 ($UPC_{12}$-Platten, $H_2O/CH_3CN$) -

3-Acetoxymethyl-7β-[2-(2-amino-4-oxazolyl)-2-Z-(2-aminoäthoxyimino)-acetylamino]-3-cephem-4-carbonsäure-natriumsalz - UV ($\lambda_{max}$): 213 (15800), 262 (13600) nm; $R_f$ ca. 0,65 ($UPC_{12}$-Platten, $H_2O/CH_3CN$) -

3-Acetoxymethyl-7β-[2-(2-amino-4-oxazolyl)-2-Z-(2-hydroxyäthoxyimino)-acetylamino]-3-cephem-4-carbonsäure-natriumsalz - UV ($\lambda_{max}$): 212 (16200), 260 (13500) nm; $R_f$: ca. 0,68 ($UPC_{12}$-Platten, $H_2O/CH_3CN$).

Beispiel 25: Trockenampullen oder Vialen enthaltend 0,5 g Wirkstoff, z.B. 3-Acetoxymethyl-7β-[2-(2-amino-4-oxazolyl)-2-Z-methoxyiminoacetyl-amino]-3-cephem-4-carbonsäure, kann man folgendermassen herstellen:

Zusammensetzung (für 1 Ampulle oder Viale):

| | |
|---|---|
| Wirkstoff | 0,5 g |
| Mannit | 0,05 g |

Eine sterile wässrige Lösung bestehend aus dem Wirkstoff und Mannit wird unter aseptischen Bedingungen in 5 ml-Ampullen oder 5 ml-Vialen eingefüllt, worauf diese verschlossen und geprüft werden.

Patentansprüche:

(für alle benannten Länder ausser Oesterreich)

1. 7β-Aminooxazolylacetylamino-3-cephem-4-carbonsäure-Verbindungen der Formel

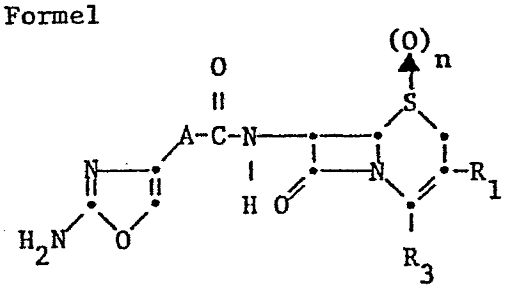

(I) ,

worin

n eine ganze Zahl von 0 bis 2, A Carbonyl, Methylen, oder eine Methylengruppe, welche durch Amino, geschütztes Amino, Hydroxy, geschütztes Hydroxy, Sulfo, geschütztes Sulfo oder durch eine Gruppe der Formel

$$=N-O-R_4$$

substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$ Wasserstoff, Niederalkyl, Niederalkoxy, Halogen oder eine Gruppe der Formel $-CH-R_2$, worin $R_2$ Hydroxy, Mercapto, verestertes Hydroxy oder Mercapto, veräthertes Hydroxy oder Mercapto oder eine Ammoniogruppe darstellt, und $R_3$ Carboxyl oder geschütztes Carboxyl bedeuten, Hydrate und Salze von Verbindungen der Formel I.

2. 7β-Aminooxazolylacetylamino-3-cephem-4-carbonsäure-Verbindungen der Formel I, gemäss Anspruch 1, worin n eine ganze Zahl von 0 bis 2, A Carbonyl oder eine Methylengruppe, welche durch Amino, geschütztes Amino, Hydroxy, geschütztes Hydroxy, Sulfo, geschütztes Sulfo oder durch eine Gruppe der Formel $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl

darstellt, $R_1$, $R_2$ und $R_3$ die in Anspruch 1 genannten Bedeutungen haben, Hydrate und Salze von diesen Verbindungen.

3. 7β-Aminooxazolylacetylamino-3-cephem-4-carbonsäure-Verbindungen der Formel I gemäss Anspruch 1, worin n eine ganze Zahl von 0 bis 2, A Carbonyl oder eine Methylengruppe, welche durch Amino, geschütztes Amino, Hydroxy, geschütztes Hydroxy, Sulfo, geschütztes Sulfo oder durch eine Gruppe der Formel $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$, $R_2$ und $R_3$ die in Anspruch 1 genannten Bedeutungen haben, Hydrate und Salze von diesen Verbindungen.

4. Verbindungen nach Anspruch 1 der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, N-Niederalkylcarbamoyloxy, einen aromatischen, monocyclischen, fünf- oder sechsgliedrigen, Diaza-, Triaza-, Tetraaza-, Thiaza-, Thiadiaza-, Thia-, Oxaza- oder Oxadiazacyclylrest, z.B. Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazinylthio, z.B. 5,6-Dioxo-1,2,5,6-tetrahydro-as-triazin-3-ylthio oder 5,6-Dioxo-1,4,5,6-tetrahydro-as-triazin-3-ylthio, welcher durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl, Carboxyniederalkyl, z.B. Carboxymethyl, Amino, Carboxyniederalkylamino, z.B. 2-Carboxyäthylamino, oder durch Carbamoyl substituiert sein kann, oder Ammonio, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl, Carboxy, Carboxyniederalkyl, z.B. Carboxymethyl, Halogen, z.B. Chlor oder Brom,

oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxyl oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl, oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A Amino, Aminomethylen, Hydroxymethyl, Sulfomethylen oder eine durch eine Gruppe der Formel $=N-O-R_4$ substituierte Methylengruppe, worin $R_4$ Wasserstoff, Niederalkyl, z.B. Methyl, Hydroxyniederalkyl, z.B. 2-Hydroxyäthyl, Aminoniederalkyl, z.B. 2-Aminoäthyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, darstellt, bedeuten, wobei die Gruppe der Formel $-O-R_4$ die syn-(oder Z-)Stellung aufweist, und Salze, insbesondere pharmazeutisch annehmbare Salze von Verbindungen der Formel I, welche salzbildende Gruppen besitzen.

5. Verbindungen nach Anspruch 2 der Formel I, worin n, A, $R_1$, $R_2$ und $R_4$ die in Anspruch 4 genannten Bedeutungen haben und $R_3$ Carboxyl oder unter physiologischen Bedingungen spaltbares Carboxyl, z.B. Acyloxymethoxycarbonyl, z.B. Niederalkanoyloxymethoxycarbonyl, z.B. Pivaloyloxymethoxycarbonyl, oder Niederalkoxycarbonyloxyniederalkoxycarbonyl, z.B. 1-(Aethoxycarbonyloxy)-äthoxycarbonyl, bedeuten, und Salze, insbesondere pharmazeutisch annehmbare Salze von diesen Verbindungen.

6. Verbindungen nach Anspruch 3 der Formel I, worin n, A, $R_1$, $R_2$ und $R_4$ die in Anspruch 4 genannten Bedeutungen haben, und $R_3$ Carboxyl, Pivaloyloxymethoxycarbonyl oder 1-(Aethoxycarbonyloxy)-äthoxycarbonyl bedeutet, und Salze, insbesondere pharmazeutisch annehmbare Salze von diesen Verbindungen.

7. Verbindungen nach Anspruch 4 zur Herstellung von Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Triazolylthio, z.B. 1H-1,2,3-Triazol-5-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Tetrazolylthio, z.B. 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio, durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydrotriazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxyl, oder unter physiologischen Bedinungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl, oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Wasserstoff, Niederalkyl, z.B. Methyl, Carboxyniederalkyl, z.B. 2-Carboxy-2-propyl, Carbamoyl oder Niederalkylcarbamoyl, z.B. Methylcarbamoyl, darstellt, bedeuten, wobei die Gruppe der Formel $-O-R_4$ die syn-(oder Z-)Stellung aufweist und Salze, insbesondere pharmazeutisch verwendbare Salze, von Verbindungen der Formel I, welche salzbildende Gruppen besitzen.

8. Verbindungen nach Anspruch 5 zur Herstellung von Verbindungen der Formel I, worin n, A, $R_1$, $R_2$ und $R_4$ die in Anspruch 7 genannten Bedeutungen haben, und $R_3$ Carboxyl, Niederalkanoyloxymethoxycarbonyl, z.B. Pivaloyloxymethoxycarbonyl oder Niederalkoxycarbonyloxyniederalkoxycarbonyl, z.B. 1-(Aethoxycarbonyloxy)-äthoxycarbonyl, bedeutet, und Salze, insbesondere pharmazeutisch annehmbare Salze von diesen Verbindungen.

9. Verbindungen nach Anspruch 6 der Formel I, worin n, A, $R_1$, $R_2$ und $R_4$ die in Anspruch 7 genannten Bedeutungen haben, und $R_3$ Carboxyl, Pivaloyloxymethoxycarbonyl oder 1-(Aethoxycarbonyloxy)-äthoxycarbonyl bedeutet, und Salze, insbesondere pharmazeutisch annehmbare Salze von diesen Verbindungen.

10. Verbindungen nach Anspruch 7 der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridino, 3- oder 4-Chlorpyridinio, 3- oder

4-Bromypridin oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel $-O-R_4$ die syn-(oder Z-)Stellung aufweist, und Salze, insbesondere pharmazeutisch verwendbare Salze, von Verbindungen der Formel I, welche salzbildende Gruppen aufweisen.

11. Verbindungen nach Anspruch 8 der Formel I, worin n, A, $R_1$, $R_2$ und $R_4$ die in Anspruch 10 genannten Bedeutungen haben, und $R_3$ Carboxyl, Niederalkanoyloxymethoxycarbonyl, z.B. Pivaloyloxymethoxycarbonyl, oder Niederalkoxycarbonyloxyniederalkoxycarbonyl, z.B. 1-(Aethoxycarbonyloxy)-äthoxycarbonyl, bedeutet, und Salze, insbesondere pharmazeutisch annehmbare Salze von diesen Verbindungen.

12. Verbindungen nach Anspruch 9 der Formel I, worin n, A, $R_1$, $R_2$ und $R_4$ die in Anspruch 10 genannten Bedeutungen haben, und $R_3$ Carboxyl, Pivaloyloxymethoxycarbonyl oder 1-(Aethoxycarbonyloxy)-äthoxycarbonyl bedeutet, und Salze, insbesondere pharmazeutisch annehmbare Salze von diesen Verbindungen.

13. 3-Acetoxymethyl-7β-[2-(2-amino-4-oxazolyl)-2-Z-methoxyiminoacetylamino]-3-cephem-4-carbonsäure gemäss Anspruch 10.

14. 7β-[2-(2-Amino-4-oxazolyl)-2-Z-methoxyiminoacetylamino]-3-cephem-4-carbonsäure gemäss Anspruch 10.

15. Die Natriumsalze von Verbindungen gemäss Anspruch 13 oder 14.

16. 3-(1-Methyl-1H-tetrazol-5-ylthiomethyl)-7β-[2-(2-amino-4-oxazolyl)-2-Z-methoxyiminoacetylamino]-3-cephem-4-carbonsäure gemäss Anspruch 11.

17. 3-(1-Carboxymethyl-1H-tetrazol-5-ylthiomethyl)-7β-[2-(2-amino-4-oxazolyl)-2-Z-methoxyiminoacetylamino]-3-cephem-4-carbonsäure gemäss Anspruch 11.

18. 3-[1-(2-Dimethylaminoäthyl)-1H-tetrazol-5-ylthiomethyl]-7β-[2-(2-amino-4-oxazolyl)-2-Z-methoxyiminoacetylamino]-3-cephem-4-carbonsäure gemäss Anspruch 11.

19. 3-[1-(2-Dimethylaminoäthyl)-1H-tetrazol-5-ylthiomethyl]-7β-[2-(2-amino-4-oxazolyl)-2-E-methoxyiminoacetylamino]-3-cephem-4-carbonsäure gemäss Anspruch 11.

20. 3-(4-Carbamoylpyridiniomethyl)-7β-[2-(2-amino-4-oxazolyl)-2-Z-methoxyiminoacetylamino]-3-cephem-4-carboxylat gemäss Anspruch 11.

21. 3-Acetoxymethyl-7β-[2-(2-amino-4-oxazolyl)-2-Z-hydroxyiminoacetylamino]-3-cephem-4-carbonsäure gemäss Anspruch 11.

22. 3-Acetoxymethyl-7β-[2-(2-amino-4-oxazolyl)-2-Z-(2-carboxyprop-2-yloxyimino)-acetylamino]-3-cephem-4-carbonsäure gemäss Anspruch 11.

23. 3-Acetoxymethyl-7β-[2-(2-amino-4-oxazolyl)-2-Z-(2-tert.-butyloxycarbonylprop-2-yloxyimino)-acetylamino]-3-cephem-4-carbonsäure gemäss Anspruch 11.

24. 3-(3-Chlorpyridiniomethyl)-7β-[2-(2-amino-4-oxazolyl)-2-Z-methoxyiminoacetylamino]-3-cephem-4-carboxylat gemäss Anspruch 11.

25. 3-(4-Carboxylatpyridiniomethyl)-7β-[2-(2-amino-4-oxazolyl)-2-Z-methoxyiminoacetylamino]-3-cephem-4-carboxylat-natriumsalz gemäss Anspruch 11.

26. 3-Carbamoyloxymethyl-7β-[2-(2-amino-4-oxazolyl)-2-Z-methoxyimino-acetylamino]-3-cephem-4-carbonsäure-natriumsalz gemäss Anspruch 11.

27. Die Natriumsalze von Verbindungen gemäss Anspruch 16, 17, 21-23, 25 oder 26.

28. Die Mono- oder Dihydrochloride von Verbindungen gemäss Anspruch 18 oder 19.

29. 3-Pyridiniomethyl-7β-[2-(2-amino-4-oxazolyl)-2-Z-methoxyimino-acetylamino]-3-cephem-4-carboxylat gemäss Anspruch 12.

30. 7β-[2-(2-Amino-4-oxazolyl)-2-Z-methoxyiminoacetylamino]-3-cephem-4-carbonsäure-tert.-butyloxycarbonylmethylester gemäss Anspruch 12.

31. 3-Carbamoyloxymethyl-7β-[2-(2-amino-4-oxazolyl)-2-Z-methoxyimino-acetylamino]-3-cephem-4-carbonsäure-1-(äthoxycarbonyloxy)-äthylester gemäss Anspruch 12.

32. Verfahren zur Herstellung von 7β-Aminooxazolylacetylamino-3-cephem-4-carbonsäure-Verbindungen der Formel I gemäss Anspruch 1, worin n, $R_1$, $R_2$, $R_3$, A und $R_4$ die in Anspruch 1 genannten Bedeutungen haben, Hydraten und Salzen von diesen Verbindungen, dadurch gekennzeichnet, dass man

a) in einer Verbindung der Formel

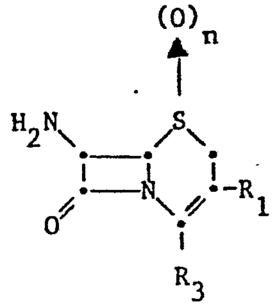

(II) ,

worin n, $R_1$ und $R_3$ die unter Formel I genannten Bedeutungen haben und die 7β-Aminogruppe in freier Form vorliegt oder durch eine die Acylierungsreaktion erlaubende Gruppe geschützt ist und in $R_1$ vorhandene funktionelle Gruppen geschützt sind, oder in einem Salz davon die 7β-Aminogruppe durch Umsetzung mit einem den Acylrest einer Carbonsäure der Formel

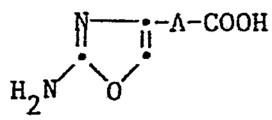

(III) ,

einführenden Acylierungsmittel, worin A die unter Formel I genannten Bedeutungen hat und die Aminogruppe in 2-Stellung des Oxazolylrests, sowie in A vorhandene funktionelle Gruppen gegebenenfalls geschützt sind, oder mit einem Salz davon acyliert oder

b) zur Herstellung einer Verbindung der Formel I, worin n Null bedeutet, eine 2-Cephem-Verbindung der Formel

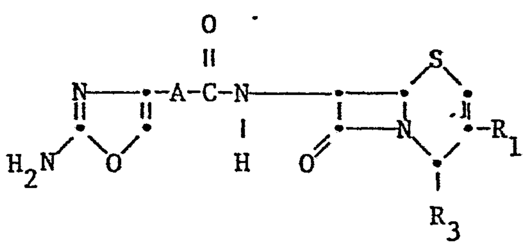

(IV) ,

worin $R_1$, $R_3$ und A die unter Formel I genannten Bedeutungen haben und die Aminogruppe in 2-Stellung des Oxazolylrests, sowie in $R_1$ und A vorhandene funktionelle Gruppen gegebenenfalls geschützt sind, oder ein Salz davon zur entsprechenden 3-Cephemverbindung isomerisiert oder

c) eine Verbindung der Formel

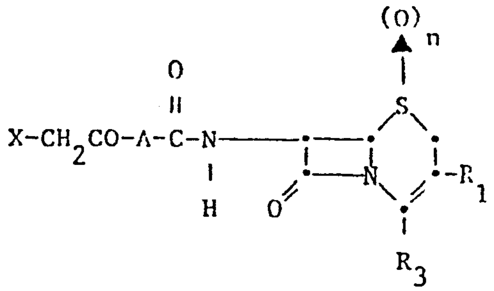 (V) ,

worin n, $R_1$, $R_3$ und A die unter Formel I genannten Bedeutungen haben und X Halogen bedeutet und in $R_1$ und A vorhandene funktionelle Gruppen gegebenenfalls geschützt sind, oder ein Salz davon mit Harnstoff kondensiert, oder

d) zur Herstellung einer Verbindung der Formel I, worin A durch Hydroxyimino substituiertes Methylen bedeutet, eine Verbindung der Formel

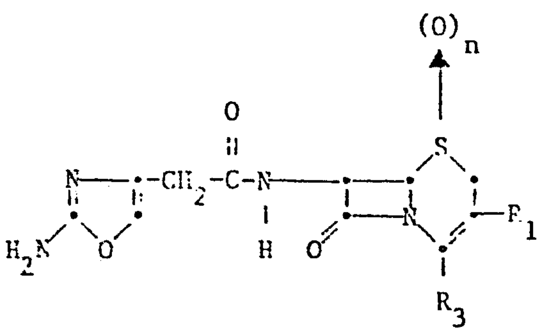 (VI) ,

worin n, $R_1$ und $R_3$ die unter Formel I genannten Bedeutungen haben und die Aminogruppe in 2-Stellung des Oxazolylrests und in $R_1$ vorhandene funktionelle Gruppen gegebenenfalls geschützt sind, oder ein Salz davon mit einem Nitrosierungsmittel behandelt und, wenn erwünscht, eine erhältliche Verbindung der Formel I in eine andere Verbindung der Formel I umwandelt und/oder eine erhältliche Verbindung, worin n Null bedeutet, in eine Verbindung überführt, worin n eins oder zwei bedeutet, und/oder eine erhältliche Verbindung, worin n eins oder zwei bedeutet, in eine Verbindung überführt, worin n Null bedeutet, und/oder eine er-

BAD ORIGINAL

hältlichen Verbindung in geschützter Form vorliegende funktionelle Gruppen in freie funktionelle Gruppen überführt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomeren auftrennt.

33. Die nach dem Verfahren gemäss Anspruch 32 erhältlichen Verbindungen.

34. Pharmazeutische Präparate enthaltend Verbindungen der Formel I gemäss Anspruch 1, Hydrate oder pharmazeutisch verwendbare Salze von solchen Verbindungen.

35. Verbindungen der Formel I gemäss Anspruch 1 zur Anwendung bei der Behandlung des menschlischen oder tierischen Körpers von bakteriellen Infektionen.

36. Carbonsäuren der Formel

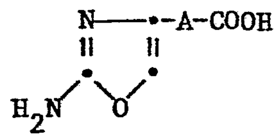

(III),

worin A Carbonyl, Methylen oder eine Methylengruppe, welche durch Amino, geschütztes Amino, Hydroxy, geschütztes Hydroxy, Sulfo, geschütztes Sulfo oder durch eine Gruppe der Formel $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt und worin die Aminogruppe sowie in A vorhandene funktionelle Gruppen gegebenenfalls geschützt sind, reaktionsfähige, funktionelle Derivate und Salze davon.

37. 2-(2-Amino-4-oxazolyl)-2-Z-methoxyiminoessigsäure, worin die Aminogruppe gegebenenfalls geschützt ist, gemäss Anspruch 36.

38. 2-(2-Amino-4-oxazolyl)-2-hydroxyiminoessigsäure, worin die Hydroxy- und/oder die Aminogruppe gegebenenfalls geschützt sind, gemäss Anspruch 36.

39. 2-(2-Amino-4-oxazolyl)-2-Z-(2-carboxyprop-2-yloxyimino)-essigsäure, worin die Carboxy- und/oder Aminogruppe gegebenenfalls geschützt sind, gemäss Anspruch 36.

40. Verfahren zur Herstellung von Carbonsäuren der Formel III, worin A die in Anspruch 36 genannte Bedeutung hat, sowie reaktionsfähigen, funktionellen Derivaten und Salzen davon, dadurch gekennzeichnet, dass man eine Verbindung der Formel

$$X\text{-}CH_2\text{-}\overset{\overset{\displaystyle O}{\|}}{C}\text{-}A\text{-}COOH \qquad \text{(VII)}$$

worin X Halogen bedeutet und A die unter Formel I genannten Bedeutungen hat und die Carboxylgruppe in veresterter Form vorliegt bzw. durch eine übliche Carboxylschutzgruppe geschützt ist, mit Harnstoff umsetzt, und, wenn erwünscht, in einer erhältlichen Verbindung, die Carboxylschutzgruppe abspaltet und/oder die in 2-Stellung des Oxazolylrests befindliche Aminogruppe schützt und/oder eine in A befindliche funktionelle Gruppe schützt und/oder die Gruppe A in eine andere Gruppe A umwandelt.

41. Die nach dem Verfahren gemäss Anspruch 40 erhältlichen Verbindungen.

Patentansprüche: (für Oesterreich)

1. Verfahren zur Herstellung von 7β-Aminooxazolylacetylamino-3-cephem-4-carbonsäure-Verbindungen der Formel

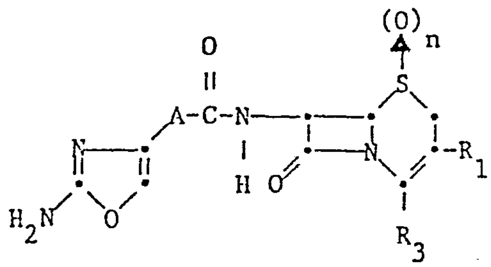

(I) ,

worin

n eine ganze Zahl von 0 bis 2, A Carbonyl, Methylen, oder eine Methylengruppe, welche durch Amino, geschütztes Amino, Hydroxy, geschütztes Hydroxy, Sulfo, geschütztes Sulfo oder durch eine Gruppe der Formel

$$=N-O-R_4$$

substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$ Wasserstoff, Niederalkyl, Niederalkoxy, Halogen oder eine Gruppe der Formel $-CH-R_2$, worin $R_2$ Hydroxy, Mercapto, verestertes Hydroxy oder Mercapto, veräthertes Hydroxy oder Mercapto oder eine Ammoniogruppe darstellt, und $R_3$ Carboxyl oder geschütztes Carboxyl bedeuten, Hydraten und Salzen von diesen Verbindungen, dadurch gekennzeichnet, dass man

a) in einer Verbindung der Formel

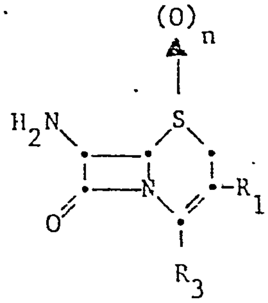

(II) ,

worin n, $R_1$ und $R_3$ die unter Formel I genannten Bedeutungen haben und die 7β-Aminogruppe in freier Form vorliegt oder durch eine die Acylierungsreaktion erlaubende Gruppe geschützt ist und in $R_1$ vorhandene funktionelle Gruppen geschützt sind, oder in einem Salz davon die 7β-Aminogruppe durch Umsetzung mit einem den Acylrest einer Carbonsäure der Formel

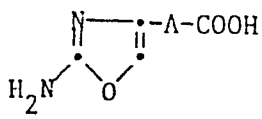

(III) ,

einführenden Acylierungsmittel, worin A die unter Formel I genannten Bedeutungen hat und die Aminogruppe in 2-Stellung des Oxazolylrests, sowie in A vorhandene funktionelle Gruppen gegebenenfalls geschützt sind, oder mit einem Salz davon acyliert oder

b) zur Herstellung einer Verbindung der Formel I, worin n Null bedeutet, eine 2-Cephem-Verbindung der Formel

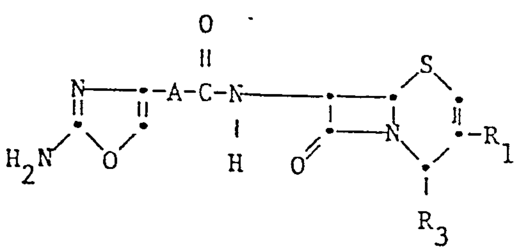

(IV) ,

worin $R_1$, $R_3$ und A die unter Formel I genannten Bedeutungen haben und die Aminogruppe in 2-Stellung des Oxazolylrests, sowie in $R_1$ und A vorhandene funktionelle Gruppen gegebenenfalls geschützt sind, oder ein Salz davon zur entsprechenden 3-Cephemverbindung isomerisiert oder

c) eine Verbindung der Formel

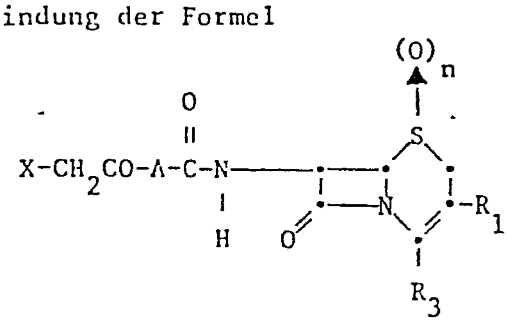

(V) ,

worin n, $R_1$, $R_3$ und A die unter Formel I genannten Bedeutungen haben und X Halogen bedeutet und in $R_1$ und A vorhandene funktionelle Gruppen gegebenenfalls geschützt sind, oder ein Salz davon mit Harnstoff kondensiert, oder

d) zur Herstellung einer Verbindung der Formel I, worin A durch Hydroxyimino substituiertes Methylen bedeutet, eine Verbindung der Formel

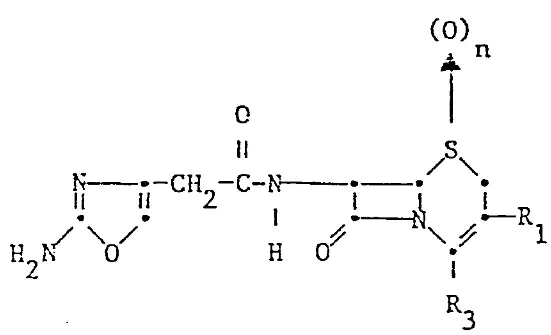

(VI) ,

worin n, $R_1$ und $R_3$ die unter Formel I genannten Bedeutungen haben und die Aminogruppe in 2-Stellung des Oxazolylrests und in $R_1$ vorhandene funktionelle Gruppen gegebenenfalls geschützt sind, oder ein Salz davon mit einem Nitrosierungsmittel behandelt und, wenn erwünscht, eine erhältliche Verbindung der Formel I in eine andere Verbindung der Formel I umwandelt und/oder eine erhältliche Verbindung, worin n Null bedeutet, in eine Verbindung überführt, worin n eins oder zwei bedeutet, und/oder eine erhältliche Verbindung, worin n eins oder zwei bedeutet, in eine Verbindung überführt, worin n Null bedeutet, und/oder in einer erhältlichen Verbindung in geschützter Form vorliegende funktionelle Gruppen in freie funktionelle Gruppen überführt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomeren auftrennt.

2. Verfahren zur Herstellung von 7β-Aminooxazolylacetylamino-3-cephem-4-carbonsäure-Verbindungen der Formel I gemäss Anspruch 1, worin n eine ganze Zahl von 0 bis 2, A Carbonyl oder eine Methylengruppe, welche durch Amino, geschütztes Amino, Hydroxy, geschütztes Hydroxy, Sulfo, geschütztes Sulfo oder durch eine Gruppe $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$, $R_2$ und $R_3$ die in Anspruch 1 genannten Bedeutungen haben, Hydraten und Salzen von diesen Verbindungen, dadurch gekennzeichnet, dass man die in Anspruch 1 genannten Massnahmen durchführt.

3. Verfahren zur Herstellung von 7β-Aminooxazolylacetylamino-3-cephem-4-carbonsäure-Verbindungen der Formel I gemäss Anspruch 1, worin n eine ganze Zahl von 0 bis 2, A Carbonyl oder eine Methylengruppe, welche durch Amino, Hydroxy, geschütztes Hydroxy, Sulfo, geschütztes Sulfo oder durch eine Gruppe der Formel $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt, $R_1$, $R_2$ und $R_3$ die in Anspruch 1 genannten Bedeutungen haben, Hydraten und Salzen von diesen Verbindungen, dadurch gekennzeichnet, dass man die in Anspruch 1 genannten Massnahmen durchführt.

4. Verfahren nach Anspruch 1 zur Herstellung von Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, N-Niederalkylcarbamoyloxy, einen aromatischen, monocyclischen, fünf- oder sechsgliedrigen, Diaza-, Triaza-, Tetraaza-, Thiaza-, Thiadiaza-,

Thia-, Oxaza- oder Oxadiazacyclylrest, z.B. Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazinylthio, z.B. 5,6-Dioxo-1,2,5,6-tetrahydro-as-triazin-3-ylthio oder 5,6-Dioxo-1,4,5,6-tetrahydro-as-triazin-3-ylthio, welcher durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl, Carboxyniederalkyl, z.B. Carboxymethyl, Amino, Carboxyniederalkylamino, z.B. 2-Carboxyäthylamino, oder durch Carbamoyl substituiert sein kann, oder Ammonio, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxyl oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl, oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A Amino, Aminomethylen, Hydroxymethylen, Sulfomethylen oder eine durch eine Gruppe der Formel $=N-O-R_4$ substituierte Methylengruppe, worin $R_4$ Wasserstoff, Niederalkyl, z.B. Methyl, Hydroxyniederalkyl, z.B. 2-Hydroxyäthyl, Aminoniederalkyl, z.B. 2-Aminoäthyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, darstellt, bedeuten, wobei die Gruppe der Formel $-O-R_4$ die syn-(oder Z-)Stellung aufweist, und Salzen, insbesondere pharmazeutisch verwendbaren Salzen, von Verbindungen der Formel I, welche salzbildende Gruppen besitzen.

5. Verfahren nach Anspruch 2 zur Herstellung von Verbindungen der Formel I, worin n, A, $R_1$, $R_2$ und $R_4$ die in Anspruch 4 genannten Bedeutungen haben und $R_3$ Carboxyl oder unter physiologischen Bedinungen spaltbares Carboxyl, z.B. Acyloxymethoxycarbonyl, z.B. Niederalkanoyloxymethoxycarbonyl, z.B. Pivaloyloxymethoxycarbonyl, oder Niederalkoxycarbonyloxyniederalkoxycarbonyl, z.B. 1-(Aethoxycarbonyloxy)-äthoxycarbonyl, bedeutet, und Salzen, insbesondere pharmazeutisch annehmbaren Salzen von diesen Verbindungen.

6. Verfahren nach Anspruch 3 zur Herstellung von Verbindungen der Formel I, worin n, A, $R_1$, $R_2$ und $R_3$ die in Anspruch 4 genannten Bedeutungen haben, und $R_3$ Carboxyl, Pivaloyloxymethoxycarbonyl oder 1-(Aethoxycarbonyloxy)-äthoxycarbonyl bedeutet, und Salzen, insbesondere pharmazeutisch annehmbaren Salzen von diesen Verbindungen.

7. Verfahren nach Anspruch 4 zur Herstellung von Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes

Pyridinio, z.B. 3- oder 4-Hydroxymethylpyridinio, 4-Carboxypyridinio, 3- oder 4-Carboxymethylpyridino, 3- oder 4-Chlorpyridinio, 3- oder 4-Brompyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel $-O-R_4$ die syn-(oder Z-)Stellung aufweist, und Salzen, insbesondere pharmazeutisch verwendbaren Salzen, von Verbindungen der Formel I, welche salzbildende Gruppen aufweisen.

8. Verfahren nach Anspruch 5 zur Herstellung von Verbindungen der Formel I, worin n, A, $R_1$, $R_2$ und $R_4$ die in Anspruch 7 genannten Bedeutungen haben, und $R_3$ Carboxyl, Niederalkanoyloxymethoxycarbonyl, z.B. Pivaloyloxymethoxycarbonyl oder Niederalkoxycarbonyloxyniederalkoxycarbonyl, z.B. 1-(Aethoxycarbonyloxy)-äthoxycarbonyl, bedeutet, und Salzen, insbesondere pharmazeutisch verwendbaren Salzen von diesen Verbindungen.

9. Verfahren nach Anspruch 6 zur Herstellung von Verbindungen der Formel I, worin n, A, $R_1$, $R_2$ und $R_4$ die in Anspruch 7 genannten Bedeutungen haben, und $R_3$ Carboxyl, Pivaloyloxymethoxycarbonyl oder 1-(Aethoxycarbonyloxy)-äthoxycarbonyl bedeutet, und Salzen, insbesondere pharmazeutisch annehmbaren Salzen von diesen Verbindungen.

10. Verfahren nach Anspruch 7 zur Herstellung von Verbindungen der Formel I, worin n Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Tetrazolylthio, z.B. 1H-Tetrazol-5-ylthio, durch Niederalkyl, z.B.

Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio, oder 1-Carboxymethyl-1H-tetrazol-5-ylthio, Thiadiazolylthio, z.B. 1,3,4-Thiadiazol-5-ylthio, oder durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridino, 3- oder 4-Chlorpyridinio, 3- oder 4-Brompyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-(Propionyloxy)-äthoxycarbonyl oder Niederalkoxycarbonyloxyniederalkoxycarbonyl, z.B. 1-(Aethoxycarbonyloxy)-äthoxycarbonyl oder tert.-Butyloxycarbonyloxymethoxycarbonyl, und A eine durch eine Gruppe der Formel $=N-O-R_4$ substituierte Methylengruppe, worin $R_4$ Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel $-O-R_4$ die syn-(oder Z-)Stellung aufweist, und Salzen, insbesondere pharmazeutisch verwendbaren Salzen, von Verbindungen der Formel I, welche salzbildende Gruppen aufweisen.

11. Verfahren nach Anspruch 8 zur Herstellung von Verbindungen der Formel I, worin n, A, $R_1$, $R_2$ und $R_4$ die in Anspruch 10 genannten Bedeutungen haben, und $R_3$ Carboxyl, Niederalkanoyloxymethoxycarbonyl, z.B. Pivaloyloxymethoxycarbonyl, oder Niederalkoxcarbonyloxyniederalkoxycarbonyl, z.B. 1-(Aethoxycarbonyloxy)-äthoxycarbonyl, bedeutet,

und Salzen, insbesondere pharmazeutisch verwendbaren Salzen von diesen Verbindungen.

12. Verfahren nach Anspruch 9 zur Herstellung von Verbindungen der Formel I, worin n, A, $R_1$, $R_2$ und $R_4$ die in Anspruch 10 genannten Bedeutungen haben, und $R_3$ Carboxyl, Pivaloyloxymethoxycarbonyl oder 1-(Aethoxycarbonyloxy)-äthoxycarbonyl bedeutet, und Salzen, insbesondere pharmazeutisch verwendbaren Salzen von diesen Verbindungen.

13. Verfahren gemäss Anspruch 10 zur Herstellung von 3-Acetoxymethyl-7β-[2-(2-amino-4-oxazolyl)-2-Z-methoxyiminoacetylamino]-3-cephem-4-carbonsäure-natriumsalz.

14. Verfahren gemäss Anspruch 10 zur Herstellung von 7β-[2-(2-Amino-4-oxazolyl)-2-Z-methoxyiminoacetylamino]-3-cephem-4-carbonsäure-natriumsalz.

15. Verfahren zur Herstellung der Natriumsalze von Verbindungen gemäss Anspruch 13 oder 14.

16. Verfahren gemäss Anspruch 11 zur Herstellung von 3-(1-Methyl-1H-tetrazol-5-ylthiomethyl)-7β-[2-(2-amino-4-oxazolyl)-2-Z-methoxyiminoacetylamino]-3-cephem-4-carbonsäure.

17. Verfahren gemäss Anspruch 11 zur Herstellung von 3-(1-Carboxymethyl-1H-tetrazol-5-ylthiomethyl)-7β-[2-(2-amino-4-oxazolyl)-2-Z-methoxyiminoacetylamino]-3-cephem-4-carbonsäure.

18. Verfahren gemäss Anspruch 11 zur Herstellung von 3-[1-(2-Dimethylaminoäthyl)-1H-tetrazol-5-ylthiomethyl]-7β-[2-(2-amino-4-oxazolyl)-2-Z-methoxyiminoacetylamino]-3-cephem-4-carbonsäure.

19. Verfahren gemäss Anspruch 11 zur Herstellung von 3-[1-(2-Dimethylaminoäthyl)-1H-tetrazol-5-ylthiomethyl]-7β-[2-(2-amino-4-oxazolyl)-2-E-methoxyiminoacetylamino]-3-cephem-4-carbonsäure.

20. Verfahren gemäss Anspruch 11 zur Herstellung von 3-(4-Carbamoylpyridiniomethyl)-7β-[2-(2-amino-4-oxazolyl)-2-Z-methoxyiminoacetylamino]-3-cephem-4-carboxylat.

21. Verfahren gemäss Anspruch 11 zur Herstellung von 3-Acetoxymethyl-7β-[2-(2-amino-4-oxazolyl)-2-Z-hydroxyiminoacetylamino]-3-cephem-4-carbonsäure.

22. Verfahren gemäss Anspruch 11 zur Herstellung von 3-Acetoxymethyl-7β-[2-(2-amino-4-oxazolyl)-2-Z-(2-carboxyprop-2-yloxyimino)-acetylamino]-3-cephem-4-carbonsäure.

23. Verfahren gemäss Anspruch 11 zur Herstellung von 3-Acetoxymethyl-7β-[2-(2-amino-4-oxazolyl)-2-Z-(2-tert.-butyloxycarbonylprop-2-yloxyimino)-acetylamino]-3-cephem-4-carbonsäure.

24. Verfahren gemäss Anspruch 11 zur Herstellung von 3-(3-Chlorpyridiniomethyl)-7β-[2-(2-amino-4-oxazolyl)-2-Z-methoxyiminoacetylamino]-3-cephem-4-carboxylat.

25. Verfahren gemäss Anspruch 11 zur Herstellung von 3-(4-Carboxylatpyridiniomethyl)-7β-[2-(2-amino-4-oxazolyl)-2-Z-methoxyiminoacetylamino]-3-cephem-4-carboxylat.

26. Verfahren gemäss Anspruch 11 zur Herstellung von 3-Carbamoyloxymethyl-7β-[2-(2-amino-4-oxazolyl)-2-Z-methoxyiminoacetylamino]-3-cephem-4-carbonsäure.

27. Verfahren zur Herstellung der Natriumsalze von Verbindungen gemäss Anspruch 16, 17, 21-23, 25 oder 26.

28. Verfahren zur Herstellung der Mono- oder Dihydrochloride von Verbindungen gemäss Anspruch 18 oder 19.

29. Verfahren gemäss Anspruch 12 zur Herstellung von 7β-[2-(2-Amino-4-oxazolyl)-2-Z-methoxyiminoacetylamino]-3-cephem-4-carbonsäurer-tert.-butyloxycarbonylmethylester.

30. Verfahren gemäss Anspruch 12 zur Herstellung von 3-Pyridiniomethyl-7β-[2-(2-amino-4-oxazolyl)-2-Z-methoxyiminoacetylamino]-3-cephem-4-carboxylat.

31. Verfahren gemäss Anspruch 12 zur Herstellung von 3-Carbamoyloxy-methyl-7β-[2-(2-amino-4-oxazolyl)-2-Z-methoxyiminoacetylamino]-3-cephem-4-carbonsäure-1-(äthoxycarbonyloxy)-äthylester.

32. Verfahren zur Herstellung von pharmazeutischen Präparaten, gekennzeichnet durch die Verarbeitung von Verbindungen gemäss Anspruch 1 der Formel I, Hydraten oder pharmazeutisch verwendbaren Salzen von solchen Verbindungen mit einem pharmazeutischen Trägermaterial.

33. Verfahren zur Herstellung von Carbonsäuren der Formel

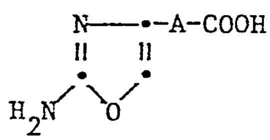

(III),

worin A Carbonyl, Methylen oder eine Methylengruppe, welche durch Amino, geschütztes Amino, Hydroxy, geschütztes Hydroxy, Sulfo, geschütztes Sulfo oder durch eine Gruppe der Formel $=N-O-R_4$ substituiert ist, worin $R_4$ Wasserstoff, Niederalkyl, substituiertes Niederalkyl, Cycloalkyl, substituiertes Cycloalkyl, Carbamoyl oder substituiertes Carbamoyl darstellt und worin die Aminogruppe sowie in A vorhandene funktionelle Gruppen gegebenenfalls geschützt sind, sowie reaktionsfähigen, funktionellen Derivaten und Salzen davon, dadurch gekennzeichnet, dass man eine Verbindung der Formel

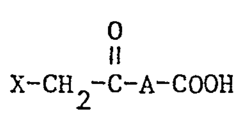

(VII)

worin X Halogen bedeutet und A die unter Formel I genannten Bedeutungen hat und die Carboxylgruppe in veresterter Form vorliegt bzw. durch eine übliche Carboxylschutzgruppe geschützt ist, mit Harnstoff umsetzt und, wenn erwünscht, in einer erhältlichen Verbindung, die Carboxylschutzgruppe abspaltet und/oder die in 2-Stellung des Oxazolylrests befindliche Aminogruppe schützt und/oder eine in A befindliche funktionelle Gruppe schützt und/oder die Gruppe A in eine andere Gruppe A umwandelt.

34. Verfahren gemäss Anspruch 23 zur Herstellung von 2-(2-Amino-4-oxazolyl)-2-Z-methoxyiminoessigsäure, worin die Aminogruppe gegebenenfalls geschützt ist.

35. Verfahren gemäss Anspruch 34 zur Herstellung von 2-(2-Amino-4-oxazolyl)-2-hydroxyiminoessigsäure, worin die Hydroxy- und/oder die Aminogruppe gegebenenfalls geschützt sind.

36. Verfahren gemäss Anspruch 33 zur Herstellung von 2-(2-Amino-4-oxazolyl)-2-Z-(2-carboxyprop-2-yloxyimino)-essigsäure, worin die Carboxy und/oder die Aminogruppe gegebenenfalls geschützt sind.